# EUROPEAN PATENT SPECIFICATION

(11) **EP 1 732 933 B1**
(45) Date of publication and mention of the grant of the patent: **23.07.2008**
(21) Application number: 05732643.1
(22) Date of filing: 17.03.2005
(51) Int. Cl.: C07D 495/04, C07D 487/04, C07D 471/04, A61K 31/44, A61P 3/04, A61P 9/00, A61K 31/47, A61K 31/435

(54) **COMPOUNDS FOR TREATING DYSLIPIDEMIA**
VERBINDUNGEN ZUR BEHANDLUNG VON DYSLIPIDEMIE
COMPOSES DESTINES AU TRAITEMENT DE LA DYSLIPIDEMIE

(30) Priority: 26.03.2004 US 557134 P; 22.10.2004 US 621162 P
(43) Date of publication of application: 20.12.2006
(73) Proprietor: ELI LILLY AND COMPANY, Indianapolis, Indiana 46285 (US)
(72) Inventor: BELL, Michael, Gregory, Indianapolis, IN 46256 (US); CAO, Guoqing, Carmel, IN 46033 (US); ESCRIBANO, Ana Maria, Lilly, S.A., E-28108 Madrid (ES); FERNANDEZ, Maria, Carmen, Lilly, S.A., E-28108 Madrid (ES); MANTLO, Nathan, Bryan, Brownsburg, IN 46112 (US); MARTIN DE LA NAVA, Eva, Maria, Lilly, S.A., E-28108 Madrid (ES); MATEO HERRANZ, Ana, Isabel, Lilly, S.A., E-28108 Madrid (ES); MAYHUGH, Daniel, Ray, Carmel, IN 46032 (US); WANG, Xiaodong, Burlingame, CA 94010 (US)
(74) Representative: Suarez-Miles, Ana Sanchiz
(86) International application number: PCT/US2005/009294
(87) International publication number: WO 2005/097805

(56) References cited:
- EP-A- 0 818 197
- WO-A-20/05037796
- CHO HIDETSURA ET AL: "Synthesis and structure-activity relationships of 5,6,7,8-tetrahydro-4H-thieno[3,2-b]azepine derivatives: novel arginine vasopressin antagonists." JOURNAL OF MEDICINAL CHEMISTRY. 1 JAN 2004, vol. 47, no. 1, 1 January 2004 (2004-01-01), pages 101-109, XP002338785 ISSN: 0022-2623
- PATENT ABSTRACTS OF JAPAN vol. 2003, no. 12, 5 December 2003 (2003-12-05) -& JP 2003 321472 A (TAKEDA CHEM IND LTD), 11 November 2003 (2003-11-11)

## Description

### FIELD OF THE INVENTION

The current invention relates to the fields of medicinal organic chemistry, pharmacology, and medicine. Further, the current invention relates to a group of compounds that demonstrate utility for treating pathological states due to dyslipidemia.

### BACKGROUND OF THE INVENTION

Coronary heart disease (CHD) is one of the major causes of morbidity and mortality worldwide. Despite attempts to modify risk factors such as obesity, smoking, lack of exercise, and treatment of dyslipidemia with dietary modification or drug therapy, CHD remains the most common cause of death in the U.S. Over 50% of all CHD deaths are due to underlying atherosclerotic coronary heart disease.

Dyslipidemia is a major risk factor for CHD. Low plasma levels of high density lipoprotein (HDL) cholesterol with either normal or elevated levels of low density (LDL) cholesterol is a significant risk factor for developing atherosclerosis and associated coronary artery disease in humans. Indeed, several studies on lipoprotein profiles of CHD patients have shown that about 50% of the CHD patients have cholesterol levels that are considered to be in the normal range (<200 mg/dl). Furthermore, these studies found low HDL cholesterol in about 40% of the normo-cholesterolemic CHD patients as compared to the general population reported in the National Health and Nutrition Examination Survey. Since low levels of HDL cholesterol increase the risk of atherosclerosis, methods for elevating plasma HDL cholesterol would be therapeutically beneficial for the treatment of cardiovascular disease including, but not limited to, atherosclerosis, CHD, stroke, and peripheral vascular disease.

Cholesterol ester transfer protein (CETP) is a 74 KD glycoprotein that facilitates the exchange of cholesterol esters in HDL for triglycerides in triglyceride-rich lipoproteins (A. R. Tall et. al., (1999) 1999 George Lyman Duss Memorial Lecture: Lipid transfer proteins, HDL metabolism and athero genesis. Arterio. Thromb. Vasc. Biol. 20:1185-1188.). The net result of CETP activity is a lowering of HDL cholesterol and an increase in LDL cholesterol. This effect on lipoprotein profile is believed to be proatherogenic, especially in subjects whose lipid profile constitutes an increased risk for CHD. Niacin can significantly increase HDL, but has serious toleration issues that reduce compliance. Fibrates and the HMG CoA reductase inhibitors raise HDL cholesterol only modestly (~10-12%). As a result, there is a significant unmet medical need for a well-tolerated agent that can significantly elevate plasma HDL levels, thereby reversing or slowing the progression of atherosclerosis.

CETP is expressed in multiple tissues and secreted into plasma, where it associates with HDL (X.C. Jiang et. al., (1991) Mammalian adipose tissue and muscle are major sources of lipid transfer protein mRNA. J. Biol. Chem. 266:4631-4639). Humans and monkeys, which express CETP, have relatively low HDL cholesterol, whereas mice and rats do not express CETP and carry nearly all their cholesterol in HDL. Further more, transgenic expression of CETP in mice results in significantly reduced HDL cholesterol levels and development of severe atherosclerosis compared to control mice (K.R. Marotti et. al., (1993) Severe atherosclerosis in trans genic mice expressing simian cholesteryl ester transfer protein. Nature:364, 73-75). Expression of human CETP in Dahl salt-sensitive hypertensive rats led to spontaneous combined hyperlipidemia, coronary heart disease and decreased survival (V.L.M. Herrera et. al., (1999) Spontaneous combined hyperlipidemia, coronary heart disease and decreased survival in DahI salt-sensitive hypertensive rats transgenic for human cholesteryl ester transfer protein. Nature Medicine: 5, 1383-1389).

Antibodies either directly injected into the plasma or generated through vaccine injection can effectively inhibit CETP activity in hamsters and rabbits resulting in elevated HDL cholesterol (C. W. Rittershaus, (1999) Vaccine-induced antibodies inhibit CETP activity in vivo and reduce aortic lesions in a rabbit model of atherosclerosis. Furthermore, antibody neutralization of CETP in rabbits has been shown to be anti-atherogenic (Arterio. Thromb. Vasc. Biol. 20, 2106-2112;G.F.Evans et. at, (1994) Inhibition of cholesteryl ester transfer protein in normocholesterolemic and hypercholesterolemic hamsters: effects on HDL subspecies, quantity, and apolipoprotein distribution. J. Lipid Research. 35, 1634-1645). However, antibody and/or vaccine therapy is not currently a viable option for the treatment of large populations of patients in need of treatment for dyslipidemia and resultant or associated disease state manifestations.

Cholesterol ester transfer protein (CETP) catalyzes the exchange of neutral lipid between HDL and apoB-containing lipoprotein particles. As a n et result of this exchange, HDL cholesterol is reduced and LDL particles are further enriched with cholesterol, resulting in LDL cholesterol elevation and formation of small dense LDL particles, which are believed to be more atherogenic. CETP inhibition (small molecule, antibody, antisense oligo etc.) effectively elevates HDL cholesterol and also reduces LDL cholesterol in animal models as well as in humans (Whitlock, M. et al., J. of Clin. Invest., 1989, Vol. 84, 129-137, Hirochi, O. et al., Nature, 2000, Vol. 406, 203-207, Grooth, G. et al., Circulation, 2002;105:2159-2165, Clark, R. et al., Arterioscler Thromb Vasc Biol. 2004;24:1-9, Brousseau M. et al., New Engl. J. Med., 2004, Vol. 350:1505-1515). Further, CETP inhibition leads to the formation of less-dense LDL particles-a benefit in addition to LDL cholesterol lowering (Brousseau M. et al., New Engl. J. Med., 2004, Vol. 350:1505-1515). Thus, administration of CETP inhibitors to humans in need thereof would significantly elevate HDL cholesterol level and reduce LDL cholesterol levels and increase LDL particle size, all of which are believed to benefit patients exposed to atherosclerotic risks.

There have been several reports of small molecule CETP inhibitors. Barrret et. al. (J.Am Chem. Soc., 188, 7863, (1996)) and Kuo et al. (J. Am. Chem. Soc.,117,10629, (1995)) describe cyclopropan-containing CETP inhibitors. Pietzonka et al. (Biorg. Med. Chem. Lett. 6, 1951 (1996)) describe phosphanate-containing analogs as CETP inhibitors. Coval et al. (Bioorg. Med. Chem. Lett. 5, 605, (1995)) describe Wiedendiol-A and-B related sesquiterpines as CETP inhibitors. Japanese Patent Application No. 10287662-A describes polycyclic, non-amine containing, polyhydroxylic natural compounds possessing CETP inhibition properties. Lee et al. (J. Antibiotics, 49, 693-96 (1996)) describe CETP inhibitors derived from an insect fungus. Busch et al. (Lipids, 25, 216-220 (1990)) describe cholesteryl acetyl bromide as a CETP inhibitor. Morton and Zillversmit (J. Lipid Res., 35, 836-47 (1982)) describe that p-chloromercuriphenyl sulfonate, p-hydroxymercuribenzoate and ethyl mercurithiosalicylate inhibit CETP. Connolly et al. (Biochem. Biophys. Res. Comm. 223, 42-47 (1996)) describe other cysteine modification reagents as CETP inhibitors. Xia et al. Describe 1,3,5-triazines as CETP inhibitors (Bioorg. Med. Chem. Lett., 6, 919-22 (1996)). B is gaier et al. (Lipids, 29, 811-8 (1994) describe 4-phenyl-5-tridecyl-4H-1,2,4-triazole-thiol as a CETP inhibitor. Oomura et al., disclose non-peptidic tetracyclic and hexacyclic phenols as CETP inhibitors in Japanese Patent Application No. 10287662.

United States patent No.6,586,448 B1 describes 4-caboxamino-2-sub stituted-1,2,3,4-tetrahydroquinolines of formula I 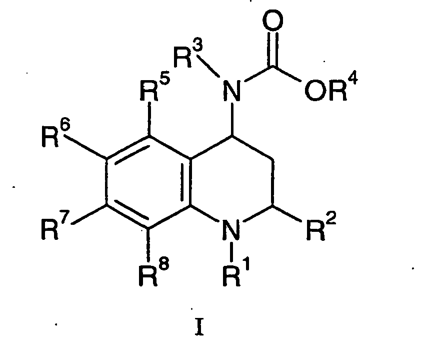 and prodrugs thereof, and pharmaceutically acceptable salts of said compounds and said prodrugs; wherein R¹,R², R³, R⁴, R⁵, R⁶, R⁷ and R⁸ are as defined therein. Similarly, PCT patent applications WO 03/063868A1, WO 0017164, No.0017165, and WO 0017166, discloses variously, formulations, methods of preparation and methods of use of tetrahydroquinoline compounds generally related to that of U.S patent 6,586,448 B1 from which it derives or is a divisional application thereof.

European Patent Application No. 818448 by Schmidt et al. describes tetrahydroquinoline derivatives as cholesteryl ester transfer protein inhibitors. European Patent Application No. 818197, Schmek et al., describe pyridines with fused heterocycles as cholesteryl ester transfer protein inhibitors. Brandes et al. in German Patent Application No. 19627430 describe bicyclic condensed pyridine derivatives as cholesteryl ester transfer protein inhibitors. In US Patent 6,207,671 Schmidt et al., describe substituted pyridine compounds as CETP inhibitors. In WO Patent Application No. 09839299, and WO Patent application No.03028727 by Muller-gliemann et al. and Erfinder/Anmelder respectively, describe quinoline derivatives as cholesteryl ester transfer protein inhibitors.

The above disclosures notwithstanding, a great need remains for effective compounds useful as CETP inhibitors to treat conditions caused by, associated with or exacerbated by dyslipidemia.

### SUMMARY OF THE INVENTION

The present invention provides a compound of formula I 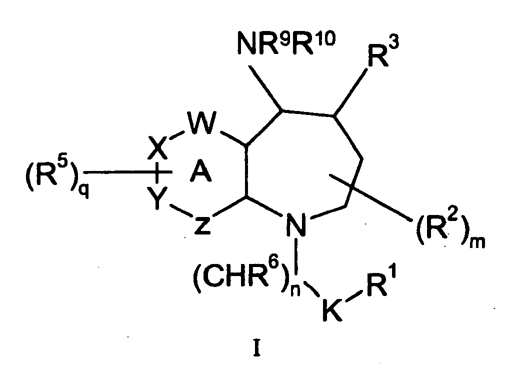 wherein
n is 0, 1, 2, or 3;
m is 0, 1, 2, 3, 4, 5 or 6;
q is 0, 1, or 2;
W, X, Y and Z are each independently CH, C, N, S, or O with appropriate single or double bonds and/or hydrogen atoms to complete valency requirements; Ring A is a five or six member ring wherein one of W, X, Y or Z may be absent; provided that ring A is not phenyl;
K is a bond, C=O, or S(O)p;
p is 0, 1 or 2;
R¹ is selected from hydroxy, C₁-C₆ alkyl, C₂-C₆ alkenyl, C₁-C₆ haloalkyl, C₁-C₆ alkylheterocyclic, C₃-C₈ cycloalkyl, C₁-C₆ alkylC₃-C₈cycloalkyl, C₁-C₆ alkylaryl, aryl, heterocyclyl, C₂-C₆ alkylalcohol, -OC₁-C₆ alkyl, -O-aryl, -OC₂-C₆ alkenyl, -OC₁-C₆ haloalkyl, -OC₁-C₆ alkylheterocyclic, -OC₃-C₈ cycloalkyl, -OC₁-C₆ alkylC₃-C₈cycloalkyl, -NR⁷R⁸, -OC₁-C₆ alkylaryl, -O-heterocyclic, -OC₁-C₆alkylCO₂R¹¹, -OC₂-C₆alkylalcohol, -OC₁-C₆alkylNR⁷R⁸, -OC₂-C₆alkylcyano, CONR¹¹R¹², NR¹¹SO₂R¹², NR¹¹COR¹², C₀-C₃ alkylNR¹¹R¹², C₁-C₃ alkylCOR¹¹ and C₀-C₆ alkylCOOR¹¹; provided that R¹ is not hydroxy when K is S(O)ₚ, CO, and/or when n is zero and K is a bond; and wherein each cycloalkyl, aryl or heterocyclic group is optionally substituted with 1 to 3 groups independently selected from oxo, hydroxy, halo, C₁-C₆ alkyl, C₂-C₆ alkenyl, C₂-C₆ alkynyl, C₁-C₆ alkoxy, C₁-C₆ haloalkyl, C₁-C₆ alkylalcohol, -OC₂-C₆alkylalcohol, C₁-C₆ haloalkoxy, CONR¹¹R¹², NR¹¹SO₂R¹², NR¹¹COR¹², C₀-C₃ alkylNR¹¹R¹², C₁-C₃ alkylCOR¹¹, C₀-C₆ alkylCOOR¹¹, C₀-C₆ alkylcyano, -OC₂-C₆alkylcyano, C₁-C₆ alkylC₃-C₈cycloalkyl, phenyl, -OC₁-C₆ alkylC₃-C₈cycloalkyl, -OC₁-C₆ alkylaryl, -OC₁-C₆ alkylheterocyclic, and C₁-C₆ alkylaryl;
R² is independently selected from hydrogen, C₁-C₆alkyl and C₃-C₈cycloalkyl;
R³ is hydrogen, C₁-C₆ alkyl, aryl, C₂-C₆ alkenyl, C₂-C₆ alkynyl, C₁-C₆ alkylaryl, C₁-C₆ alkylheterocyclic, C₃-C₈ cycloalkyl, or C₁-C₆ alkylC₃-C₈cycloalkyl;
R⁵ is selected from hydrogen, halogen, hydroxy, C₁-C₆haloalkyl, C₁-C₆alkyl, C₂-C₆alkenyl, C₁-C₆alkylheterocyclic, C₁-C₆alkylaryl, aryl, -OC₁-C₆alkyl, -O-aryl, -OC₂-C₆alkenyl, -OC₁-C₆haloalkyl, NR⁷R⁸, -CH₂NR⁷R⁸, -CN, -COOH and NO₂;
R⁶ is independently selected from hydrogen, C₁-C₆ alkyl, C₂-C₆ alkenyl, hydroxy, -OC₁-C₆ alkyl, -O-aryl, -OC₂-C₆ alkenyl, C₁-C₆ haloalkyl, -OC₁-C₆ haloalkyl, C₁-C₆ alkylNR⁷R⁸, C₃-C₈ cycloalkyl, and C₁-C₆ alkylC₃-C₈cycloalkyl;
R⁷ and R⁸ are independently selected from hydrogen, C₁-C₆ alkyl, C₂-C₆ alkenyl, C₃-C₈ cycloalkyl, C₁-C₆ alkylC₃-C₈cycloalkyl, C₁-C₆ alkylheterocyclic, heterocyclic, aryl, C₁-C₆ alkylaryl, hydroxy, oxo, COOH, C(O)OC₁-C₄ alkyl, C₂-C₆ alkynyl, C₁-C₆ alkoxy, C₁-C₆ haloalkyl, C₁-C₆ alkylalcohol, C₁-C₆ alkylamine, C₂-C₆ alkenylaryl, C₂-C₆ alkynylaryl, C₁-C₆ alkyl-O-C₁-C₆ alkylaryl, C₁-C₆ alkyl-NR¹¹-C₁-C₆ alkylaryl, C₁-C₆ alkylcyano, C₁-C₆ alkylCONR⁷R⁸, C₁-C₆ alkylNR⁷R⁸ and C₁-C₆alkylNR¹¹COR¹², wherein each alkyl, cycloalkyl, heterocyclic, or aryl group is optionally substituted with 1 to 3 groups independently selected from hydroxy, oxo, amino, halogen, C₁-C₆ alkylC₃-C₈cycloalkyl, C₃-C₈ cycloalkyl, C₁-C₆ alkylheterocyclic, C₁-C₆ haloalkyl, COOH, C(O)OC₁-C₄ alkyl, C₁-C₆ alkyl, C₂-C₆ alkenyl, C₂-C₆ alkynyl, C₁-C₆ alkoxy, C₁-C₆ alkylalcohol, C₁-C₆ alkylamine and NR¹¹R¹²; or R⁷ and R⁸ combine to form a nitrogen containing heterocyclic ring which may have 0, 1, or 2 additional hetero-atoms selected from oxygen, nitrogen or sulfur and may be optionally substituted with oxo, or C₁-C₆ alkyl;
R⁹ is selected from C₁-C₆ alkyl, C₂-C₆ alkenyl, C₃-C₈ cycloalkyl, C₁-C₆ alkylcycloalkyl, aryl, heterocyclic, C₁-C₆ alkylheterocyclic, COR⁷, CO₂R⁷, C₀-C₃ alkylCONR⁷R⁸, C₀-C₃ alkylS(O)ₚNR⁷R⁸ and C₀-C₃ alkylS(O)ₚR⁷ wherein R⁷ is as defined above, and wherein each alkyl, cycloalkyl, aryl, and heterocyclic is optionally substituted with one to two groups independently selected from halo, hydroxy, oxo, COOH, C(O)OC₁-C₄ alkyl, C₁-C₆ haloalkyl, C₁-C₆ alkyl, C₂-C₆ alkenyl, C₂-C₆ alkynyl, C₁-C₆ alkoxy, C₁-C₆ alkylalcohol, C₁-C₆ alkylamine, C₁-C₆ alkylaryl, C₂-C₆ alkenylaryl, C₂-C₆ alkynylaryl, C₁-C₆ alkylheterocyclic, -NR⁷R⁸, C₃-C₈ cycloalkyl, C₁-C₆ alkylC₃-C₈cycloalkyl, C₁-C₆ alkyl-O-C₁-C₆ alkylaryl, C₁-C₆ alkylcyano, C₁-C₆ alkylCONR⁷R⁸, C₁-C₆ alkylNR⁷R⁸, C₁-C₆alkylNR¹¹COR¹², and aryl, wherein each cycloalkyl or aryl group is optionally substituted with halo, hydroxy, oxo, amino, COOH, C(O)OC₁-C₄ alkyl, C₁-C₆ haloalkyl, C₁-C₆ alkyl, C₂-C₆ alkenyl, C₂-C₆ alkynyl, C₁-C₆ alkoxy, C₁-C₆ alkylalcohol, and C₁-C₆ alkylamine;
R¹⁰ is selected from aryl, C₁-C₆ alkylaryl, C₂-C₆ alkenylaryl, C₂-C₆ alkynylaryl, C₁-C₆ haloalkylaryl, C₁-C₆ alkylheterocyclic, C₂-C₆ alkenylheterocyclic, C₁-C₆ alkylC₃-C₈cycloalkyl, C₃-C₈cycloalkyl and C₁-C₆ alkyl-O-C₁-C₆ alkylaryl, wherein each cycloalkyl, aryl, or heterocyclic group is optionally substituted with 1 to 3 groups independently selected from hydroxy, oxo, -SC₁-C₆ alkyl, C₁-C₆ alkyl, C₁-C₆ alkenyl, C₁-C₆ alkynyl, C₁-C₆ haloalkyl, halogen, C₁-C₆ alkoxy, aryloxy, C₁-C₆ alkenyloxy, C₁-C₆ haloalkoxyalkyl, C₀-C₆ alkylNR¹¹R¹², -OC₁-C₆ alkylaryl, nitro, cyano, -OC₁-C₆ haloalkyl, C₁-C₆ haloalkylalcohol, and C₁-C₆ alkylalcohol;
R¹¹ and R¹² are independently selected from hydrogen, C₁-C₆ alkyl, C₂-C₆ alkenyl, C₃-C₈ cycloalkyl, heterocyclic, aryl, and C₁-C₆ alkylaryl, wherein each aryl group is optionally substituted with 1 to 3 groups independently selected from halogen, C₁-C₆ alkylheterocyclic, and C₁-C₆ haloalkyl, or R¹¹ and R¹² combine to form a nitrogen containing heterocyclic ring which may have 0, 1, or 2 additional heteroatoms selected from oxygen, nitrogen or sulfur and is optionally substituted with oxo, or C₁-C₆ alkyl; or a pharmaceutically acceptable salt, enantiomer, racemate, diastereomer or mixture of diastereomers thereof.

The present invention provides a us of a compound according to any one of claims 1 to 9, or a pharmaceutically acceptable salt, enantiomer, racemate, diastereomer or a mixture of diastereomers thereof, for the manufacture of a medicament for the treatment or prevention of dyslipidemia.

The present invention provides a use of a compound according to any one of claims 1 to 9, or a pharmaceutically acceptable salt, enantiomer, racemate, diastereomer or a mixture of diastereomers thereof, for the manufacture of a medicament for the treatment of hyperlipidemia, atherosclerosis, coronary heart disease, elevated blood pressure, CHF, stroke, hypertension, hypertriglyceremia, diabetes, obesity, inflammatory diseases including dermatitis, arthritis and pain, and diseases of the central nervous system including dementia and cognitive disorders; and a use of a compound according to any one of claims 1 to 9, or a pharmaceutically acceptable salt, enantiomer, racemate, diastereomer or a mixture of diastereomers thereof, for the manufacture of a medicament for raising the level of plasma HDL cholesterol in a mammal; and a use of a compound according to any one of claims 1 to 9, or a pharmaceutically acceptable salt, enantiomer, racemate, diastereomer or a mixture of diastereomers thereof, for the manufacture of a medicament for lowering the level of plasma LDL cholesterol in a mammal.

The present invention provides a compound according to any one of claims 1 to 9 or a pharmaceutically acceptable salt, enantiomer, racemate, diastereomer or a mixture of diastereomers thereof for use as a medicament.

The present invention also provides: A compound according to any one of claims 1 to 9, or a pharmaceutically acceptable salt, enantiomer, racemate, diastereomer or a mixture of diastereomers thereof, for use in the treatment or prevention of dyslipidemia. A compound according to any one of claims 1 to 9, or a pharmaceutically acceptable salt, enantiomer, racemate, diastereomer or a mixture of diastereomers thereof, for use in the treatment of hyperlipidemia, atherosclerosis, coronary heart disease, elevated blood pressure, CHF, stroke, hypertension, hypertriglyceridemia, diabetes, obesity, inflammatory diseases including dermatitis, arthritis and pain, and diseases of the central nervous system including dementia and cognitive disorders; a compound according to any one of claims 1 to 9, or a pharmaceutically acceptable salt, enantiomer, racemate, diastereomer or a mixture of diastereomers thereof, for use in raising the level of plasma HDL cholesterol in a mammal; a compound according to any one of claims 1 to 9, or a pharmaceutically acceptable salt, enantiomer, racemate, diastereomer or a mixture of diastereomers thereof, for use in lowering the level of plasma LDL cholesterol in a mammal.

The present invention also provides a pharmaceutical composition comprising a compound of formula I or a pharmaceutically acceptable salt, enantiomer, racemate, diastereomer or mixture of diastereomers thereof, and a carrier.

### DETAILED DESCRIPTION OF THE INVENTION

The current invention provides novel compounds of formula I useful in modulating CETP activity.

The terms "modulation" or "regulating" would include, but not be limited to, up-regulation, down-regulation, inhibition, agonism, antagonism of the CETP receptor as appropriate to achieve HDL raising, or LDL lowering and the resulting biological sequelae from such intervention.

The phrase "diseases" or "diseases related to abnormal activity CETP" or "diseases mediated by CETP activity" refers to pathological states where atherosclerosis and/or other cardiovascular diseases are prone because of dyslipidemia and/or other risk factors and are therefore beneficially affected by modulation, particularly down-regulation, of CETP activity. These diseases include but are not limited to hyperlipidemia and its sequelae such as atherosclerosis, CHD, elevated blood pressure, CHF, stroke, hypertension, hypertriglyceremia, diabetes, obesity, inflammatory diseases including but not limited to dermatitis, arthritis, and pain, and diseases of the central nervous system including but not limited to dementia, cognitive disorders such as, for example, Alzheimer's disease.

The term "treatment" bears its usual meaning which includes prohibiting, inhibiting, ameliorating, halting, restraining, slowing or reversing the progression, or reducing the severity of a pathological symptom related to or resultant from the modulation of CETP activity, especially as related to raising plasma levels of HDL, or lowering LDL-cholesterol levels or raising the HDL/LDL ratio or controlling atherosclerosis, hyperlipidemia and/or hypercholesterolemia.

Generally, one of skill in the art is aware that valency must be conserved (complete) for all stable molecules. Therefore, the necessary implication that hydrogen atoms are necessary and available to complete valency in all structures including formula I unless expressly indicated otherwise, is imputed to the general knowledge of one of skill in the art.

General chemical terms used in the description of compounds herein described bear their usual meanings. For example, the term "C₁₋₆ alkyl," or "(C₁-C₆)alkyl" or "C₁-C₆ alkyl" refers to a straight or branched aliphatic chain of 1 to 6 carbon atoms including but not limited to methyl, ethyl, propyl, iso-propyl, n-butyl, pentyl, and hexyl. Unless otherwise stated, the term "alkyl" means C₁-C₆ alkyl. Similarly, the term "C₀-C₆ alkyl" implies an alkyl group as indicated wherein when the term C₀ applies, the alkyl group is not present, and the remaining groups attach directly to the substrate. For example C₀-C₆ alkylcyano is the cyano group when C₀ applies. The invention also contemplates that the term C₁-C₆ alkyl or C₂-C₆ alkenyl or similar terms also encompass the specified alkyl or alkenyl or similar group, which may be chiral, regio or steroisomeric. Such chiral or regio or stereoisomeric groups are also objects of the present invention.

The term "alkylaryl" refers to an alkyl group substituted by an aryl group. For example, C₁-C₆ alkylaryl indicates that a C₁-C₆ alkyl group is attached to the aryl group, and that the resulting C₁-C₆ alkylaryl is attached to the nucleus via the alkyl group. Preferred alkylaryl groups include phenylethyl (phenethyl), and benzyl.

The term "substituted phenyl" or "optionally substituted phenyl" refers to a phenyl group having one or more substituents selected from the group consisting of C₁-C₆ alkyl, C₁-C₆ alkoxy, hydroxy, COR⁷, -COOR⁷, C₀-C₆ alkylNR⁷R⁸, nitro, chloro, fluoro, bromo, iodo, C₁-C₆haloalkyl, C₁-C₆ haloalkoxyalkyl, and C₀-C₆ alkylheterocyclic.

The term "optionally substituted carbocyclic or heterocyclic ring" refers to a saturated or unsaturated, aromatic or non-aromatic five or six member ring having optional substituents selected from the group consisting of C₁-C₆ alkyl, C₁-C₆ alkoxy, hydroxy, COR⁷, -COOR⁷, C₀-C₆ alkylNR⁷R⁸, nitro, chloro, fluoro, bromo, iodo, C₁-C₆haloalkyl, C₁-C₆ haloalkoxyalkyl, and C₀-C₆ alkylheterocyclic.

The term "aryl" refers to a substituted or unsubstituted aromatic or beteroaromatic, or heterocyclic radical. Illustrative aryl groups include but is not limited to napthyl, quinolyl, tetrahydroquinolyl, indazolyl, pyrimidinyl, triazinyl, pyrazine, pyridazinyl, piperidyl, pyrrolidinyl, piperazinyl, morpholinyl, tetrahydrofuranyl, pyranyl, tetrazolyl, imidazolyl, 1,2,3-trazolyl, 1,2,4-triazolyl, oxadiazolyl, thiadiazolyl, thiazolyl, oxazolyl, isoxazolyl, isothiazolyl, pyrazolyl, imidazopyridine, benzimidazolyl, triazolone-yl, imidazolone-yl, imidazolidinone-yl, 2-furyl, 3-furyl, 2-thienyl 3- thienyl, 1-pyrrolyl, 2-pyrrolyl, 3-pyrrolyl, phenyl, 2-pyridyl, 3-pyridyl, 4-pyridyl, 1-naphthyl, 2-naphthyl, 2-benzofuryl, 3-benzofuryl, 4-benzofuryl, 5-benzofuryl, 6-benzofuryl, 7-benzofuryl, 2-benzothieny, 3-benzothienyl, 4-benzothienyl, 5-benzothienyl, 6-benzothienyl, 7-benzothienyl, 1-indolyl, 2-indolyl, 3-indolyl, 4-indolyl, 5-indolyl, 6-indolyl, tetrazole, imidazole, isoxazole, pyrazole, 7-indolyl, and isomers thereof. As used herein the term aryl also encompasses the benzyl group.

The term "C₃-C₈ cycloalkyl" or similar terms refer to a saturated carbocyclic ring having from 3 to 8 carbon atoms where the term "cycloalkyl" is used a carbocyclic ring having 3 to 8 carbon atoms is implied.

The term "carbocycle" as used herein refers to a cyclic group having only carbon and appropriate number of hydrogen atoms. The term encompasses groups such as cycloalkyl, cycloalkene, cycloalkylene, naphthyl, phenyl and the like.

The term "heterocycle", "heterocyclyl", or "heterocyclic" refers to a 5, 6, 7, 8, 9 or 10 member saturated, partially unsaturated, or aromatic, mono-cyclic or a bicyclic ring containing 1-5 heteroatoms selected from N, S or O, wherein said heterocycle is optionally substituted at carbon or nitrogen atom(s) unless otherwise specified. Most preferred heterocyclic groups include pyridinyl, pyrolidinyl, piperidinyl, hexamethyleneimmino, morpholino, thiophene, indolyl, quinolyl, isoquinolyl, and tetrazolyl.

As a corollary, the term "alkylheterocyclic" or "alkylheterocycle" is understood to mean that the alkyl group is attached to the heterocycle and the point of attachment to the molecular backbone or nucleus is the alkyl group. The term "alkyl" without a qualifier implies a C₁-C₆ alkyl group.

The term "haloalkoxyalkyl" as used herein include for example trifluoromethoxy, pentafluoroethoxy, trifluoroethoxy (OCH₂CF₃) and the like.

The term "Prodrugs" describes derivatives of the compounds of the invention that have chemically or metabolically cleavable groups and become by solvolysis or under physiological conditions the compounds of the invention, which are pharmaceutically active, in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives, such as, esters prepared by reaction of the parent acidic compound with a suitable alcohol, or amides prepared by reaction of the parent acid compound with a suitable amine. Simple aliphatic esters (e.g., methyl, ethyl, propyl, isopropyl, butyl, sec-butyl, tert-butyl) or aromatic esters derived from acidic groups pendent on the compounds of this invention are preferred prodrugs. Other preferred esters include morpholinoethyloxy, diethylglycolamide and diethylaminocarbonylmethoxy. In some cases it is desirable to prepare double ester type prodrugs such as (acyloxy) alkyl esters or ((alkoxycarbonyl)oxy)alkyl esters.

As used herein, the term "protecting group" refers to a group useful for masking reactive sites in a molecule to enhance the reactivity of another group or allow reaction at another desired site or sites following which the protecting group may be removed. Protecting groups are usually used to protect or mask groups including but not limited to -OH, -NH, and -COOH. Suitable protecting groups are known to one of skill in the art and are described in Protecting groups in Organic Synthesis, 3rd edition, Greene, T. W.; Wuts, P.G.M. Eds., John Wiley and Sons, New York, 1999.

As used herein, the term "solvate" is a form of the compound of the invention wherein a crystal or crystals of a compound of the invention have been formed from a stoichiometric or non-stoichiometric amount of the compound of formula I and a solvent. Typical solvating solvents include for example, water, methanol, ethanol, acetone and dimethylformamide.

In those instances where a compound of the invention possesses acidic or basic functional groups, various salts may be formed which are more water soluble and/or more physiologically suitable than the parent compound. Representative pharmaceutically acceptable salts, include but are not limited to, the alkali and alkaline earth salts such as lithium, sodium, potassium, calcium, magnesium, aluminum and the like. Salts are conveniently prepared from the free acid by treating the acid in solution with a base or by exposing the acid to an ion-exchange resin.

Included within the definition of pharmaceutically acceptable salts are the relatively non-toxic, inorganic and organic base or acid addition salts of compounds of the present invention. Base addition salts include for example, ammonium, quaternary ammonium, and amine cations, derived from nitrogenous bases of sufficient basicity to form salts with the compounds of this invention (see, for example, S. M. Berge, et al., "Pharmaceutical Salts," J. Phar. Sci., 66: 1-19 (1977)). Moreover, the basic group(s) of the compound of the invention may be reacted with suitable organic or inorganic acids to form salts such as acetate, benzenesulfonate, benzoate, bicarbonate, bisulfate, bitartrate, borate, hydrobromide, camsylate, carbonate, clavulanate, citrate, chloride, edetate, edisylate, estolate, esylate, fluoride, fumarate, gluceptate, gluconate, glutamate, glycolylarsanilate, hexylresorcinate, hydrochloride, hydroxynaphthoate, hydroiodide, isothionate, lactate, lactobionate, laureate, maleate, mandelate, mesylate, methylbromide, methylnitrate, methylsulfate, mucate, napsylate, nitrate, oleate, oxalate, palmitate, pantothenate, phosphate, polygalacturonate, salicylate, stearate, subacetate, succinate, tannate, tartrate, tosylate, trifluoroacetate, trifluoromethane sulfonate, and valerate. Preferred salts for the purpose of the invention include the hydrochloride salt, the hydrobromide salt, the bisulfate salt, the methane sulfonic acid salt, the *p*-toluenesulfonic acid salt, bitartrate, the acetate and the citrate salt.

A compound of the invention as illustrated by formula I may occur as any one of its positional isomers, stereochemical isomers or regio-isomers, all of which are objects of the invention. Certain compounds of the invention may possess one or more chiral centers, and thus, may exist in optically active forms. Likewise, when the compounds contain an alkenyl or alkenylene group, there exist the possibility of cis- and trans-isomeric forms of the compounds. The R- and S- isomers and mixtures thereof, including racemic mixtures as well as mixtures of enantiomers or cis- and trans- isomers, are contemplated by and within the purview of this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereo-specific reactions with starting materials that contain the asymmetric centers and are already resolved. Alternatively desired stereoisomers may be prepared by methods that lead to mixtures of the stereoisomers and subsequent resolution by known methods. For example, a racemic mixture may be reacted with a single enantiomer of some other compound i.e. a chiral resolving agent. This changes the racemic form into a mixture of stereoisomers and diastereomers, because they have different melting points, different boiling points, and different solubilities and can be separated by conventional means, such as crystallization.

### Preferred Embodiments of The Invention

Preferred n, m, p, and q
Preferably n is 0, or 1.
Preferably m is 0, 1, 2 or 3. More preferably m is 0, 1 or 2.
Preferably p is 1, or 2.
Preferably, q is 0, 1 or 2. More preferably q is 0 or 1.

### Preferred A ring

A preferred a ring is selected from pyridine, pyrimidine, pyrazine, pyridazine, 1,2,5-triazine, thiophene, furan, pyrrole, pyrazole, isoxazole, isothiazole, imidazole, oxazole, thiazole, and 1,2,3-triazole. More preferred is an A ring selected from the group consisting of pyridine, pyrazine, thiophene, pyrazole, isoxazole, oxazole, and thiazole. Most preferred A-ring is pyridine.

### Preferred R¹

A preferred R¹ groups wherein n is 0 and K is C=O is selected from hydroxy, -C₁-C₆ alkyl, -C₁-C₆ alkylC₃-C₈cycloalkyl, -C₁-C₆ alkylheterocyclic, -C₁-C₆ haloalkyl, C₁-C₆ alkylaryl, -OC₁-C₆ alkyl , -OC₃-C₈ cycloalkyl, -OC₁-C₆ alkylC₃-C₈cycloalkyl, -OC₁-C₆ alkylaryl, -OC₁-C₆ haloalkyl, OC₂-C₆alkylcyano, OC₁-C₆aklCO₂R¹¹ ,-OC₂-C₆alkylalcohol, -OC₁-C₆ alkylNR⁷R⁸ and -OC₁-C₆ alkylheterocyclic ; and wherein each cycloalkyl, aryl, or heterocyclic is optionally substituted with 1 or 2 groups selected from halo, C₁-C₃ alkylalcohol, C₀-C₃ alkylamine, C₀-C₃ alkylCOOH, -CONH₂ and Co-C₃alkylcyano. More preferred is an R¹ group selected from hydroxy, C₁-C₆ alkyl, C₁-C₆ alkylaryl, -C₁-C₆ alkylcycloalkyl, -C₁-C₆ alkylheterocyclic -OC₁-C₆ alkyl, -OC₀-C₆ alkylaryl, -OC₁-C₆ alkylcycloalkyl, -OC₁-C₆alkylcyano, -OC₁-C₆ alkylheterocyclic, -OC₁-C₆ alkylhydroxy, -OC₁-C₆ alkylNR⁷R⁸, -OC₁-C₆alkylCO₂R₁, heterocyclic, cycloalkyl, and -OC₀-C₆ alkylcycloalkylNR⁷R⁸ wherein each alkyl, cycloalkyl, heterocyclic and aryl groups are each optionally substituted as described herein. Most preferred is an R¹ group represented by -OC₁-C₆ alkyl

### Preferred R²

Most preferred is an R² group represented by hydrogen or C₁-C₆ alkyl, or C₃-C₈ cycloalkyl.

### Preferred R³ Groups

Preferably each R³ is hydrogen.

Also preferred is the R⁹ group CO₂R⁷ wherein R⁷ is as defined above.

### Preferred R⁵ groups

R⁵ is selected from hydrogen, halogen, hydroxy, C₁-C₆ haloalkyl, C₁-C₆ alkyl, C₂-C₆ alkenyl, C₁-C₆ alkylheterocyclic, C₁-C₆ alkylaryl, aryl, -OC₁-C₆ alkyl, aryloxy (-O-aryl), -OC₂-C₆ alkenyl, -OC₁-C₆ haloalkyl, -NR7R8 -CH₂NR⁷R⁸, -CN, -COOH, and NO₂;

More preferably, R⁵ is at each occurrence is independently selected from hydrogen, halogen, C₁-C₆ alkyl and C₁-C₃ haloalkyl.

### Preferred R⁶

R⁶ is preferably selected from hydrogen, C₁-C₆ haloalkyl, C₁-C₆ alkyl, C₂-C₆ alkenyl, aryloxy, -OC₂-C₆ alkenyl and -CH₂NR⁷R⁸.
More preferably, R⁶ is at each occurrence independently selected from hydrogen and C₁-C₆ alkyl.

### Preferred R⁷ and R⁸

Preferred R⁷ and R⁸ are independently selected from hydrogen, C₁-C₆ alkyl, C₂-C₆ alkenyl, C₁-C₆ alkylaryl, and C₁-C₆alkylheterocyclic, wherein each aryl group is optionally substituted with 1-3 groups independently selected from C₁-C₆ alkyl, halo, and C₁-C₆ haloalkyl.

### Preferred R¹¹ and R¹²

Preferred R¹¹ and R¹² are independently selected from hydrogen, C₁-C₆ alkyl, C₂-C₆ alkenyl and C₁-C₆ alkylaryl, wherein each aryl group is optionally substituted with 1-3 groups independently selected from halo, and C₁-C₆ haloalkyl.

A most preferred compound of the invention is a compound selected from:
5-[Acetyl-(3,5-bis-trifluoromethyl-benzyl)-amino]-2,3,4,5-tetrahydro-thieno[3,4-b]azepine-1-carboxylic acid isopropyl ester,
8-[Acetyl-(3,5-bis-trifluoromethyl-benzyl)-amino]-3-methyl-5,6,7,8-tetrahydrothieno[3,2-b]azepine-4-carboxylic acid isopropyl ester,
8-[Acetyl-(3,5-bis-trifluoromethyl-benzyl)-amino]-2-bromo-5,6,7,8-tetrahydrothieno[3,2-b]azepine-4-carboxylic acid isopropyl ester,
5-[Acetyl-(3,5-bis-trifluoromethyl-benzyl)-amino]-5,6,7,8-tetrahydro-pyrido[2,3-b]azepine-9-carboxylic acid isopropyl ester,
5-[Acetyl-(3,5-bis-trifluoromethyl-benzyl)-amino]-2,3,4,5-tetrahydro-pyrido[3,4-b]azepine-1-carboxylic acid isopropyl ester,
5-[Acetyl-(3,5-bis-trifluoromethyl-benzyl)-amino]-2,3,4,5-tetrahydro-pyrido[4,3-b]azepine-1-carboxylic acid isopropyl ester,
9-[Acetyl-(3,5-bis-trifluoromethyl-benzyl)-amino]-6,7,8,9-tetrahydro-pyrido[3,2-b]azepine-5-carboxylic acid isopropyl ester,
9-[Acetyl-(3,5-bis-trifluoromethyl-benzyl)-amino]-2-trifluoromethyl-6,7,8,9-tetrahydropyrido[3,2-b]azepine-5-carboxylic acid isopropyl ester,
9-[Acetyl-(3,5-bis-trifluoromethyl-benzyl)-amino]-3-trifluoromethyl-6,7,8,9-tetrahydropyrido[3,2-b]azepine-5-carboxylic acid isopropyl ester,
4-[Acetyl-(3,5-bis-trifluoromethyl-benzyl)-amino]-1-methyl-4,5,6,7-tetrahydro-1H-1,2,8-triaza-azulene-8-carboxylic acid isopropyl ester,
9-[acetyl-(3,5-bis-trifluoromethylbenzyl)amino]-2-chloro-6,7,8,9-tetrahydro-pyrido[3,2-*b*]azepine-5-carboxylic acid isopropyl ester,
9-[acetyl-(3,5-bis-trifluoromethylbenzyl)amino]-2-methoxy-6,7,8,9-tetrahydropyrido[3,2-*b*]azepine-5-carboxylic acid isopropyl ester,
9-[acetyl-(3,5-bis-trifluoromethylbenzyl)amino]-2-bromo-6,7,8,9-tetrahydro-pyrido[3,2-*b*]azepine-5-carboxylic acid isopropyl ester,
9-[Acetyl-(3,5-bis-trifluoromethylbenzyl)amino]-2-dimethylamino-6,7,8,9-tetrahydropyrido[3,2-*b*]azepine-5-carboxylic acid isopropyl ester,
9-[Acetyl-(3,5-bis-trifluoromethylbenzyl)amino]-2-methyl-6,7,8,9-tetrahydro-pyrido[3,2-*b*]azepine-5-carboxylic acid isopropyl ester,
9-[Acetyl-(3,5-bis-trifluoromethylbenzyl)amino]-2-cyano-6,7,8,9-tetrahydro-pyrido[3,2-*b*]azepine-5-carboxylic acid isopropyl ester,
9-[Acetyl-(3,5-bis-trifluoromethylbenzyl)amino]-3-chloro-2-methoxy-6,7,8,9-tetrahydropyrido[3,2-*b*]azepine-5-carboxylic acid isopropyl ester,
9-[Acetyl-(3,5-bis-trifluoromethylbenzyl)amino]-3-chloro-2-ethoxy-6,7,8,9-tetrahydropyrido[3,2-*b*]azepine-5-carboxylic acid isopropyl ester,
9-[Acetyl-(3,5-bis-trifluoromethyl-benzyl)amino]-2-methyl-3-trifluoromethyl-6,7,8,9-tetrahydro-pyrido[3,2-*b*]azepine-5-carboxylic acid isopropyl ester,
9-[Acetyl-(3,5-bis-trifluoromethyl-benzyl)amino]-2-methyl-3-trifluoromethyl-6,7,8,9-tetrahydro-pyrido[3,2-*b*]azepine-5-carboxylic acid tert-butyl ester,
9-[(3,5-Bis-trifluoromethyl-benzyl)-2-methyl-2*H*-tetrazol-5-yl)-amino]-2-methyl-3-trifluoromethyl-6,7,8,9-tetrahydro-pyrido[3,2-*b*]azepine-5-carboxylic acid isopropyl ester,
9-[(3,5-Bis-trifluoromethyl-benzyl)-2-methyl-2*H*-tetrazol-5-yl)-amino]-2-methyl-3-trifluoromethyl-6,7,8,9-tetrahydro-pyrido[3,2-*b*]azepine-5-carboxylic acid *tert*-butyl ester,
(3,5-Bis-trifluoromethyl-benzyl)-(5-cyclopentylmethyl-2-methyl-3-trifluoromethyl-6,7,8,9-tetrahydro-5H-pyrido[3,2-b]azepin-9-yl)-(2-methyl-2H-tetrazol-5-yl)-amine,
(3,5-Bis-trifluoromethyl-benzyl)-(5-cyclopropylmethyl-2-methyl-3-trifluoromethyl-6,7,8,9-tetrahydro-5H-pyrido[3,2-b]azepin-9-yl)-(2-methyl-2H-tetrazol-5-yl)-amine,
(3,5-Bis-trifluoromethyl-benzyl)-(2-methyl-5-pyridin-3-ylmethyl-3-trifluoromethyl-6,7,8,9-tetrahydro-5H-pyrido[3,2-b]azepin-9-yl)-(2-methyl-2H-tetrazol-5-yl)-amine,
(3,5-Bis-trifluoromethyl-benzyl)-(2-methyl-5-pyridin-4-ylmethyl-3-trifluoromethyl-6,7,8,9-tetrahydro-5H-pyrido[3,2-b]azepin-9-yl)-(2-methyl-2H-tetxazol-5-yl)-amine,
3-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl}-benzoic acid,
4-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl }-benzoic acid,
5-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-yl}-3,3-dimethyl-pentanoic acid,
(4-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl }-cyclohexyl)-acedc acid,
(3,5-Bis-trifluoromethyl-benzyl)-(5-ethyl-2-methyl-3-trifluorometl yl-6,7,8,9-tetrahydro-5H-pyrido[3,2-b]azepin-9-yl)-(2-methyl-2H-tetrazol-5-yl)-amine,
5-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl}-thiophene-2-carboxylic acid,
2-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-yl}-ethanol,
(5-Benzyl-2-methyl-3-trifluoromethyl-6,7,8,9-tetrahydro-5H-pyrido[3,2-b]azepin-9-yl)-(3,5-bis-triflu oromethyl-benzyl)-(2-methyl-2H-tetrazol-5-yl)-amine,
(3,5-Bis-trifluoromethyl-benzyl)-(2-methyl-2H-tetrazol-5-yl)-(2-methyl-5-thiazol-2-ylmethyl-3-trifluoromethyl-6,7,8,9-tetrahydro-5H-pyrido[3,2-b]azepin-9-yl)-amine,
9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepine-5-carboxylic acid tetrahydrofuran-3-yl ester,
(3,5-Bis-trifluoromethyl-benzyl)-(2-methyl-5-pyridin-4-ylmethyl-3-trifluoromethyl-6,7,8,9-tetrahydro-5H-pyrido[3,2-b]azepin-9-yl)-carbamic acid methyl ester,
N-(3,5-Bis-trifluoromethyl-benzyl)-N-(2-methyl-5-pyridin-4-ylmethyl-3-trifluoromethyl-6,7,8,9-tetrahydro-5H-pyrido[3,2-b]azepin-9-yl)-acetamide
or a pharmaceutically acceptable salt, solvate enantiomer or diastereomer or mixture thereof.

The positional isomers and geometric isomers associated with the asymmetric carbon atoms of compounds of formula I are also contemplated to be within the scope of the current invention as useful for the treatment of diseases related to CETP modulation.

### Synthesis of Compounds of the Invention

The compounds of the instant invention can be synthesized as exemplified in the following schemes. Aryl amino ester intermediates of Formula 1 can be chemically prepared, for example, by following the synthetic routes set forth in the Schemes below. However, the following discussion is not intended to be limiting to the scope of the present invention in any way. The reagents and starting materials are readily available to one of ordinary skill in the art. Other necessary reagents and starting materials may be made by procedures which are selected from standard techniques of organic and heterocyclic chemistry, techniques which are analogous to the syntheses of known structurally similar intermediates or starting materials and the procedures described in the preparations and examples below, including any novel procedures. Such known procedures include, but are not limited to, esterification of a carboxylic acid, hydrolysis of a nitrile to a carboxylic acid, and subsequent esterification. In addition, one of ordinary skill will appreciate that many of the necessary reagents or starting materials can be readily obtained from commercial suppliers or custom synthesis groups. The R, R¹, R², R³, R⁵, R⁶, W, X, Y, Z, etc, used within this section for the purpose of illustrating the various methods of synthesizing compounds of the invention are not necessarily synonymous in scope or meaning with similar groups used in the generic structure for compounds of formula I, assuming W, X, Y, Z do not all equal carbon. However, groups in similar positions are co-extensive in scope and meaning compared to groups occupying similar positions as defined for the generic structure of compounds of formula I. 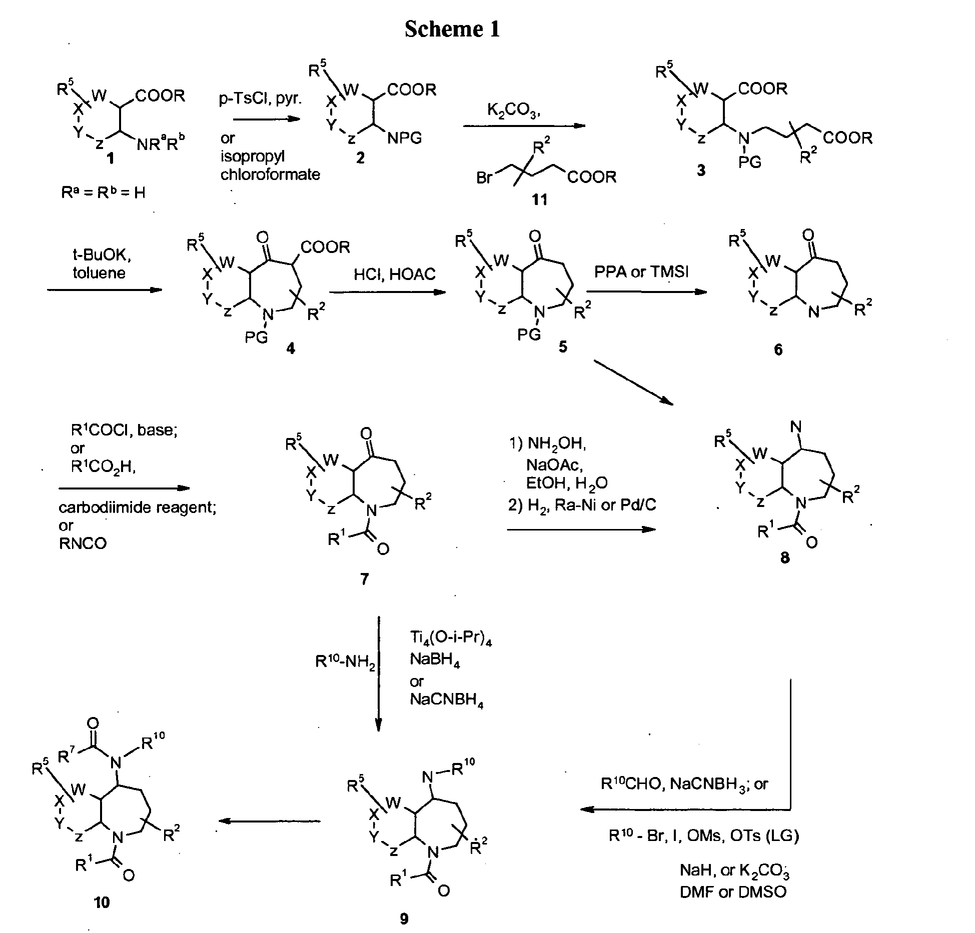 Synthetic scheme 1 shows preparation of compounds of formula I wherein n is 0. For example, substituted heteroarylamino esters 1 that are either commercially available or prepared as set forth in the literature or in Schemes 1a to 1d can be protected with tosyl chloride, isopropyl chloroformate, or other suitable protecting group to provide **2.** The compound **2** may in turn be alkylated with appropriately substituted, or unsubstituted 3-bromopropanoic acid esters **11** thus affording **3.** Dieckmann condensation-cyclization of intermediate **3** yields N-protected heteroarylazepine **4,** which is subjected to acid hydrolysis and decarboxylation to afford heteroarylazepin-5-one derivatives **5.** Removal of the protecting group, if necessary, with acid (e.g. PPA (polyphosphoric acid)), TMSI (trimethylsilyliodide), or HCl provides the intermediate heteroarylazepin-5-one **6.** Alternatively, utilizing the same conditions to effect **7** to **8,** one can proceed directly to **8** without deprotection.

N-acylation of **6** by treatment with an appropriately substituted aryl or alkyl chloroformate in the presence of an organic base such as pyridine affords carbamates of structure 7. Alternatively, treatment of 6 with an acid chloride or an appropriate activated ester, such as those generated *in-situ* from the reaction of an appropriately substituted aryl or alkyl carboxylic acid affords compounds of formula **7.**

Generation of urea derivatives from **6** is accomplished by treatment with a carbamoyl chloride in the presence of base such as pyridine and DMAP (dimethylamino pyridine) or an alternative base such as NaH in DMF. Alternatively, treatment with phosgene, or carbodiimide (CDI) reagent such as cyclohexylcarbodiimide or analog thereof, followed by the addition of an appropriately di-substituted amine will afford ureas of structure **7.** Formation of sulfonamide derivatives from **6** can be accomplished by reaction with appropriately substituted sulfonyl chlorides in the presence of a base.

Conversion of ketone **7** to **10** may be performed through direct reductive amination with an appropriately substituted alkylamine or aryl amine to afford compound **9.** Alternatively, compound **9** may be prepared through formation of the amine derivate **8** by reduction of an intermediate oxime, followed by alkylation with an appropriately substituted benzylic halide, mesylate or tosylate, or by reductive alkylation with the appropriate aldehyde or ketone in the presence of a reducing reagent such as NaCNBH₃. Compound **9** is converted to **10** (a compound of the invention) by acylation with an appropriately substituted symmetrical anhydride or acid halides to afford amides. Reaction of compound **9** with chloroformates affords the corresponding carbamates, Reaction of **9** with isocyanates, carbamoyl chlorides, or appropriately substituted sulfonyl chlorides affords the corresponding urea or sulfonamides respectively.

The intermediate compound **(1)** may be prepared as shown and described in schemes la through 1d below:

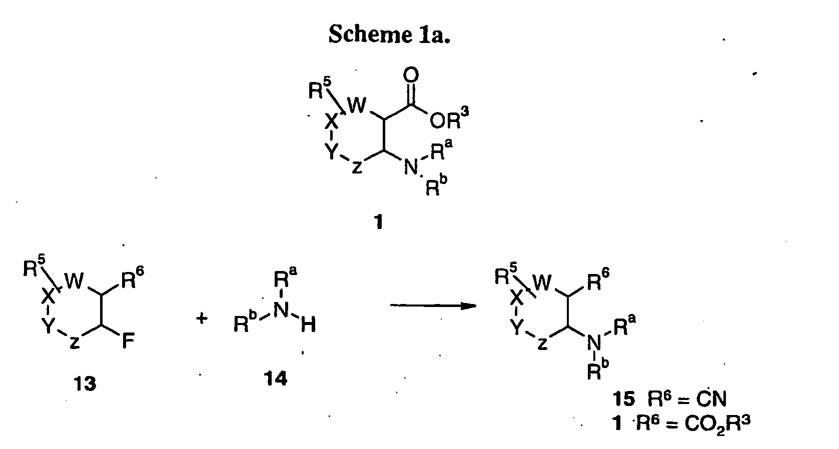

In scheme 1a, the nucleophilic aromatic substitution occurs by methods known in the art, (Wells, K. M. et al. Tetrahedron Letters, 1996, 37(36), 6439-6442). The appropriately substituted amine **14,** such as benzylamine, is dissolved in a suitable solvent, such as DMF or DMSO. A base such as cesium carbonate is added. The appropriately substituted fluoro heterobenzoate or heterobenzonitrile **13** (R⁶ = CN or CO₂R³), such as methyl fluoronicotinate ester is also added. The reaction proceeds at 0 °C to elevated (up to about 150 °C) temperatures in anywhere from ten minutes to several days depending on the stability of the starting materials. The product of structure **15** (R⁶ = CN) or 1 (R⁶ = CO₂R₃) can then be isolated by a standard aqueous workup, followed by normal phase chromatographic methods or recrystallization techniques commonly employed in the art.

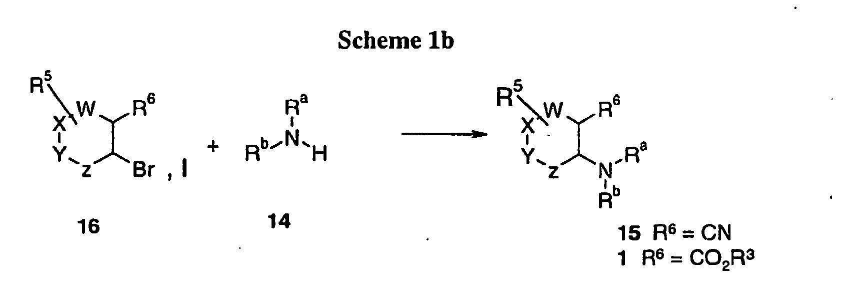

In scheme 1b, the N-Aryl coupling occurs by methods known in the art, (Hartwig, J. F. et al. Angew. Chem., Int. Ed. Engl. 1998,37,2046-2067). The appropriately substituted amine **14** is dissolved in a suitable solvent, such as DMF. A base, such as cesium carbonate or sodium *tert*-butoxide, the appropriately substituted halogenated heterobenzoate or heterobenzonitrile **16** (R⁶ = CN or CO₂R³), and a suitable catalyst complex, such as palladium acetate and diphenyl phospino ferrocene ligand are added. The reaction proceeds at 0 °C to elevated temperatures (up to 150 °C) in anywhere from ten minutes to several days depending on the stability of the starting materials. The product of structure **15** (R⁶ = CN) or **1** (R⁶ = CO₂R³) can then be isolated by a standard aqueous workup, followed by normal phase chromatographic methods or recrystallization techniques commonly employed in the art.

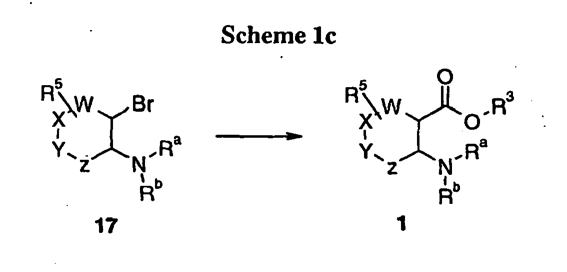

In scheme 1c, the carbonylation occurs by methods known in the art, (Heck, Palladium Reagents in Organic Synthesis; Academic Press: New York, 1985, p. 348-358). The appropriately substituted heteroaryl bromide 17 is dissolved in a suitable solvent, such as DMF, followed by addition of a base, such as cesium carbonate or sodium *tert*-butoxide/ A suitable catalyst complex, such as palladium acetate and diphenyl phospino ferrocene, an appropriate alcohol (R³-OH) are added. The reaction mixture is then saturated with carbon monoxide. The reaction proceeds at 0 °C to elevated temperatures (up to about 150°C) in anywhere from ten minutes to several days depending on the stability of the starting materials. The reaction may also be preformed under pressure using procedures known to one of skill in the art. The product of structure **1** may then be isolated by a standard aqueous workup, optionally followed by normal phase chromatographic methods or recrystallization techniques commonly employed in the art.

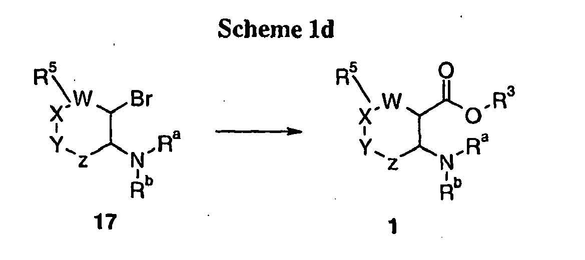

In scheme 1d, the aromatic carboxylation occurs by methods known in the art, (Boger, D. L. et al, Journal of Organic Chemistry, 1994, 59(17), 4943-4949, Volpin et al, Organomet. Reactions, 1975, 5, 313-386). The appropriately substituted heteroaryl bromide **17** is dissolved in a suitable solvent, such as diethyl ether or tetrahydrofuran. An alkyl lithium, such as n-butyl lithium or *tert*-butyl lithium or magnesium turnings is added. The resulting anion is quenched with a suitable carbon dioxide source, such as dry ice, or dimethyl carbonate. The reaction proceeds at -78 °C to room temperature in anywhere from about five minutes to several hours depending on the stability of the starting materials. The product of structure **1** can then be isolated by a standard aqueous workup, followed by normal phase chromatographic methods or recrystallization techniques commonly employed in the art.

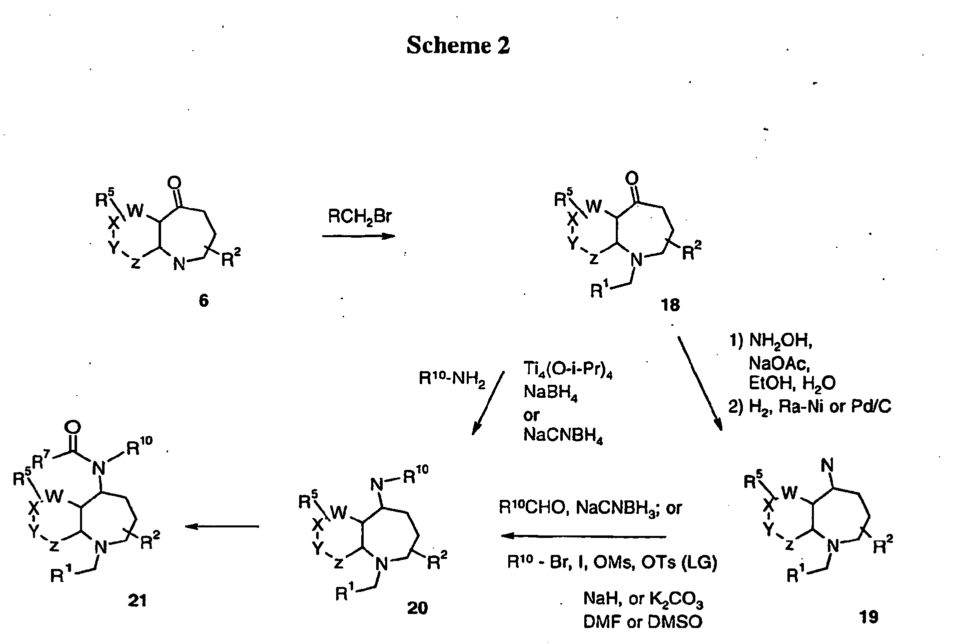

In scheme 2, the ring nitrogen of compound **6** can be alkylated by methods known in the art (Tetrahedron, 2002, 58 (43), 8719-8727) such as by treating the appropriately substituted heterobenzazapine, **6,** with a base such as sodium carbonate or sodium hydride, and an alkylating agent, such as methyl bromoacetate or chloroacetonitrile, to afford the intermediate **18.** The final product **21** can then be obtained according to the procedure described in scheme 1.

Compounds of formula I wherein R³ is not hydrogen may also be prepared as shown below in scheme 5.

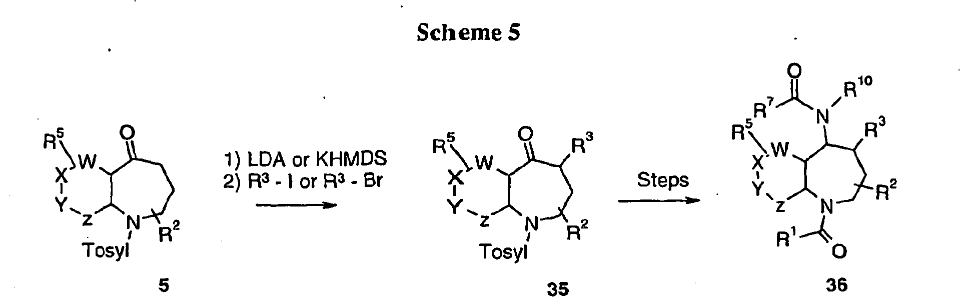

Installation of substituents alpha to the carbonyl of 1-heterobenzazapin-5-one 5 can be accomplished for example according to the method outlined in scheme 5 by enolate formation followed by alkylation with an Appropriate alkyl halide. Conversion of **35** to **36** is as described, for example in Scheme 1.

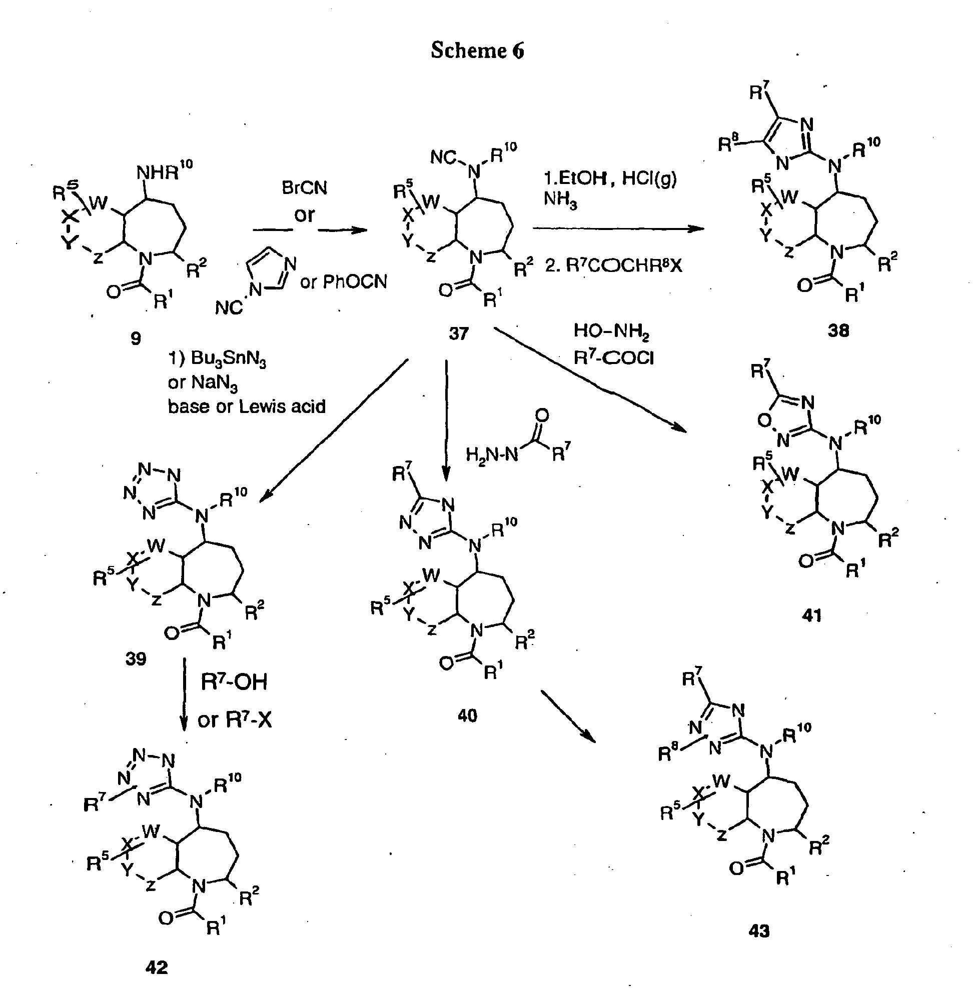

As shown in Scheme 6, amine **9** can be treated with for example cyanogen bromide or N-cyano imidazole in the presence or absence of base to form the N-cyano derivative **37.** The synthesis of imidazole **38,** tetrazole **39,** triazole **40** and oxadiazole **41** is illustrated in the scheme. Tetrazole **39** can be alkylated using the appropriate alcohol under Mitsunobu conditions, or with the appropriate alkyl iodide, mesylate, or the like in the presence of base to provide **42.** Triazole **40** can be alkylated using an appropriate alkyl iodide, mesylate, or the like in the presence of base to afford 43.

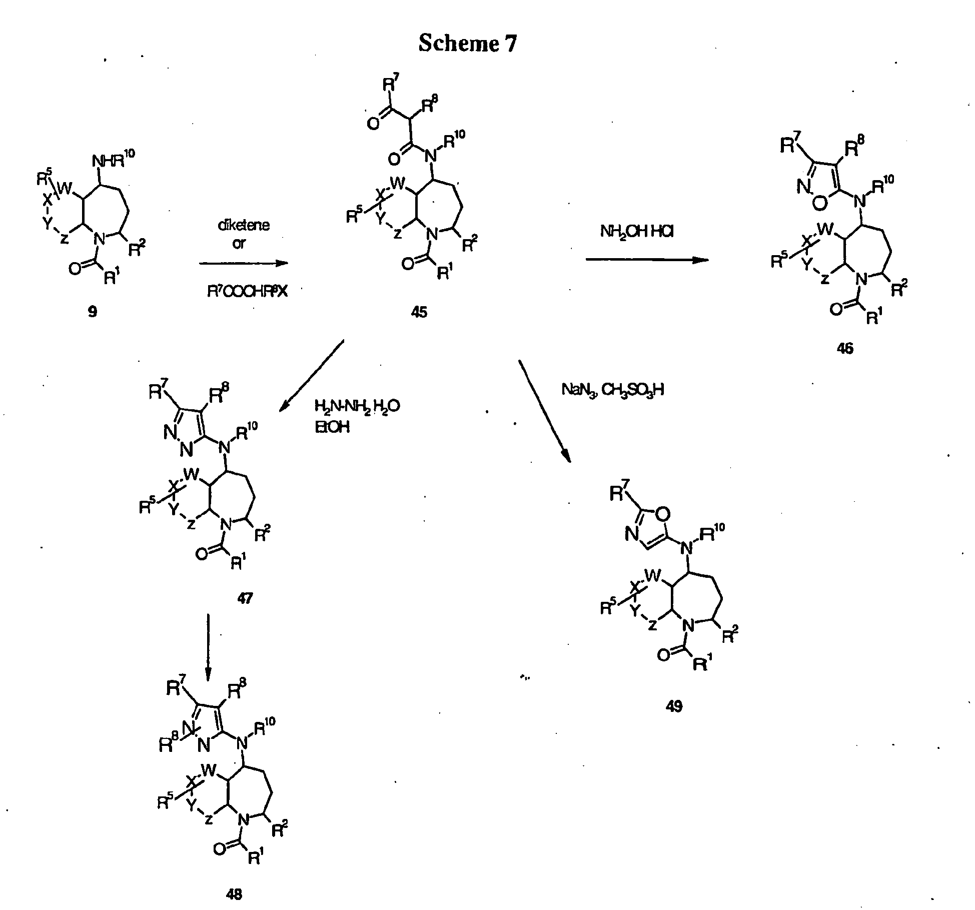

As shown in scheme 7 compound **9** can be transformed to **44** by reaction with diketene or a α-haloketone, further treatment with hydroxylamine hydrochloride can afford isoxazole **45.** Alternatively **45** can react with hydrazine in a solvent such as ethanol to afford pyrazole **47** that can be alkylated or acylated to give rise compound **48.** Or alternatively compound **45** can be converted into the oxazole **49** by treatment with sodium azide and methanesulfonic acid.

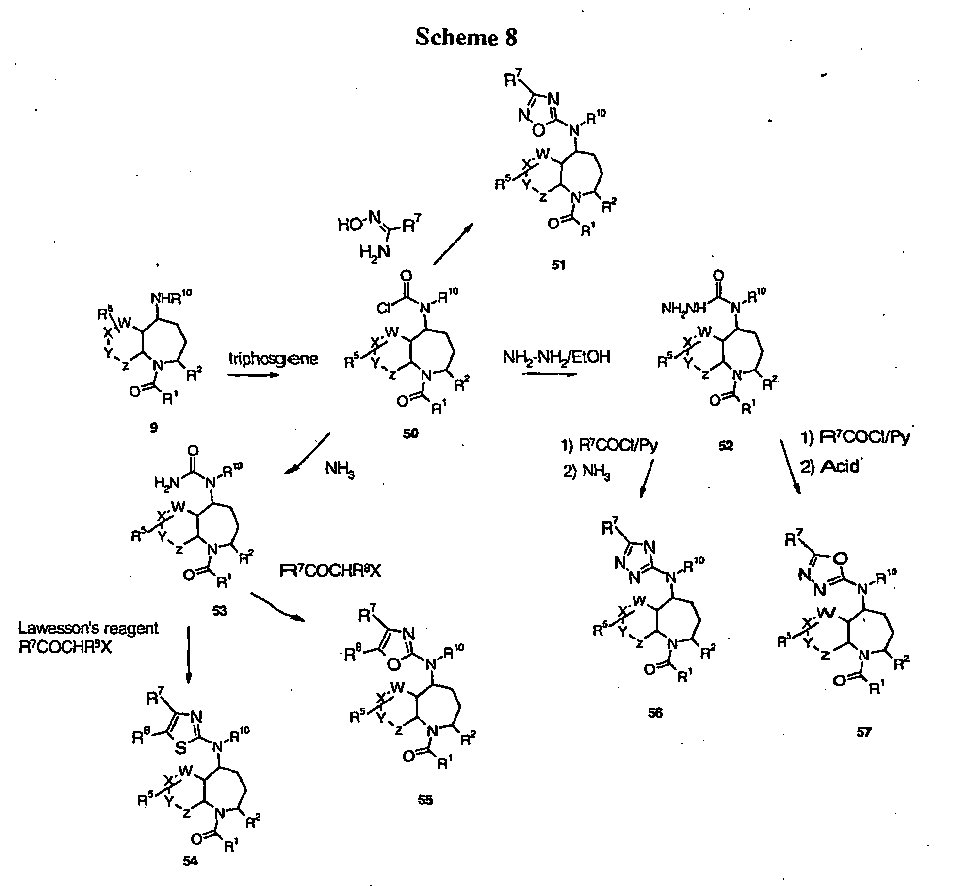

As shown in scheme 8, secondary amine **9** can be transformed in the acyl chloride **50** by treatment with triphosgene. Compound **50** may be converted into the oxadiazole **51** by reaction with an appropriate amidoxime. Alternatively **50** can be reacting with hydrazine to yield compound 53, that after treatment with an appropriate acyl chloride in a presence of ammonia can yield triazole **56** or in the presence of acid such as sulfuric acid and water can give rise the oxadiazole **57.** Compound **50** can be treated with ammonia to yield the ureido derivative **53** that can be transformed into oxazole **55** by reaction with an α-haloketone in the presence of a base. Alternatively compound **53** can be first converted to the corresponding thioamide with Lawesson's reagent and after reaction with a α-haloketone can afford the thiazole **54.**

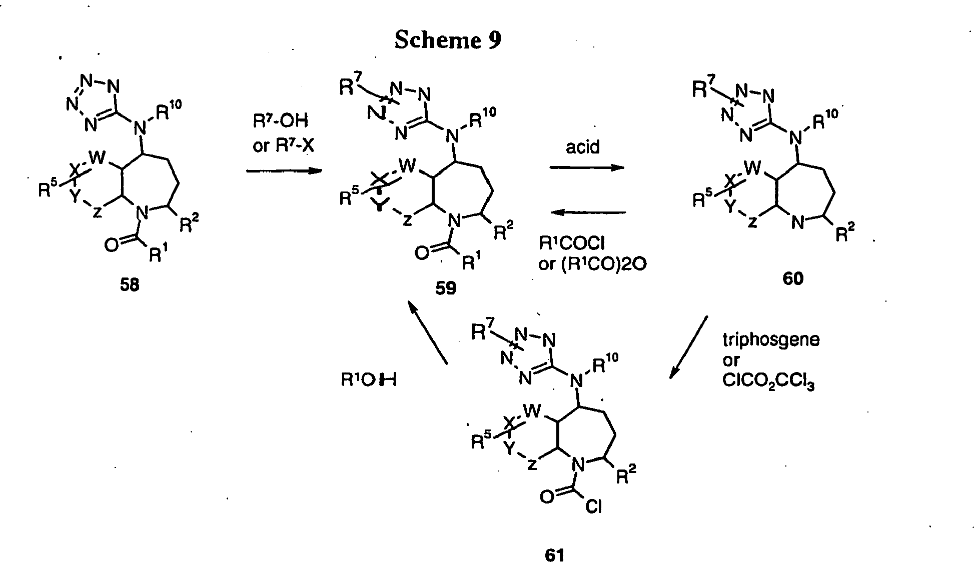

As shown in scheme **9,** compound **59** may be hydrolyzed to the corresponding amine **60,** and may be further acylated using standard procedures by one skilled in the art to provide **64.** Alteratively, **60** can be treated with triphosgene or trichloromethylchoroformate to provide **61.** Compound **61** can afford compound **59** by reaction with the appropriate alcohols.

### ASSAY

The following assay protocol and result(s) thereof demonstrating the utility and efficacy of the compounds and/or methods of the current invention are given for the purpose of illustration and are not meant to be limiting in any way.

### In Vitro Cetp Inhibitor Assay: Spa Assay

An in vitro Scintillation proximity assay (SPA) has been used to test the ability of compounds of this invention to inhibit the transfer of radiolabeled cholesterol esters between HDL and LDL. This assay monitors the inhibition of the transfer of [³H]cholesterol esters from HDL (Amersham) to biotinylated LDL (Amersham) by a CETP source. CETP produced by AV-12 cells that have been created to express human CETP has been used to mediate the transfer. After 30 minutes incubation in which the radiolabeled cholesterol ester is transferred in a HEPES-NaCl based buffer, the reaction is stopped and the biotinylated LDL is bound to streptavidin/scintillant coated SPA beads (Amersham). Then the radioactive signal is measured in a Packard 96-well scintillation TopCounter with window settings fully open. A decrease in radioactive signal represents the ability of compounds of the invention to inhibit the activity of CETP.

Alternatively, additional CETP sources can be used to mediate the transfer of radiolabeled cholesterol ester in this assay. Endogenous CETP from human plasma, CETP from mice made to express human CETP, and endogenous CETP from hamsters can be used as the CETP source in this assay.

Alternatively, other sources may be used as the buffer. In addition to the HEPES-NaCl based buffer that has been used in this assay, human plasma, mouse plasma or a Tris-bufer that is high in albumin may be used as the buffer in which the transfer of radiolabeled cholesterol esters from HDL to LDL may occur.

Alternatively, other sources of radioactivity may be used to track the CETP activity in this assay. In yet another alternative, radiolabeled-LDL may be used in this assay.

Compounds of the present invention tested have shown inhibition of CETP activity below about 100 micromolar when subjected to the SPA assay procedure above.

### Assay Of Cetp Activity In Vivo.

Syrian Golden Hamsters, which express endogenous CETP, are used to assess the activity of the compounds in vivo. Test compounds are administered orally in selected aqueous or oil based vehicles for up to one week. At various times after dosing, ranging from 4h to 48h, blood can be obtained. CETP activity is determined by a method similar to that described for the *in vitro* CETP activity assay, except that plasma from treated animals is used as the CETP source in the assay.

A strain of transgenic mice that express human CETP (Taconic, Germantown, NY) are used to test compounds of this invention. Test compounds are administered orally in selected aqueous or oil based vehicles for up to one week. At various times after dosing, ranging from 4h to 48h, blood can be obtained. CETP activity is determined by a method similar to that described for the *in vitro* CETP activity assay, except that plasma from treated animals is used as the CETP source in the assay.

Alternatively, a strain of transgenic mice that express both human CETP and human apolipoprotein A-1 (Taconic, Germantown, NY) are used to test compounds of this invention. Test compounds are administered orally in selected aqueous or oil based vehicles for up to one week. At various times after dosing, ranging from 4h to 48h, blood is obtained. CETP activity is determined by a method similar to that described for the *in vitro* CETP activity assay, except that plasma from treated animals is used as the CETP source in the assay.

### Assay Of Plasma Lipids In Vivo.

Activity of compounds of this invention *in vivo* can be determined by the level of elevation of HDL cholesterol relative to control by a given amount of compound in a CETP-containing animal species. A strain of transgenic mice that express both human CETP and human apolipoprotein A-1 (Taconic, Germantown, NY) is used to test compounds of this invention. Test compounds are administered once orally in selected aqueous or oil based vehicles. At various times after dosing, ranging from 4h to 24h, blood is obtained. Blood is allowed to clot and serum, is obtained by centrifugation. HDL cholesterol levels in the serum is determined by HDL-C plus reagents (Roche/Hitachi, Indianapolis, IN) with a clinical chemistry analyzer (Roche/Hitachi, Indianapolis, IN). Additional serum lipids can be analyzed by enzymatic methods. Lipids in the VLDL, LDL and HDL fractions are analyzed by enzymatic methods after precipitation or size exclusion chromatography. An example of the elevation of HDL cholesterol levels at 8hr are summarized in table 1

**Table 1. Elevation of HDL cholesterol levels at 8 hr**

| Compound of Example No. | Single Oral Dose (mg/kg) | % HDL cholesterol increase |
|---|---|---|
| 14 | 30 | 91 |

The efficacy of compounds of the invention *in vivo* can also be determined utilizing Syrian Golden Hamsters. The compounds can be tested in hamsters made hypercholesterolemic by feeding a high fat high cholesterol diet for a minimum of two weeks or in non-hypercholesterolemic hamsters fed normal chow for two weeks. Test compounds can be administered orally in selected aqueous or oil based vehicles for up to 1 week. Serum can be obtained and lipids can be analyzed by enzymatic methods. Lipids in the VLDL, LDL and HDL fractions are analyzed by enzymatic methods after precipitation or size exclusion chromatography.

Alternatively, a strain of transgenic mice that express human CETP (Taconic, Germantown, NY) are used to test the efficacy of the compounds of this invention. The hCETP mice can be made hypercholesterolemic by feeding a high fat chow diet such as TD 88051, as described by Nishina et al. (J Lipid Res., 31, 859-869 (1990)) for at least two weeks before the start of the study. Test compounds can be administered orally in selected aqueous or oil based vehicles for up to 1 week. Serum can be obtained and lipids can be analyzed by enzymatic methods. Lipids in the VLDL, LDL and HDL fractions are analyzed by enzymatic methods after precipitation or size exclusion chromatography.

### Method of Treatment

As used herein, the term "effective amount" means an amount of compound of the present invention, i.e., formula I, which is capable of alleviating the symptoms of the various pathological conditions herein described. The specific dose of a compound administered according to this invention will, of course, be determined by the particular circumstances surrounding the case including, for example, the compound administered, the route of administration, the state of being of the patient, and the pathological condition being treated. A typical daily dose will contain a nontoxic dosage level of from about 0.01 mg to about 100 mg/day of a compound of the present invention. Preferred daily doses generally will be from about 1 mg to about 250 mg/day.

The compounds of this invention can be administered by a variety of routes including oral, rectal, transdermal, subcutaneous, intravenous, intramuscular, and intranasal. These compounds preferably are formulated prior to administration, the selection of which will be decided by the attending physician. Thus, another aspect of the present invention is a pharmaceutical composition comprising an effective amount of a compound of Formula I, or a pharmaceutically acceptable salt thereof, solvate, prodrug, enantiomer or prodrug thereof, and a pharmaceutically acceptable carrier, diluent, or excipient.

The total active ingredients in such formulations comprises from 0.1% to 99.9% by weight of the formulation. By "pharmaceutically acceptable" it is meant the carrier, diluent, excipients and salt must be compatible with the other ingredients of the formulation, and not deleterious to the recipient thereof.

Pharmaceutical formulations of the present invention can be prepared by procedures known in the art using well-known and readily available ingredients. For example, the compounds of formula I can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl-pyrrolidone; moisturizing agents such as glycerol; disintegrating agents such as calcium carbonate and sodium bicarb onate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds also can be formulated as elixirs or solutions for convenient oral administration or as solutions appropriate for parenteral administration, for example, by intramuscular, subcutaneous or intravenous routes. Additionally, the compounds are well suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Compounds of formula I, generally, will be administered in a convenient formulation as determined by the attending physician. The following formulation examples are only illustrative and are not intended to limit the scope of the present invention.

### Formulations

In the formulations which follow, "Active Ingredient" means a compound of formula I, a salt, solvate, racemate, enantiomer diastereomer or mixture of diastereomers, or prodrug thereof, or a combination of a compound of formula I and other effective agent for the treatment or prevention of dyslipidemia or atherosclerosis.

**Formulation 1: Gelatin Capsules**

| Hard gelatin capsules are prepared using the following: | |
|---|---|
| Ingredient | Quantity (mg/capsule) |
| Active ingredient | 0.1-1000 |
| Starch, NF | 0 - 650 |
| Starch flowable powder | 0 - 650 |
| Silicone fluid 350 centistokes | 0 - 15 |

The formulation above may be changed in compliance with the reasonable variations provided.

A tablet formulation is prepared using the ingredients below:

**Formulation 2: Tablets**

| Ingredient | Quantity (mg/tablet) |
|---|---|
| Active ingredient | 2.5 - 1000 |
| Cellulose, microcrystalline | 200 - 650 |
| Silicon dioxide, fumed | 10 - 650 |
| Stearate acid | 5-15 |

The components are blended and compressed to form tablets.

Alternatively, tablets each containing 2.5 - 1000 mg of active ingredient are made up as follows:

**Formulation 3: Tablets**

| Ingredient | Quantity (mg/tablet) |
|---|---|
| Active ingredient | 25-1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders that are then passed through a No. 14 mesh U.S. sieve. The granules so produced are dried at 50-60 °C and passed through a No. 18 mesh U.S. sieve. The sodium carboxymethyl starch, magnesium stearate, and talc, previously passed through a No. 60 U.S. sieve, are then added to the granules, which after mixing, are compressed on a tablet machine to yield tablets.

Suspensions each containing 0.1 - 1000 mg of medicament per 5 ml dose are made as follows:

**Formulation 4: Suspensions**

| Ingredient | Quantity (mg/5 ml) |
|---|---|
| Active ingredient | 0.1-1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

An aerosol solution is prepared containing the following ingredients:

**Formulation 5: Aerosol**

| Ingredient | Quantity (% by weight) |
|---|---|
| Active ingredient | 0.25 |
| Ethanol | 25.75 |
| Propellant 22 (Chlorodifluoromethane) | 70.00 |

The active ingredient is mixed with ethanol and the mixture added to a portion of the propellant 22, cooled to 30 C, and transferred to a filling device. The required amount is then fed to a stainless steel container and diluted with the remaining propellant. The valve units are then fitted to the container.

**Formulation 6: Intravenous Solution**

| Ingredient | Quantity |
|---|---|
| Active ingredient | 50 mg |
| Isotonic saline | 1,000 mL |

The solution of the above ingredients is intravenously administered to a patient at a rate of about 1 mL per minute.

### Examples

The following examples are illustrative of compounds made or compounds that could be made by one of skill in the art following the teachings disclosed herein and known to one of skill in the art and requiring minimal experimentation. The disclosed examples should in no way limit the scope of the claims.

### Example 1

### 5-[Acetyl-(3,5-bis-trifluoromethyl-benzyl)-amino]-2,3,4,5-tetrahydro-thieno[3,4-b]azepine-1-carboxylic acid isopropyl ester

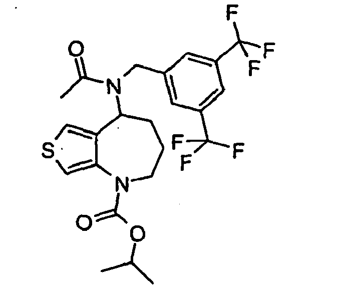

Step 1. Preparation of 4-Isopropoxycarbonylamino-thiophene-3-carboxylic acid methyl, ester 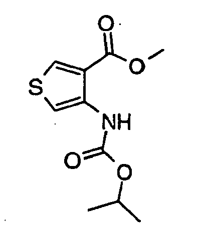 Combine methyl 3-aminothiophene-4-carboxylate hydrochloride (3.26 g, 16.8 mmol) and isopropyl chloroformate (1.0 N in toluene, 50.5 ml) in dichloromethane (100 ml) and add 2 N NaOH (100 mL). Stir the reaction mixture at room temperature for 4 h. Adjust to pH = 2 to 4 by adding 1 N HCl. After separating the layers, extract the aqueous layer with dichloromethane (100 ml). Wash the combined organic phases with brine (3 x 200 ml), dry over Na₂SO₄, filter, and concentrate. Purify using silica gel column chromatography (gradient eluent, 0-20% ethyl acetate in hexane) to provide 4-isopropoxycarbonylaminothiophene-3-carboxylic acid methyl ester (3.15 g, 77%) as an oil. MS (ES+): 244 (M+H).
Step 2. Preparation of 4-[Isopropoxycarbonyl-(3-methoxycarbonyl-propyl)-amino]-thiophene-3-carboxylic acid methyl ester 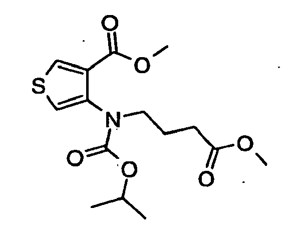 Suspend NaH (60% in mineral oil, 0.508 g, 12.7 mmol) in anhydrous DMF (50 ml) and cool the mixture to 0 °C. Inject a solution of 4-isopropoxycarbonylamino-thiophene-3-carboxylic acid methyl ester (3.10 g, 12.7 mmol) in DMF (50 ml) dropwise and then warm up the mixture to room temperature for an hour. Add methyl 4-bromobutyrate (3.57 g, 19.1 mmol), and then stir for 4 h at room temperature. Dilute the reaction mixture with ethyl acetate (200 mL) and wash with aqueous HCl (200 ml). Extract the aqueous portion with more ethyl acetate (100 ml). Combine the organic layers, wash with brine (3 x 300 ml), dry over Na₂SO₄, filter and concentrate. Purify using silica gel column chromatography (gradient eluent, 0-20% ethyl acetate in hexane) to provide 4-[isopropoxycarbonyl-(3-methoxycarbonyl-propyl)-amino]-thiophene-3-carboxylic acid methyl ester (2.38 g, 55%) as an oil. MS (ES+): 344 (M+H).
Step 3. Preparation of 5-Oxo-2,3,4,5-tetrahydro-thieno[3,4-b]azepine-1,4-dicarboxylic acid 1-isopropyl ester 4-methyl ester 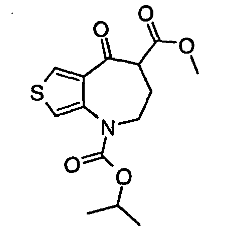 Inject a solution of 4-[isopropoxycarbonyl-(3-methoxycarbonyl-propyl)-amino]-thiophene-3-carboxylic acid methyl ester (2.30 g, 6.70 mmol) in toluene (100 ml) dropwise to a preheated solution of potassium t-butoxide (1.53 g, 13.4 mmol) in toluene (100 ml) at 70 °C. After the addition is complete, cool the mixture down to room temperature. Pour the reaction mixture into ice water (100 ml) and adjust the pH by adding 1 N HCl (15 ml). Separate the organic layer and extract the aqueous portion with ethyl acetate (2 x 100 ml). Combine the organic layers, wash with brine (3 x 300 ml), dry over Na₂SO₄, filter, and evaporate the solvents *in vacuo.* Purify using silica gel column chromatography (gradient eluent, 0-10% ethyl acetate in hexane) to give 5-oxo-2,3,4,5-tetrahydro-thieno[3,4-b]azepine-1,4-dicarboxylic acid 1-isopropyl ester 4-methyl ester (1.68 g, 80%) as an oil. MS (ES+): 312 (M+H); (ES-): 310 (M-H).
Step 4. Preparation of 5-Oxo-2,3,4,5-tetrahydro-thieno[3,4-b]azepine-1-carboxylic acid isopropyl ester 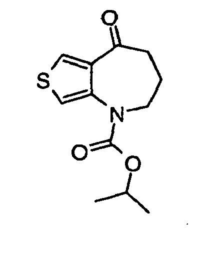 Add LiCl (0.300 g, 7.18 mmol) in one portion to a mixture of 5-oxo-2,3,4,5-tetrahydrothieno[3,4-b]azepine-1,4-dicarboxylic acid 1-isopropyl ester 4-methyl ester (0.930 g, 2.99 mmol) in DMSO (24 ml) and water (2 drops). Heat the mixture at 160 °C for 2 h. Cool the reaction to room temperature and partition between ethyl acetate (50 ml) and brine (50 ml). Separate the organic layer, wash with brine (3 x 50 ml), dry over Na₂SO₄, filter, and concentrate to an oil. Purify using silica gel column chromatography (gradient eluent, 0-15% EtOAc in hexane) to give 5-oxo-2,3,4,5-tetrahydro-thieno[3,4-b]azepine-1-carboxylic acid isopropyl ester (0.272 g, 36%) as an oil. MS (ES+): 254 (M+H).
Step 5. Preparation of 5-(3,5-Bis-trifluoromethyl-benzylamino)-2,3,4,5-tetrahydrothieno[3,4-b]azepine-1-carboxylic acid isopropyl ester 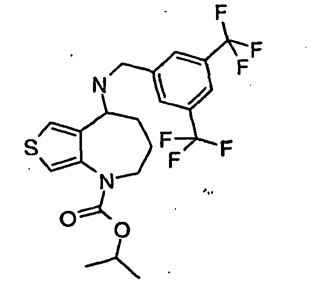 Inject titanium(IV)isopropoxide (0.390 ml, 1.33 mmol) to a mixture of 5-oxo-2,3,4,5-tetrahydro-thieno[3,4-b]azepine-1-carboxylic acid isopropyl ester (0.269 g, 1.06 mmol) and 3,5-bis(trifluoromethyl)benzylairiine (0.258 g, 1.06 mmol), and stir at room temperature for 16 h. Inject a solution of NaCNBH₃ (0.266 g, 4.24 mmol) in methanol (10 ml) to the reaction mixture and continue to stir at room temperature overnight. Treat the mixture with 0.1 N NaOH (25 ml) for 10 min, and then filter through a Celite® pad. Wash the filtered residue thoroughly with ethyl acetate. Separate the organic layer, wash with brine (3 × 50 ml), dry over Na₂SO₄, filter, and concentrate to provide crude 5-(3,5-bis-trifluoromethyl-benzylamino)-2,3,4,5-tetrahydro-thieno[3,4-b]azepine-1-carboxylic acid isopropyl ester (0.518 g) which was elaborated without further purification. MS (ES+):481 (M+H).
Step 6. Preparation of 5-[Acetyl-(3,5-bis-trifluoromethyl-benzyl)-amino]-2,3,4,5-tetrahydro-thieno[3,4-b]azepine-1-carboxylic acid isopropyl ester 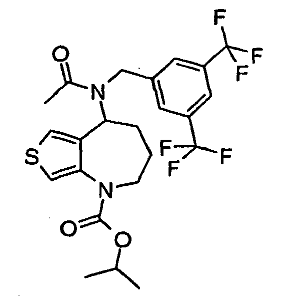 Inject acetic anhydride (0.40 ml, 4.24 mmol) dropwise to a solution of crude 5-(3,5-bis-trifluoromethyl-benzylamino)-2,3,4,5-tetrahydro-thieno[3,4-b]azepine-1-carboxylic acid isopropyl ester (0.277 g, 0.577 mmol) and pyridine (0.40 ml, 4.96 mmol) in dichloromethane (4 ml) at room temperature. Stir the mixture at room temperature for 16 h. Dilute the reaction mixture with ethyl acetate (50 mL), and wash with aqueous HCl (50 ml) and brine (3 x 50 ml). Dry over Na₂SO₄, filter, and concentrate to an oil. Purify using silica gel column chromatography (gradient eluent, 0-30% ethyl acetate in hexane) to provide the title compound (230 mg, white crystalline). MS (ES+): 523 (M+H).

### Example 2

### 8-[Acetyl-(3,5-bis-trifluoromethyl-benzyl)-amino]-3-methyl-5,6,7,8-tetrahydrothieno[3,2-b]azepine-4-carboxylic acid isopropyl ester

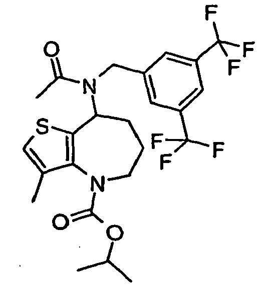

Prepare the title compound by essentially following the procedures described in Example 1, Steps 1-6, by replacing methyl 3-aminothiophene-4-carboxylate hydrochloride with methyl 3-amino-4-methylthiophene-2-carboxylate in Example 1, Step 1. MS (ES+): 537 (M+H).

### Example 3

### 4-[Acetyl-(3,5-bis-trifluorometbyl-benzyl)-amino]-1-methyl-4,5,6,7-tetrahydro-1H-1,2,8-triaza-azulene-8-carboxylic acid isopropyl ester

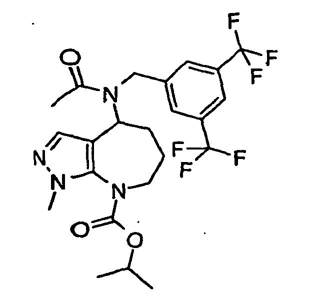

Prepare the title compound by essentially following the procedures described in Example 1, Steps 1-6, by replacing methyl 3-aminothiophene-4-carboxylate hydrochloride with 5-amino-1-methyl-1H-pyrazole-4-carboxylic acid ethyl ester in Example 1, Step 1. MS (ES+): 521 (M+H).

### Example 4

### Synthesis of (+/-)-9-[acetyl-(3,5-bis-trifluoromethylbenzyl)amino]-2-chloro-6,7,8,9-tetrahydro-pyrido[3,2-b]azepine-5-carboxylic acid isopropyl ester

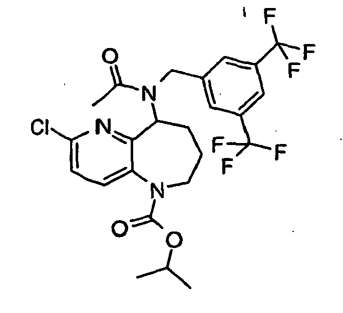

Step 1. Preparation of 6-chloro-3-nitro-pyridine-2-carbonitrile 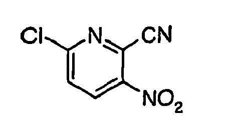 Combine 2,6-dichloro-3-nitro-pyridine (5.0 g, 25.9 mmol) and copper (I) cyanide (2.55 g, 28.5 mmol) in N-methyl pyrrolidinone (19 mL) and heat at 180 °C for 15 min. Cool the reaction mixture to room temperature, pour onto ice-water, and stir for 10 min. Separate the water and extract the oil with boiling toluene and then with ethyl acetate. Dry over sodium sulfate, filter, and concentrate under reduced pressure. Triturate the solid with diethyl ether and filter to afford the title compound (1.76 g, 39%). ¹H-NMR (CDCl₃, 300 MHz): δ 7.78 (d, *J* = 8.9 Hz, 1H), 8.54 (d, *J* = 8.9 Hz, 1H).
Step 2. Preparation of 3-amino-6-chloro-pyridine-2-carboxylic acid amide 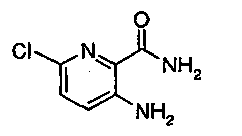 Combine 6-chloro-3-nitro-pyridine-2-carbonitrile (1.0 g, 5.45 mmol) and tin (II) chloride (5.0 g, 21.8 mmol) in ethanol (10 mL) and heat at 90 °C for 3 h. Evaporate the solvent under reduced pressure, suspend the residue in ethyl acetate and add a saturated solution of sodium bicarbonate until pH = 7 and then a solution of 2 N sodium hydroxide until pH = 8 to 9. Filter the precipitate through Celite® and extract the filtrate with ethyl acetate several times. Combine the organic layers, dry over sodium sulfate, filter, and concentrate under reduced pressure to afford the title compound (0.87 g, 93%). MS (ES+): 172 (M+H).
Step 3. Preparation of 3-amino-6-chloro-pyridine-2-carboxylic acid methyl ester hydrochloride 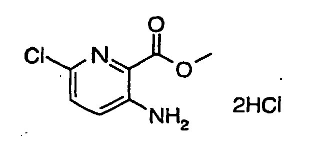 Combine 3-amino-6-chloro-pyridine-2-carboxylic acid amide (0.87 g, 5.09 mmol) and 35% hydrochloric acid (5 mL) and heat at 110 °C for 5 h. Evaporate the solvent under reduced pressure until dryness to afford 3-amino-6-chloro-pyridine-2-carboxylic acid hydrochloride (1.22 g, 98%). MS (ES+): 246 (M+H). Suspend 3-amino-6-chloropyridine-2-carboxylic acid hydrochloride (1.2 g, 4.89 mmol) in methanol (10 mL), add thionyl chloride (0.85 mL, 6.36 mmol) dropwise at room temperature and stir the mixture at 100 °C for 24 h. Remove the solvent under reduced pressure to afford the title compound (1.21 g, 95%). MS (ES+): 260 (M+H).
Step 4. Preparation of 6-chloro-3-isopropoxycarbonylamino-pyridine-2-carboxylic acid methyl ester 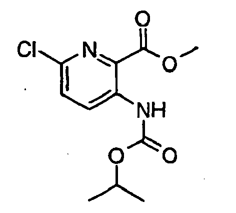 Add isopropyl chloroformate (3.43 mL, 3.53 mmol, 1.0 M in toluene) dropwise to a solution of 3-amino-6-chloro-pyridine-2-carboxylic acid methyl ester (600 mg, 3.21 mmol) and pyridine (0.508 mL, 6.42 mmol) in dichloromethane (6 mL) at 0°C under an atmosphere of nitrogen and stir at room temperature for 30 min. Add water and separate the layers. Extract the aqueous layer with dichloromethane. Dry the organic layers over anhydrous sodium sulfate, filter, and remove the solvent under reduced pressure, to afford the title compound (717 mg, 82%). ¹H-NMR (CDCl₃, 300 MHz): δ 1.27 (d, *J* = 6.5 Hz, 6 H); 3.97 (s, 3 H); 4.98 (sp, *J* = 6.0 Hz, 1 H); 7.43 (d, *J* = 8.9 Hz, 1H), 8.87 (d, *J* = 8.9 Hz, 1H); 10.2 (bs, 1 H).
Step 5. Preparation of 6-chloro-[(3-ethoxycarbonyl-propyl)-3-isopropoxycarbonylamino]-pyridine-2-carboxylic acid methyl ester 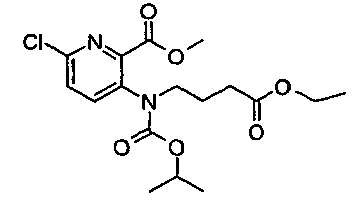 Add a solution of 6-chloro-3-isopropoxycarbonylamino-pyridine-2-carboxylic acid methyl ester (1.0 g, 3.68 mmol) in dimethylformamide (2 mL) to a suspension of sodium hydri de 60% in mineral oil (0.176 g, 4.42 mmol) in dry dimethylformamide (5 mL) at 0 °C under an atmosphere of nitrogen. Stir for 30 min at room temperature, cool to 0 °C and add ethyl 4-bromobutyrate (0.665 mL, 4.42 mmol). Stir the mixture at room temperature for 18 h. Pour the reaction mixture onto ice water and extract with diethyl ether. Dry the organic layer over anhydrous sodium sulfate, filter, and remove the solvent under reduced pressure. Purify the residue by flash chromatography, eluting with hexanes/ethyl acetate, to provide the title compound (696 mg, 49%). MS (ES+): 387 (M+H).
Step 6. Preparation of 2-chloro-9-oxo-6,7,8,9-tetrahydro-pyrido[3,2-*b*]azepine-5-carboxylic acid isopropyl ester 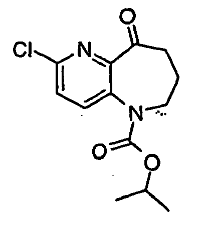 Add potassium *tert*-butoxide (1.9 M in tetrahydrofuran, 1.71 mL, 0.171 mmol) to a solution of 6-chloro-[(3-ethoxycarbonyl-propyl)-3-isopropoxycarbonyl-amino]-pyridine-2-carboxylic acid methyl ester (330 mg, 0.855 mmol) in toluene (15 mL) at 0 °C. Remove the bath and stir the reaction mixture at room temperature for 15 min. Add a saturated solution of ammonium chloride and extract with ethyl acetate. Dry the organic layer over anhydrous sodium sulfate, filter, and remove the solvent under reduced pressure. Dissolve the residue in dimethylsulfoxide (1.5 mL) and add water (1 drop) followed by addition of lithium chloride (128 mg, 3.02 mmol) and heat the resulting solution at 160 °C for 2 h. Cool the mixture to room temperature and pour onto brine. Extract the mixture with ethyl acetate and purify the residue over a silica gel cartridge eluting with hexanes/ethyl acetate (4:1), to afford the title compound (168 mg, 33%). MS (ES+): 331 (M+H). Also obtain 2-methoxy-9-oxo-6,7,8,9-tetrahydro-pyrido[3,2-*b*]azepine-5-carboxylic acid isopropyl ester (20 mg, 6%). MS (ES+): 279 (M+H).
Step 7. Preparation of 9-[acetyl-(3,5-bis-trifluoromethyl-benzyl)amino]-2-chloro-6,7,8,9-tetrahydro-pyrido[3,2-*b*]azepine-5-carboxylic acid isopropyl ester - 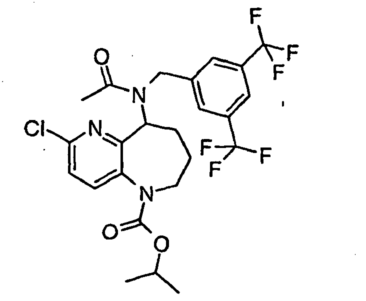 Add 3,5-bis(trifluoromethyl)benzylamine (190 mg, 0.623 mmol) followed by titanium isopropoxide (226 mg, 0.794 mmol) to 2-chloro-9-oxo-6,7,8,9-tetrahydro-pyrido[3,2-b]azepine-5-carboxylic acid isopropyl ester (160 mg, 0.567 mmol) at room temperature under an atmosphere of nitrogen and stir the solution for 15 h. Add methanol (2 mL) and sodium borohydride (33 mg, 0.851 mmol) and stir the mixture at room temperature for 30 min. Add 0.1 M sodium hydroxide (12 mL), stir for 1 h, filter through Celite® and wash the residue with diethyl ether and then with dichloromethane. Separate the organic layer and dry over anhydrous sodium sulfate. Filter and remove the solvent under reduced pressure. Dissolve the residue in dichloromethane (2 mL), add acetic anhydride (0.107 mL, 1.08 mmol), followed by pyridine (0.085 mL, 1.08 mmol), and stir at room temperature for 15 h. Dilute with ethyl acetate (3 mL) and wash with brine. Separate the organic layer, dry over sodium sulfate, and filter. Remove the solvent under reduced pressure and purify the residue by silica gel chromatography, eluting with ethyl acetate/hexanes to afford the title compound (37 mg, 31 %). MS (ES+): 552 (M+H).

### Example 5

### Synthesis of (+/-)-9-[acetyl-(3,5-bis-trifluoromethylbenzyl)amino]-2-methoxy-6,7,8,9-tetrahydro-pyrido[3,2-b]azepine-5-carboxylic acid isopropyl ester

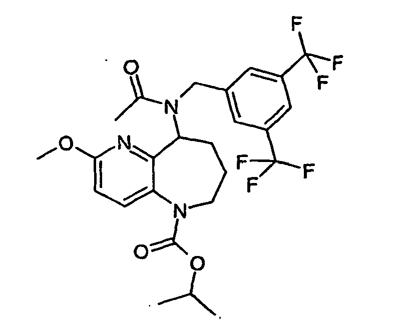

Prepare the title compound by essentially following the procedures described in Example 4, Step 7, by replacing 2-chloro-9-oxo-6,7,8,9-tetrahydro-pyrido[3,2-*b*]azepine-5-carboxylic acid isopropyl ester with 2-methoxy-9-oxo-6,7,8,9-tetrahydro-pyrido[3,2-*b*]azepine-5-carboxylic acid isopropyl ester (minor product from Example 4, Step 6). MS (ES+): 548 (M+H).

### Example 6

### Synthesis of (+/-)-9-[acetyl-(3,5-bis-trifluoromethylbenzyl)amino]-2-bromo-6,7,8,9-tetrahydro-pyrido[3,2-b]azepine-5-carboxylic acid isopropyl ester

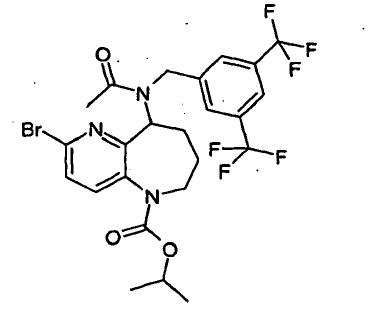

Step 1. Preparation of 3-amino-pyridine-2-carboxylic acid methyl ester 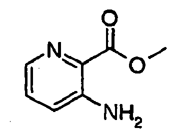 Add dropwise a solution of trimethylsilyl diazomethane (2.0 M in toluene, 7.24 mL, 14.48 mmol) to a solution of 3-amino-pyridine-2-carboxylic acid (1.0 g, 7.24 mmol) in ethyl acetate (5 mL) and methanol (5 mL) at room temperature. After the addition is complete, remove the solvent under reduced pressure. Suspend the residue in water (5 mL), add a saturated solution of sodium bicarbonate, and extract with ethyl acetate. Separate the organic layer, dry over sodium sulfate, and filter. Remove the solvent under reduced pressure to afford the title compound (590 mg, 54%). MS (ES+): 153 (M+H).
Step 2. Preparation of 3-amino-6-bromo-pyridine-2-carboxylic acid methyl ester 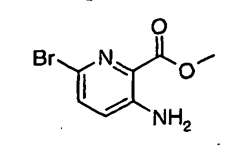 Add a solution of 2 M sulfuric acid (2 mL) to a suspension of 3-amino-pyridine-2-carboxylic acid methyl ester (0.59 g, 3.88 mmol) in water (10 mL) and stir the mixture until the precipitate is dissolved. Add dropwise a solution of bromine (0.199 mL, 3.88 mmol) in acetic acid (1.5 mL). Add a solution of 2 M sodium hydroxide until pH = 6 and extract with ethyl acetate. Separate the organic layer and extract with ethyl acetate. Dry the combined organic layers over anhydrous sodium sulfate, filter, and remove the solvent under reduced pressure. Purify the residue using silica gel chromatography eluting with ethyl acetate/hexanes to afford the title compound (0.616 g, 69%). ¹H-NMR (CDCl₃, 300 MHz): δ 3.90 (s, 3 H); 6.90 (d, *J* = 8.5 Hz, 1H), 7.30 (d, *J* = 8.5 Hz, 1H).
Step 3. Preparation of 6-bromo-3-isopropoxycarbonylamino-pyridine-2-carboxylic, acid methyl ester 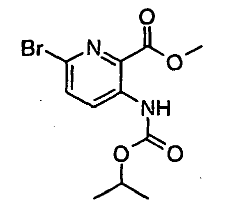 Prepare the title compound by essentially following the procedure described in Example 4, step 4, by replacing 3-amino-6-chloro-pyridine-2-carboxylic acid methyl ester with 3-anvno-6-bromo-pyridine-2-carboxylic acid methyl ester and stirring the mixture for 1 h at room temperature. MS (ES+): 316, 318 (M+H).
Step 4. Preparation of (+/-)-9-[Acetyl-(3,5-bis-trifluoromethylbenzyl)amino]-2-bromo-6,7,8,9-tetrahydro-pyrido[3,2-b]azepine-5-carboxylic acid isopropyl ester 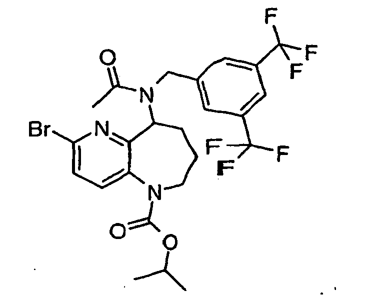 Prepare the title compound by essentially following the procedures described in Example 4, Steps 5 - 7, by replacing 6-chloro-3-isopropoxycarbonylamino-pyridine-2-carboxylic acid methyl ester with 6-bromo-3-isopropoxycarbonylamino-pyridine-2-carboxylic acid methyl ester in Example 4, Step 5. MS (ES+): 595, 597 (M+H).

### Example 7

### (+/-)-9-[Acetyl-(3,5-bis-trifluoromethylbenzyl)amino]-2-dimethylamino-6,7,8,9-tetrahydro-pyrido[3,2-b]azepine-5-carboxylic acid isopropyl ester

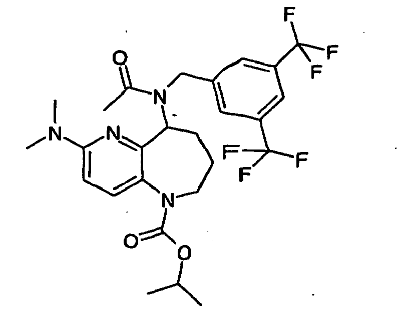

Add *N,N*-dimethylamine (40% in water, 0.4 mL) to a solution of (+/-)-9-[acetyl-(3,5-bis-trifluoromethylbenzyl)amino]-2-chloro-6,7,8,9-tetrahydro-pyrido[3,2-*b*]azepine-5-carboxylic acid isopropyl ester (45 mg, 0.082 mmol) in dimethylsulfoxide (0.12 mL) and heat the mixture at 100 °C in a sealed tube for 20 h. Cool the reaction mixture to room temperature, add water, and extract with ethyl acetate. Dry the organic layer over anhydrous sodium sulfate, filter, and remove the solvent under reduced pressure. Chromatograph the residue over a silica gel cartridge, eluting with hexanes/ethyl acetate to provide the title compound (39 mg, 85%). MS (ES+): 561 (M+H).

### Example 8

### (+/-)-9-[Acetyl-(3,5-bis-trifluoromethylbenzyl)amino]-2-methyl-6,7,8,9-tetrahydropyrido[3,2-b]azepine-5-carboxylic acid isopropyl ester

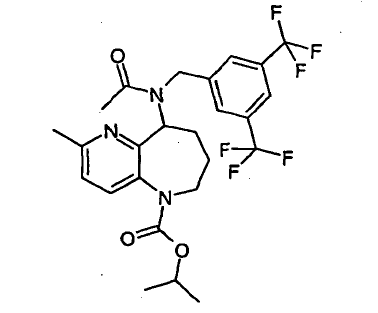

Add 1,1'-bis(diphenylphosphino)(II) chloride, complex with dichloromethane (10 mg, 0.012 mmol) to a suspension of (+/-)-9-[acetyl-(3,5-bis-trifluoromethylbenzyl)amino]-2-chloro-6,7,8,9-tetrahydro-pyrido[3,2-*b*]azepine-5-carboxylic acid isopropyl ester (44 mg, 0.080 mmol), methyl boronic acid (15 mg, 0.24 mmol) and cesium fluoride (42 mg, 0.28 mmol) in dry dioxane (1 mL) and heat the mixture at 80 °C in a sealed tube for 15 h. Cool the reaction mixture to room temperature, add water, and extract with dichloromethane. Dry the organic layer over anhydrous sodium sulfate, filter, and remove the solvent under reduced pressure. Chromatograph the residue over silica gel cartridge, eluting with hexanes/ethyl acetate to provide the title compound (34 mg, 79%). MS (ES+): 532 (M+H).

### Example 9

### (+/-)-9-[Acetyl-(3,5-bis-trifluoromethylbenzyl)amino]-2-cyano-6,7,8,9-tetrahydropyrido[3,2-b]azepine-5-carboxylic acid isopropyl ester

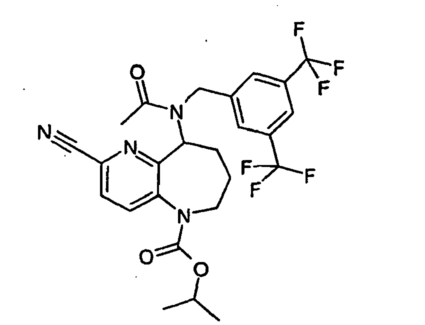

Combine (+/-)-9-[acetyl-(3,5-bis-trifluoromethylbenzyl)amino]-2-chloro-6,7,8,9-tetrahydro-pyrido[3,2-*b*]azepine-5-carboxylic acid isopropyl ester (45 mg, 0.081 mmol), zinc (II) cyanide (19 mg, 0.162 mmol) and tetrakis(triphenylphosphine) palladium (0) (9 mg, 0.0081 mmol) in dry dimethylformamide (1 mL) and heat at 120 °C for 15 h. Then add more zinc (II) cyanide (19 mg, 0.162 mmol) and tetrakis(triphenylphosphine) palladium (0) (9 mg, 0.0081 mmol) and stir the mixture for 48 h. Cool to room temperature, add water, and extract with ethyl acetate. Separate the organic layer, dry over sodium sulfate, filter and remove the solvent under reduced pressure. Purify the residue using silica gel chromatography, eluting with ethyl acetate/hexanes to afford the title compound (10 mg, 23%). MS (ES+): 543 (M+H).

### Example 10

### (+/-)-9-[Acetyl-(3,5-bis-trifluoromethylbenzyl)amino]-3-chloro-2-methoxy-6,7,8,9-tetrahydro-pyrido[3,2-b]azepine-5-carboxylic acid isopropyl ester

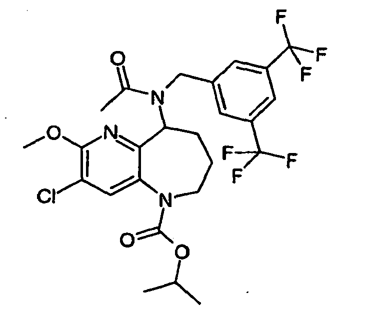

Step 1. Preparation of 5-Chloro-3-nitro-pyridine-2-carbonitrile 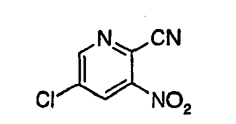 Add 3-nitro-5-chloro-pyridin-2-ol (3.9 g, 22.3 mmol) to a mixture of phosphorous oxychloride (4.17 mL) and dimethylformamide (10 mL). Heat the resulting mixture at 110 °C for 30 min. Cool the reaction mixture to room temperature and pour onto ice-water. Add sodium bicarbonate slowly until neutralization occurs and extract with ethyl acetate. Dry the organic layer over anhydrous sodium sulfate, filter, and remove the solvent under reduced pressure. Dissolve the residue in *N*-methyl pyrrolidinone (4 mL), add copper (I) cyanide (2.39 g, 26.7 mmol) and heat at 160 °C for 15 min. Cool the reaction mixture to room temperature and pout onto ice water and ethyl acet ate. Add a saturated solution of sodium bicarbonate, separate the organic layer, and extract the aqueous layer with ethyl acetate. Dry the combined organic layers over sodium sulfate, filter, and concentrate under reduced pressure. Purify the residue using silica gel chromatography, eluting with ethyl acetate/hexanes 1:3 to afford the title compound (1.01 g, 26%). ¹H-NMR (CDCl₃, 300 MHz): δ 8.61 (s, 1H), 8.95 (s, 1H).
Step 2. Preparation of 3-Amino-5-chloro-pyridine-2-carboxylic acid methyl ester 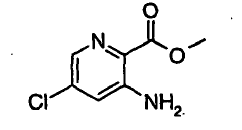 Heat at 90 °C a mixture of 5-chloro-3-nitro-pyridine-2-carbonitrile (1.0 g, 5.90 mmol) and tin (II) chloride (6.79 g, 29.5 mmol) in ethanol (10 mL) for 3 h. Evaporate the solvent under reduced pressure, add a solution of 35% hydrochloric acid (5 mL) and reflux the mixture for 6 h. Concentrate the reaction *in vacuo* to dryness and dissolve the resulting residue in methanol (20 mL). Add thionyl chloride (0.95 mL, 7.08 mmol) at room temperature and heat the mixture at 90 °C for 24 h. Remove the solvent under reduced pressure, add ethyl acetate, and wash with a saturated solution of sodium bicarbonate. Separate the organic layer, dry over sodium sulfate, filter, and concentrate under reduced pressure. Purify the residue using silica gel chromatography, eluting with ethyl acetate to afford the title compound (0.77 g, 70%). ¹H-NMR (CDCl₃, 3 00 MHz): δ 3.97 (s, 3 H); 5.85 (bs, 2 H); 7.06 (d, *J* = 2.0 Hz, 1H), 7.97 (d, *J* = 2.0 Hz, 1H).
Step 3. Preparation of 3-Amino-6-bromo-5-chloro-pyridine-2-carboxylic acid methyl ester 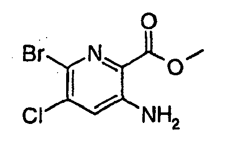 Prepare the title compound by essentially following the procedure described in Example 6, Step 2 by replacing 3-amino-pyridine-2-carboxylic acid methyl ester with 3-amino-5-chloro-pyridine-2-carboxylic acid methyl ester (2.15 mmol) and using 25 mL of sulfuric acid. ¹H-NMR (CDCl₃, 300 MHz): δ 3.90 (s, 3 H); 5.83 (bs, 2H); 7.13 (s, 1 H).
Step 4. Preparation of 6-Bromo-5-chloro-3-isopropoxycarbonylamino-pyridine-2-carboxylic acid methyl ester 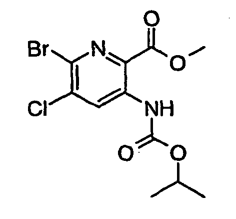 Prepare the tide compound by essentially following the procedure described in Example 4, Step 4 by replacing 3-amino-6-chloro-pyridine-2-carboxylic acid methyl ester with 3-anuno-6-bromo-5-chloro-pyridine-2-carboxylic acid methyl ester. ¹H-NMR (CDCl₃, 300 MHz): δ 1.27 (d, *J* = 6.5 Hz , 6 H); 3.96 (s, 3 H); 4.98 (septuplet, *J* = 6.5 Hz , 1 H); 9.03 (s, 1 H); 10.24 (bs, 1 H).
Step 5. Preparation of 6-Bromo-5-chloro-[(3-ethoxycarbonyl-propyl)-3-isopropoxycarbonyl-amino]-pyridine-2-carboxylic acid methyl ester 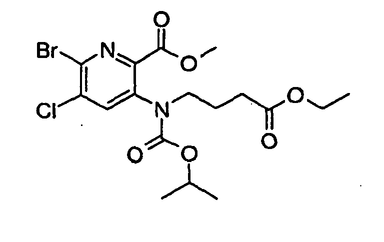 Prepare the title compound by essentially following the procedure described in Example 4, Step 5 by replacing 6-chloro-3-isopropoxycarbonylamino-pyridine-2-carboxylic acid methyl ester with 6-bromo-5-chloro-3-isopropoxycarbonylamino-pyridine-2-carboxylic acid methyl ester. MS (ES+): 464, 466 (M+H).
Step 6. Preparation of 3-Chloro-2-methoxy-9-oxo-6,7,8,9-tetrahydro-pyrido[3,2-*b*]azepine-5-carboxylic acid isopropyl ester 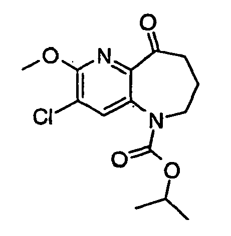 Prepare the title compound by essentially following the procedure described in Example 4, Step 6 by replacing 6-chloro-[(3-ethoxycarbonyl-propyl)-3-isopropoxycarbonylamino]-pyridine-2-carboxylic acid methyl ester with 6-bromo-5-chloro-[(3-ethoxycarbonyl-propyl)-3-isopropoxycarbonyl-amino]-pyridine-2-carboxylic acid methyl ester. MS (ES+): 313 (M+H). 3-Chloro-2-ethoxy-9-oxo-6,7,8,9-tetrahydro-pyrido[3,2-b]azepine-5-carboxylic acid isopropyl ester is also obtained during this process. MS (ES+): 327 (M+H).
Step 7. Preparation of (+/-)-9-[Acetyl-(3,5-bis-trifluoromethylbenzyl)amino]-3-chloro-2-methoxy-6,7,8,9-tetrahydro-pyrido[3,2-b]azepine-5-carboxylic acid isopropyl ester 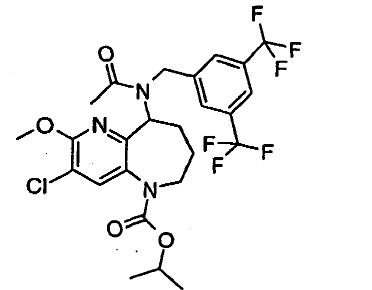 Prepare the title compound by essentially following the procedure described in Example 4, Step 7 by replacing 2-chloro-9-oxo-6,7,8,9-tetrahydro-pyrido[3,2-*b*]azepine-5-carboxylic acid isopropyl ester with 3-chloro-2-methoxy-9-oxo-6,7,8,9-tetrahydropyrido[3,2-*b*]azepine-5-carboxylic acid isopropyl ester. MS (ES+): 582 (M+H).

### Example 11

### (+/-)-9-[Acetyl-(3,5-bis-trifluoromethylbenzyl)amino]-3-chloro-2-ethoxy-6,7,8,9-tetrahydro-pyrido[3,2-b]azepine-5-carboxylic acid isopropyl ester

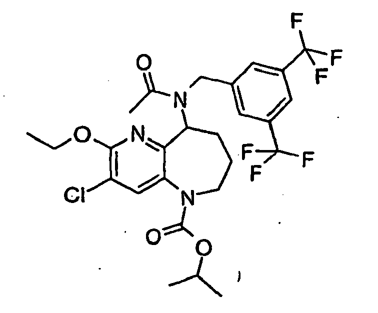

Prepare the title compound by essentially following the procedure described in Example 4, Step 7 by replacing 2-chloro-9-oxo-6,7,8,9-tetrahydro-pyrido[3,2-*b*]azepine-5-carboxylic acid isopropyl ester with 3-chloro-2-ethoxy-9-oxo-6,7,8,9-tetrahydropyrido[3,2-*b*]azepine-5-carboxylic acid isopropyl ester. MS (ES+): 596 (M+H).

### Example 12

### (+/-)-9-[Acetyl-(3,5-bis-trifluoromethyl-benzyl)amino]-2-methyl-3-trifluoromethyl-6,7,8,9-tetrahydro-pyrido[3,2-b]azepine-5-carboxylic acid isopropyl ester

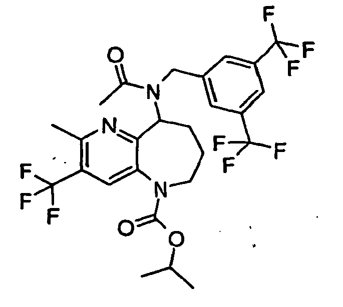

Step 1. Preparation of 2-Chloro-3-nitro-5-trifluoromethyl-pyridine 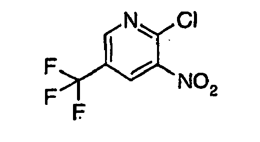 Add 3-nitro-5-trifluoromethyl-pyridin-2-ol (10.0 g, 48 mmol) to a mixture of phosphorous oxychloride (27 mL) and dimethylformamide (1.5 mL). Heat the resulting mixture at 110 °C for 30 min. Cool the reaction mixture to room temperature and pour onto ice-water. Add sodium carbonate slowly to pH = 7 and extract with dichloromethane. Dry the organic layer over anhydrous sodium sulfate, filter, and remove the solvent under reduced pressure to afford the title compound (10.6 g, 86%). ¹H-NMR (CDCl₃, 300 MHz) δ 9.10 (s, 1 H); 8.82 (s, 1 H).
Step 2. Preparation of 3-Nitro-5-trifluoromethyl-pyridin-2-carbonitrile 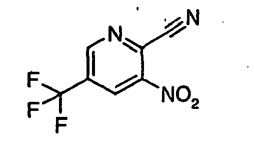 Add copper (I) cyanide (3.87 g, 43.28 mmol) to a solution of 2-chloro-3-nitro-5-trifluoromethylpyridine (10.5 g, 43.28 mmol) in *N*-methyl-2-pyrrolidinone (17 mL) and heat the mixture at 175 °C for 15 min. Cool the reaction mixture to room temperature, add ice, and stir for 15 min. Filter the precipitate through Celite®, washing with ethyl acetate and methanol. Separate the organic layer and extract the aqueous with ethyl acetate. Dry the combined organic layers over anhydrous sodium sulfate, filter, and remove the solvent under reduced pressure. Purify the residue using silica gel chromatography, eluting with ethyl acetate/hexanes to afford the title compound (5.30 g, 56%). ¹H-NMR (CDCl₃, 300 MHz): δ 9.30 (s, 1 H); 8.85 (s, 1 H).
Step 3. Preparation of 3-Amino-5-trifluoromethyl-pyridine-2-carboxylic acid methyl ester 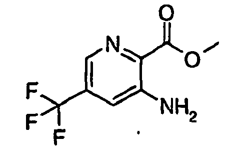 Prepare the title compound by essentially following the procedure described in Example 10, Step 2, by replacing 5-chloro-2-nitro-pyridine-2-carbonitrile with 3-nitro-5-trifluoromethyl-pyridin-2-carbonitrile. After removing the ethanol under vacuum, suspend the solid in ethyl acetate, filter through Celite®, and wash with a saturated solution of sodium bicarbonate. Combine the organic layers, dry over sodium sulfate, filter, and remove the solvent under vacuum. Then, follow the procedure described in Example 10, Step 2. MS (ES+): 221 (M+H).
Step 4. Preparation of 3-Amino-6-bromo-5-trifluoromethyl-pyridine-2-carboxylic acid methyl ester 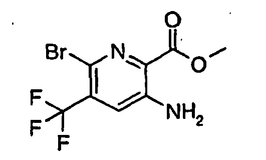 Prepare the title compound by essentially following the procedure described in Example 6, Step 2, by replacing 3-amino-pyridine-2-carboxylic acid methyl ester with 3-amino-5-trifluoromethyl-pyridine-2-carboxylic acid methyl ester (9.54 mmol) and using 80 mL of sulfuric acid. MS (ES+): 300, 302 (M+H).
Step 5. Preparation of 3-Amino-6-methyl-5-trifluoromethyl-pyridine-2-carboxylic acid methyl ester 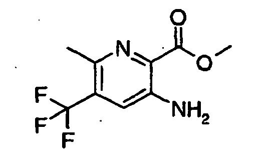 Prepare the title compound by essentially following the procedure described in Example 8, by replacing (+/-)-9-[acetyl-(3,5-bis-trifluoromethylbenzyl)amino]-2-chloro-6,7,8,9-tetrahydro-pyrido[3,2-*b*]azepine-5-carboxylic acid isopropyl ester with 3-amino-6-bromo-5-trifluoromethyl-pyridine-2-carboxylic acid methyl ester. MS (ES+): 235 (M+H).
Step 6. Preparation of 3-Isopropoxycarbonylamino-6-methyl-5-trifluoromethyl-pyridine-2-carboxylic acid methyl ester 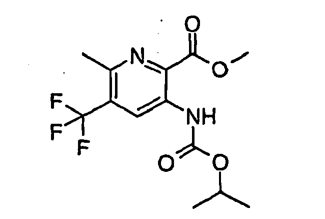 Prepare the title compound by essentially following the procedure described in Example 4, Step 4, by replacing 3-amino-6-chloro-pyridine-2-carboxylic acid methyl ester with 3-amino-6-methyl-5-trifluoromethyl-pyridine-2-carboxylic acid methyl ester and stirring the mixture at room temperature for 2 h. MS (ES+): 321 (M+H).
Step 7. Preparation of 3-[Isopropoxycarbonyl-(3-methoxycarbonyl-propyl)-amino]-6-methyl-5-trifluoromethyl-pyridine-2-carboxylic acid methyl ester 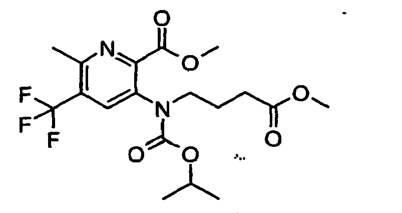 Add cesium carbonate (0.84 g, 2.56 mmol) followed by methyl 4-bromobutyrate (0.21 mL, 1.53 mmol) to a solution of 3-isopropoxycarbonylamino-6-methyl-5-trifluoromethylpyridine-2-carboxylic acid methyl ester (410 mg, 1.28 mmol) in dimethylformamide (2 mL) at room temperature under nitrogen atmosphere and stir the mixture at 80 °C for 15 h. Cool the mixture to room temperature, add water, and extract with ethyl acetate. Dry the organic layer over anhydrous sodium sulfate, filter, and remove the solvent under reduced pressure. Purify the residue by flash chromatography, eluting with hexanes/ethyl acetate, to provide the title compound (330 mg, 61%). MS (ES+): 582 (M+H).
Step 8. Preparation of 2-Methyl-9-oxo-3-trifluoromethyl-6,7,8,9-tetrahydro-pyrido[3,2-b]azepine-5-carboxylic acid isopropyl ester 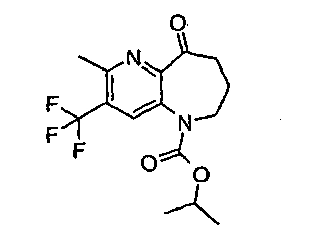 Prepare the title compound by essentially following the procedure described in Example 4, Step 6, by replacing 6-chloro-[(3-ethoxycarbonyl-propyl)-3-isopropoxycarbonylamino]-pyridine-2-carboxylic acid methyl ester with 3-[isopropoxycarbonyl-(3-methoxycarbonyl-propyl)-arnino]-6-methyl-5-trifluoromethyl-pyridine-2-carboxylic acid methyl ester. MS (ES+): 331 (M+H).
Step 9. (+/-)-9-[Acetyl-(3,5-bis-trifluoromethyl-benzyl)amino]-2-methyl-3-trifluoro methyl-6,7,8,9-tetrahydro-pyrido[3,2-b]azepine-5-carboxylic acid isopropyl ester 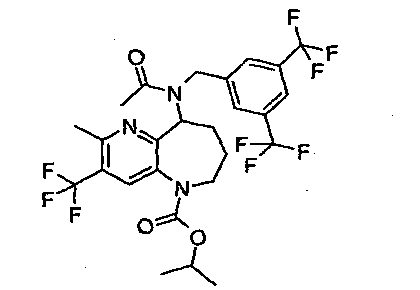 Prepare the title compound by essentially following the procedures described in Example 4, Step 7, by replacing 2-chloro-9-oxo-6,7,8,9-tetrahydro-pyrido[3,2-*b*]azepine-5-carboxylic acid isopropyl ester with 2-methyl-9-oxo-3-trifluoromethyl-6,7,8,9-tetrahydropyrido[3,2-*b*]azepine-5-carboxylic acid isopropyl ester. Besides the title compound, 9-[acetyl-(3,5-bis-trifluorometllyl-benzyl)-amino]-2-methyl-3-trifluoromethyl-6,7-dihydropyrido[3b]-azepine-5-carboxylic acid isopropyl ester is also obtained in this reaction. Dissolve the residue in methanol (1 mL), add platinum (II) oxide (2 mg) and stir the mixture under hydrogen atmosphere for 4,h. Filter the residue through Celite®, wash with dichloromethane, and remove the residue under reduced pressure. Purify the residue by flash chromatography, eluting with ethyl acetate/hexanes to afford the title compound (18.5 mg, 26%). MS (ES+): 600 (M+H).

### Example 13

### (+/-)-9-[Acetyl-(3,5-bis-trifluoromethyl-benzyl)amino]-2-methyl-3-trifluoromethyl-6,7,8,9-tetrahydro-pyrido[3,2-b]azepine-5-carboxylic acid tert-butyl ester

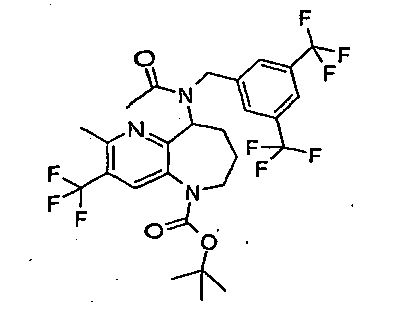

Step 1. Preparation of 2-Methyl-9-oxo-3-trifluoromethyl-6,7,8,9-tetrahydro-pyrido[3,2-*b*]azepine 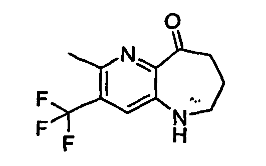 Add iodotrimethylsilane (0.071 mL, 0.484 mmol) to a solution of 2-methyl-9-oxo-3-trifluoromethyl-6,7,8,9-tetrahydro-pyrido[3,2-*b*]azepine-5-carboxylic acid isopropyl ester (80 mg, 0.242 mmol) (Example 12, Step 8) in chloroform (2 mL) at 0 °C. Remove the ice bath and stir the mixture at room temperature for 3 h and at reflux temperature for 24 h. Concentrate under vacuum, dissolve the residue in dichloromethane, and wash with an aqueous solution of sodium thiosulfite. Separate the organic layer, dry over sodium sulfate, and remove the solvent under reduced pressure. Purify the residue using silica gel chromatography, eluting with ethyl acetate /hexanes to afford the title compound (31 mg, 52%). MS (ES+): 245 (M+H).
Step 2. Preparation of 2-Methyl-9-oxo-3-trifluoromethyl-6,7,8,9-tetrahydro-pyrido[3,2-b]azepine-5-carboxylic acid *tert*-butyl ester 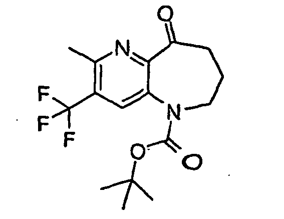 Add *tert*-butoxycarbonyl dicarbonate (33 mg, 0.147 mmol), diisopropylethyl amine (0.41 mL, 0.271 mmol) and 4,4-dimethylaminopiridine (32 mg, 0.061 mmol) to a solution of 2-methyl-9-oxo-3-trifluoromethyl-6,7,8,9-tetrahydro-pyrido[3,2-*b*]azepine (30 mg, 0.13 mmol) in dichloromethane (1 mL) and stir the mixture at room temperature for 20 h. Add water and extract with dichloromethane, dry over sodium sulfate, filter, and remove the solvent under reduced pressure. Purify the residue using silica gel chromatography, eluting with ethyl acetate/hexane to afford the title compound (30 mg, 70%). MS (ES+): 245 (M-Boc).
Step 3. Preparation of (+/-)-9-(3,5-Bis-trifluoromethyl-benzyl)amino-2-methyl-3-trifluoromethyl-6,7,8,9-tetrahydro-pyrido[3,2-b]azepine-5-carboxylic acid *tert*-butyl ester 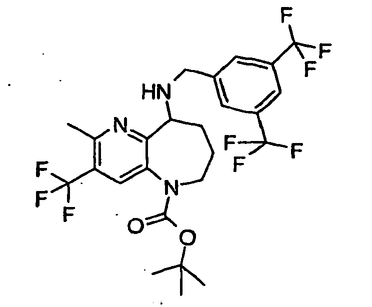 Add 3,5-bis(trifluoromethyl)benzylamine (565 mg, 2.26 mmol) followed by titanium isopropoxide (642 mg, 2.26 mmol) to 2-methyl-9-oxo-3-trifluoromethyl-6,7,8,9-tetrahydro-pyrido[3,2-*b*]azepine-5-carboxylic acid tert-butyl ester (380 mg, 1.13 mmol) at room temperature under an atmosphere of nitrogen and stir the solution for 20 h. Add methanol (1 mL) and sodium borohydride (85 mg, 2.26 mmol) and stir the mixture at room temperature for 3 h. Add 0.1M sodium hydroxide and stir for 30 min. Filter through Celite® and wash the residue with ethyl acetate. Separate the organic layer and extract the aqueous portion with ethyl acetate. Dry the organic layers over anhydrous sodium sulfate, filter, and remove the solvent under reduced pressure. Dissolve the residue in methanol (3 mL), add 10% palladium on carbon (20 mg) and stir the mixture under an atmosphere of hydrogen for 4 h. Filter the crude material over Celite®, washing with methanol and dichloromethane. Remove the solvent under reduced pressure and purify the residue using silica gel chromatography, eluting with ethyl acetate/hexanes to afford the title compound (578 mg, 89%). MS (ES+): 572 (M+H).
Step 4. (+/-)-9-[Acetyl-(3,5-bis-trifluoromethyl-benzyl)amino]-2-methyl-3-trifluoromethyl-6,7,8,9-tetrahydro-pyrido[3,2-*b*]azepine-5-carboxylic acid tert-butyl ester 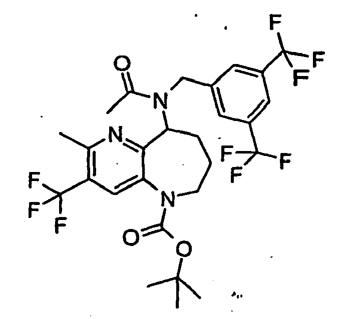 Add acetyl chloride (0.006 mL, 0.084 mmol) followed by pyridine (0.011 mL, 0.14 mmol) to a solution of (3,5-bis-trifluoromethyl-benzyl)amino-2-methyl-3-trifluoromethyl-6,7,8,9-tetrahydro-pyrido[3,2-*b*]azepine-5-carboxylic acid *tert*-butyl ester (16 mg, 0.028 mmol) in dichloromethane (0.5 mL) and stir at room temperature for 15 h. Remove the solvent under reduced pressure and purify the residue using silica gel chromatography, eluting with ethyl acetate/hexanes 1:4 to afford the title compound (10 mg, 59%). MS (ES+): 614 (M+H).

### Example 14

### (+/-)-9-[(3,5-Bis-trifluoromethyl-benzyl)-2-methyl-2H-tetrazol-5-yl)-amino]-2-methyl-3-trifluoromethyl-6,7,8,9-tetrahydro-pyrido[3,2-b]azepine-5-carboxylic acid isopropyl ester

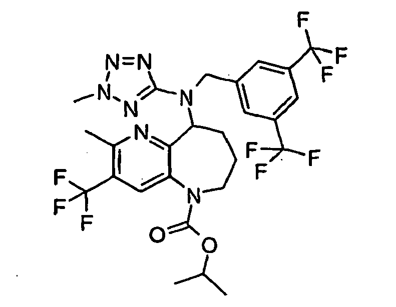

Step 1. (+/-)-9-[(3,5-Bis-trifluoromethyl-benzyl)-cyano-amino]-2-methyl-3-trifluoromethyl-6,7,8,9-tetrahydro-pyrido[3,2-*b*]azepine-5-carboxylic acid isopropyl ester 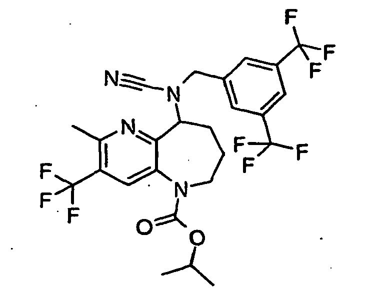 Combine (+/-)-9-(3,5-bis-trifluoromethyl-benzyl)amino-2-methyl-3-trifluoromethyl-6,7,8,9-tetrahydro-pyrido[3,2-*b*]azepine-5-carboxylic acid isopropyl ester (130 mg, 0.233 mmol) and cyanogen bromide (76 mg, 0.699 mmol) in diethyl ether (1.5 mL) and stir at room temperature for 15 h. Remove the solvent *in vacuo* and purify the resulting residue using silica gel chromatography, eluting with ethyl acetate to afford the title compound (121 mg, 89%). MS (ES+): 583 (M+H).
Step 2. Preparation of (+/-)-9-[(3,5-Bis-trifluoromethyl-benzyl)-2-methyl-2*H*-tetrazol-5-yl)-amino]-2-methyl-3-trifluoromethyl-6,7,8,9-tetrahydro-pyrido[3,2-*b*]azepine-5-carboxylic acid isopropyl ester 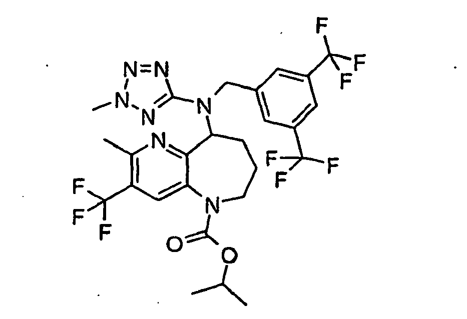 Combine (+/-)-9-[(3,5-bis-trifluoromethyl-benzyl)-amino-cyano]-2-methyl-3-trifluoromethyl-6,7,8,9-tetrahydro-pyrido[3,2-*b*]azepine-5-carboxylic acid isopropyl ester (120 mg, 0.268 mmol), sodium azide (17 mg, 0.258 mmol), and triethyl amine hydrochloride (37 mg, 0.268 mmol) in dry toluene (3 mL) under a nitrogen atmosphere and heat at 120 °C for 7 h. Cool the reaction to room temperature, dilute with ethyl acetate, and wash with 1 N hydrochloric acid. Separate the organic layer, dry over anhydrous sodium sulfate, filter, and remove the solvent under reduced pressure to afford (+/-)-9-[(3,5-bis-trifluoromethyl-benzyl)-2H-tetrazol-5-yl)-amino]-2-methyl-3-trifluoromethyl-6,7,8,9-tetrahydro-pyrido[3,2-*b*]azepine-5-carboxylic acid isopropyl ester (110 mg). Add triphenylphosphine (110 mg, 0.176 mmol), methanol (22 mg, 0.704 mmol) and diisopropylazodicarboxylate (0.031 mL, 0.176 mmol) to a solution of (+/-)-9-[(3,5-bis-trifluoromethyl-benzyl)-2*H*-tetrazol-5-yl)-amino]-2-methyl-3-trifluoromethyl-6,7,8,9-tetrahydro-pyrido[3,2-*b*]azepine-5-carboxylic acid isopropyl ester in dry dichloromethane (1 mL) and stir the mixture for 15 h at room temperature. Remove the solvent under vacuum and purify the residue using a silica gel cartridge, eluting with ethyl acetate/hexanes 1:6 to afford the title compound (12 mg, 7%). MS (ES+): 640 (M+H).

### Example 15

### (+/-)-9-[(3,5-Bis-trifluoromethyl-benzyl)-2-methyl-2H-tetrazol-5-yl)-amino]-2-methyl-3-trifluoromethyl-6,7,8,9-tetrahydro-pyrido[3,2-b]azepine-5-carboxylic acid tert-butyl ester

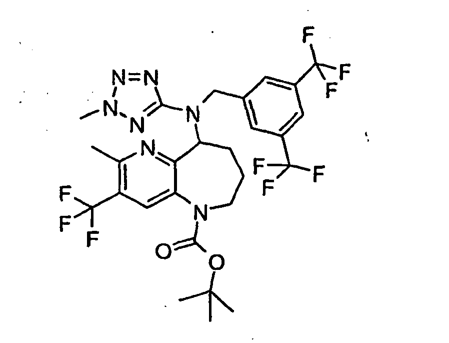

Step 1. Preparation of (+/-)-9-[(3,5-Bis-trifluoromethyl-benzyl)-cyano-amino]-2-methyl-3-trifluoromethyl-6,7,8,9-tetrahydro-pyrido[3,2-b]azepine-5-carboxylic acid *tert*-butyl ester 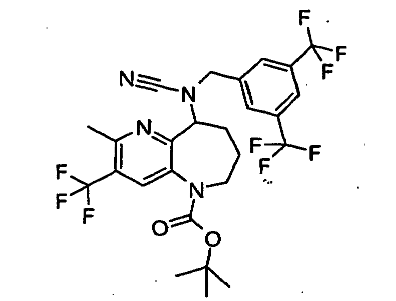 Prepare the title compound by essentially following the procedures described in Example 14, Step 1, by replacing (+/-)-9-(3,5-bis-trifluoromethyl-benzyl)-amino-2-methyl-3-trifluoromethyl-6,7,8,9-tetrahydro-pyrido[3,2-*b*]azepine-5-carboxylic acid isopropyl ester with (+/-)-9-(3,5-bis-trifluoromethyl-benzyl)amino-2-methyl-3-trifluoromethyl-6,7,8,9-tetrahydro-pyrido[3,2-*b*]azepine-5-carboxylic acid *tert*-butyl ester. MS (ES+): 597 (M+H).
Step 2. (+/-)-9-[(3,5-Bis-trifluoromethyl-benzyl)-2-methyl-2*H*-tetrazol-5-yl)-amino]-2-methyl-3-trifluoromethyl-6,7,8,9-tetrahydro-pyrido[3,2-*b*]azepine-5-carboxylic acid *tert-*butyl ester 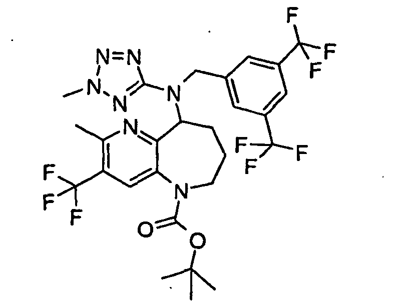 Prepare the title compound by essentially following the procedures described in Example 14, Step 2, by replaci ng (+/-)-9-[(3,5-bis-trifluoromethyl-benzyl)-amino-cyano]-2-methyl-3-trifluoromethyl-6,7,8,9-tetrallydro-pyrido[3,2-*b*]azepine-5-carboxylic acid isopropyl ester with (+/-)-9-[(3,5-bis-trifluoromethyl-benzyl)-cyano-amino]-2-methyl-3-trifluoromethyl-6,7,8,9-tetrahydro-pyrido[3,2-*b*]azepine-5-carboxylic acid *tert*-butyl ester. MS (ES+): 654 (M+H).

## Claims

1. A compound of formula I 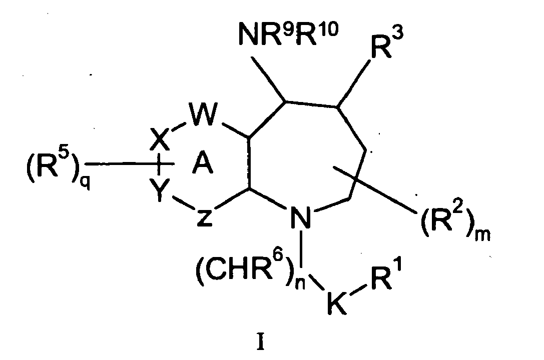 wherein
n is 0,1,2, or 3;
m is 0, 1, 2, 3, 4, 5 or 6;
q is 0, 1, or 2;
W, X, Y and Z are each independently CH, C, N, S, or O with appropriate single or double bonds and/or hydrogen atoms to complete valency requirements; Ring A is a five or six member ring wherein one of W, X, Y or Z may be absent; provided that ring A is not phenyl;
K is a bond, C=O, or S(O)ₚ;
p is 0, 1 or 2;
R¹ is selected from hydroxy, C₁-C₆ alkyl, C₂-C₆ alkenyl, C₁-C₆ haloalkyl, C₁-C₆ alkylheterocyclic, C₃-C₈ cycloalkyl, C₁-C₆ alkylC₃-C₈cycloalkyl, C₁-C₆ alkylaryl, aryl, heterocyclyl, C₂-C₆ alkylalcohol, -OC₁-C₆ alkyl, -O-aryl, -OC₂-C₆ alkenyl, -OC₁-C₆ haloalkyl, -OC₁-C₆ alkylheterocyclic, -OC₃-C₈ cycloalkyl, -OC₁-C₆ alkylC₃-C₈cycloalkyl, -NR⁷R⁸, -OC₁-C₆ alkylaryl, -O-heterocyclic, -OC₁-C₆alkylCO₂R¹¹, -OC₂-C₆alkylalcohol, -OC₁-C₆alkylNR⁷R⁸, -OC₂-C₆alkylcyano, CONR¹¹R¹², NR¹¹SO₂R¹², NR¹¹COR¹², C₀-C₃ alkylNR¹¹R¹², C₁-C₃ alkylCOR¹¹ and C₀-C₆ alkylCOOR¹¹; provided that R¹ is not hydroxy when K is S(O)ₚ, CO, and/or when n is zero and K is a bond; and wherein each cycloalkyl, aryl or heterocyclic group is optionally substituted with 1 to 3 groups independently selected from oxo, hydroxy, halo, C₁-C₆ alkyl, C₂-C₆ alkenyl, C₂-C₆ alkynyl, C₁-C₆ alkoxy, C₁-C₆ haloalkyl, C₁-C₆ alkylalcohol, -OC₂-C₆alkylalcohol, C₁-C₆ haloalkoxy, CONR¹¹R¹², NR¹¹SO₂R¹², NR¹¹COR¹², C₀-C₃ alkylNR¹¹R¹², C₁-C₃ alkylCOR¹¹, C₀-C₆ alkylCOOR¹¹, C₀-C₆ alkylcyano, -OC₂-C₆alkylcyano, C₁-C₆ alkylC₃-C₈cycloalkyl, phenyl, -OC₁-C₆ alkylC₃-C₈cycloalkyl, -OC₁-C₆ alkylaryl, -OC₁-C₆ alkylheterocyclic, and C₁-C₆ alkylaryl;
R² is independently selected from hydrogen, C₁-C₆alkyl and C₃-C₈cycloalkyl;
R³ is hydrogen, C₁-C₆ alkyl, aryl, C₂-C₆ alkenyl, C₂-C₆ alkynyl, C₁-C₆ alkylaryl, C₁-C₆ alkylheterocyclic, C₃-C₈ cycloalkyl, or C₁-C₆ alkylC₃-C₈cycloalkyl;
R⁵ is selected from hydrogen, halogen, hydroxy, C₁-C₆haloalkyl, C₁-C₆alkyl, C₂-C₆alkenyl, C₁-C₆alkylheterocyclic, C₁-C₆alkylaryl, aryl, -OC₁-C₆alkyl, -O-aryl, -OC₂-C₆alkenyl, -OC₁-C₆haloalkyl, NR⁷R⁸, -CH₂NR⁷R⁸, -CN, -COOH and NO₂;
R⁶ is independently selected from hydrogen, C₁-C₆ alkyl, C₂-C₆ alkenyl, hydroxy, -OC₁-C₆ alkyl, -O-aryl, -OC₂-C₆ alkenyl, C₁-C₆ haloalkyl, -OC₁-C₆ haloalkyl, C₁-C₆ alkylNR⁷R⁸, C₃-C₈ cycloalkyl, and C₁-C₆ alkylC₃-C₈cycloalkyl;
R⁷ and R⁸ are independently selected from hydrogen, C₁-C₆ alkyl, C₂-C₆ alkenyl, C₃-C₈ cycloalkyl, C₁-C₆ alkylC₃-C₈cycloalkyl, C₁-C₆ alkylheterocyclic, heterocyclic, aryl, C₁-C₆ alkylaryl, hydroxy, oxo, COOH, C(O)OC₁-C₄ alkyl, C₂-C₆ alkynyl, C₁-C₆ alkoxy, C₁-C₆ haloalkyl, C₁-C₆ alkylalcohol, C₁-C₆ alkylamine, C₂-C₆ alkenylaryl, C₂-C₆ alkynylaryl, C₁-C₆ alkyl-O-C₁-C₆ alkylaryl, C₁-C₆ alkyl-NR¹¹-C₁-C₆ alkylaryl, C₁-C₆ alkylcyano, C₁-C₆ alkylCONR⁷R⁸, C₁-C₆ alkylNR⁷R⁸ and C₁-C₆alkylNR¹¹COR¹², wherein each alkyl, cycloalkyl, heterocyclic, or aryl group is optionally substituted with 1 to 3 groups independently selected from hydroxy, oxo, amino, halogen, C₁-C₆ alkylC₃-C₈cycloalkyl, C₃-C₈ cycloalkyl, C₁-C₆ alkylheterocyclic, C₁-C₆ haloalkyl, COOH, C(O)OC₁-C₄ alkyl, C₁-C₆ alkyl, C₂-C₆ alkenyl, C₂-C₆ alkynyl, C₁-C₆ alkoxy, C₁-C₆ alkylalcohol, C₁-C₆ alkylamine and NR¹¹R¹²; or R⁷ and R⁸ combine to form a nitrogen containing heterocyclic ring which may have 0, 1, or 2 additional hetero-atoms selected from oxygen, nitrogen or sulfur and may be optionally substituted with oxo, or C₁-C₆ alkyl;
R⁹ is selected from C₁-C₆ alkyl, C₂-C₆ alkenyl, C₃-C₈ cycloalkyl, C₁-C₆ alkylcycloalkyl, aryl, heterocyclic, C₁-C₆ alkylheterocyclic, COR⁷, CO₂R⁷, C₀-C₃ alkylCONR⁷R⁸, C₀-C₃ alkylS(O)ₚNR⁷R⁸ and C₀-C₃ alkylS(O)ₚR⁷ wherein R⁷ is as defined above, and wherein each alkyl, cycloalkyl, aryl, and heterocyclic is optionally substituted with one to two groups independently selected from halo, hydroxy, oxo, COOH, C(O)OC₁-C₄ alkyl, C₁-C₆ haloalkyl, C₁-C₆ alkyl, C₂-C₆ alkenyl, C₂-C₆ alkynyl, C₁-C₆ alkoxy, C₁-C₆ alkylalcohol, C₁-C₆ alkylamine, C₁-C₆ alkylaryl, C₂-C₆ alkenylaryl, C₂-C₆ alkynylaryl, C₁-C₆ alkylheterocyclic, -NR⁷R⁸, C₃-C₈ cycloalkyl, C₁-C₆ alkylC₃-C₈cycloalkyl, C₁-C₆ alkyl-O-C₁-C₆ alkylaryl, C₁-C₆ alkylcyano, C₁-C₆ alkylCONR⁷R⁸, C₁-C₆ alkylNR⁷R⁸, C₁-C₆alkylNR¹¹COR¹², and aryl, wherein each cycloalkyl or aryl group is optionally substituted with halo, hydroxy, oxo, amino, COOH, C(O)OC₁-C₄ alkyl, C₁-C₆ haloalkyl, C₁-C₆ alkyl, C₂-C₆ alkenyl, C₂-C₆ alkynyl, C₁-C₆ alkoxy, C₁-C₆ alkylalcohol, and C₁-C₆ alkylamine;
R¹⁰ is selected from aryl, C₁-C₆ alkylaryl, C₂-C₆ alkenylaryl, C₂-C₆ alkynylaryl, C₁-C₆ haloalkylaryl, C₁-C₆ alkylheterocyclic, C₂-C₆ alkenylheterocyclic, C₁-C₆ alkylC₃-C₈cycloalkyl, C₃-C₈cycloalkyl and C₁-C₆ alkyl-O-C₁-C₆ alkylaryl, wherein each cycloalkyl, aryl, or heterocyclic group is optionally substituted with 1 to 3 groups independently selected from hydroxy, oxo, -SC₁-C₆ alkyl, C₁-C₆ alkyl, C₁-C₆ alkenyl, C₁-C₆ alkynyl, C₁-C₆ haloalkyl, halogen, C₁-C₆ alkoxy, aryloxy, C₁-C₆ alkenyloxy, C₁-C₆ haloalkoxyalkyl, C₀-C₆ alkylNR¹¹R¹², -OC₁-C₆ alkylaryl, nitro, cyano, -OC₁-C₆ haloalkyl, C₁-C₆ haloalkylalcohol, and C₁-C₆ alkylalcohol;
R¹¹ and R¹² are independently selected from hydrogen, C₁-C₆ alkyl, C₂-C₆ alkenyl, C₃-C₈ cycloalkyl, heterocyclic, aryl, and C₁-C₆ alkylaryl, wherein each aryl group is optionally substituted with 1 to 3 groups independently selected from halogen, C₁-C₆ alkylheterocyclic, and C₁-C₆ haloalkyl, or R¹¹ and R¹² combine to form a nitrogen containing heterocyclic ring which may have 0, 1, or 2 additional heteroatoms selected from oxygen, nitrogen or sulfur and is optionally substituted with oxo, or C₁-C₆ alkyl; or a pharmaceutically acceptable salt, enantiomer, racemate, diastereomer or mixture of diastereomers thereof.

2. A compound according to claim 1, or a pharmaceutically acceptable salt, enantiomer, racemate, diastereomer or mixture of diastereomers thereof, wherein n is 0, K is C=O and R¹ is selected from hydroxy, C₁-C₆ alkyl, C₁-C₆ alkylC₃-C₈cycloalkyl, C₁-C₆ alkylheterocyclic, C₁-C₆ haloalkyl, C₁-C₆ alkylaryl, -OC₁-C₆ alkyl, -OC₃-C₈ cycloalkyl, - OC₁-C₆ alkylC₃-C₈cycloalkyl, -OC₁-C₆ alkylaryl, -OC₁-C₆ haloalkyl, -OC₂-C₆alkylcyano, -OC₁-C₆alkylCO₂R¹¹, -OC₂-C₆alkylalcohol, -OC₁-C₆ alkylNR⁷R⁸, and -OC₁-C₆ alkylheterocyclic and wherein each cycloalkyl, aryl, or heterocyclic is optionally substituted with 1 or 2 groups selected from halo, C₁-C₃ alkylalcohol, -C₀-C₃ alkylamine, C₀-C₃ alkylCOOH, -CONH₂ and C₀-C₃alkylcyano.

3. A compound according to claim 1, or a pharmaceutically acceptable salt, enantiomer, racemate, diastereomer or mixture of diastereomers thereof, wherein R⁹ is a heterocyclic group optionally substituted with one or two groups independently selected from hydroxy, halo, amino, C(O)OC₁-C₄ alkyl, C₁-C₆ haloalkyl, C₁-C₆ alkyl, C₂-C₆ alkenyl, C₂-C₆ alkynyl, C₁-C₆ alkoxy, C₁-C₆ alkylalcohol, C₁-C₆ alkylamine, C₃-C₈ cycloalkyl, C₁-C₆ alkylCONR⁷R⁸, C₁-C₆ alkylcyano, C₁-C₆ alkylNR⁷R⁸, and C₁-C₆ alkylC₃-C₈cydoalkyl.

4. A compound according to claim 1, or a pharmaceutically acceptable salt, enantiomer, racemate, diastereomer or mixture of diastereomers thereof, wherein n, m, and q are independently 0 or 1.

5. A compound according to claim 1, or a pharmaceutically acceptable salt, enantiomer, racemate, diastereomer or mixture of diastereomers thereof, wherein the A ring is selected from pyridine, pyrazine, thiophene, pyrazole, isoxazole, oxazole, and thiazole.

6. A compound according to claim 1, or a pharmaceutically acceptable salt, enantiomer, racemate, diastereomer or mixture of diastereomers thereof, wherein the A ring is pyridine.

7. A compound according to claim 1, or a pharmaceutically acceptable salt, enantiomer, racemate, diastereomer or mixture of diastereomers thereof, wherein the A ring is thiophene.

8. A compound according to claim 1, or a pharmaceutically acceptable salt, enantiomer, racemate, diastereomer or mixture of diastereomers thereof, wherein R⁹ is COOR⁷.

9. A compound according to claim 1 selected from:
5-[Acetyl-(3,5-bis-trifluoromethyl-benzyl)-amino]-2,3,4,5-tetrahydro-thieno[3,4-b]azepine-1-carboxylic acid isopropyl ester,
8-[Acetyl-(3,5-bis-trifluoromethyl-benzyl)-amino]-3-methyl-5,6,7,8-tetrahydrothieno[3,2-b]azepine-4-carboxylic acid isopropyl ester,
8-[Acetyl-(3,5-bis-trifluoromethyl-benzyl)-amino]-2-bromo-5,6,7,8-tetrahydrothieno[3,2-b]azepine-4-carboxylic acid isopropyl ester,
5-[Acetyl-(3,5-bis-trifluoromethyl-benzyl)-amino]-5,6,7,8-tetrahydro-pyrido[2,3-b]azepine-9-carboxylic acid isopropyl ester,
5-[Acetyl-(3,5-bis-trifluoromethyl-benzyl)-amino]-2,3,4,5-tetrahydro-pyrido[3,4-b]azepine-1-carboxylic acid isopropyl ester,
5-[Acetyl-(3,5-bis-trifluoromethyl-benzyl)-amino]-2,3,4,5-tetrahydro-pyrido[4,3-b]azepine-1-carboxylic acid isopropyl ester,
9-[Acetyl-(3,5-bis-trifluoromethyl-benzyl)-amino]-6,7,8,9-tetrahydro-pyrido[3,2-b]azepine-5-carboxylic acid isopropyl ester,
9-[Acetyl-(3,5-bis-trifluoromethyl-benzyl)-amino]-2-trifluoromethyl-6,7,8,9-tetrahydropyrido[3,2-b]azepine-5-carboxylic acid isopropyl ester,
9-[Acetyl-(3,5-bis-trifluoromethyl-benzyl)-amino]-3-trifluoromethyl-6,7,8,9-tetrahydropyrido[3,2-b]azepine-5-carboxylic acid isopropyl ester,
4-[Acetyl-(3,5-bis-trifluoromethyl-benzyl)-amino]-1-methyl-4,5,6,7-tetrahydro-1H-1,2,8-triaza-azulene-8-carboxylic acid isopropyl ester,
9-[acetyl-(3,5-bis-trifluoromethylbenzyl)amino]-2-chloro-6,7,8,9-tetrahydro-pyrido[3,2-b]azepine-5-carboxylic acid isopropyl ester,
9-[acetyl-(3,5-bis-trifluoromethylbenzyl)amino]-2-methoxy-6,7,8,9-tetrahydropyrido[3,2-*b*]azepine-5-carboxylic acid isopropyl ester,
9-[acetyl-(3,5-bis-trifluoromethylbenzyl)amino]-2-bromo-6,7,8,9-tetrahydro-pyrido[3,2-b]azepine-5-carboxylic acid isopropyl ester,
9-[Acetyl-(3,5-bis-trifluoromethylbenzyl)amino]-2-dimethylamino-6,7,8,9-tetrahydropyrido[3,2-*b*]azepine-5-carboxylic acid isopropyl ester,
9-[Acetyl-(3,5-bis-trifluoromethylbenzyl)amino]-2-methyl-6,7,8,9-tetrahydro-pyrido[3,2-*b*]azepine-5-carboxylic acid isopropyl ester,
9-[Acetyl-(3,5-bis-trifluoromethylbenzyl)amino]-2-cyano-6,7,8,9-tetrahydro-pyrido[3,2-*b*]azepine-5-carboxylic acid isopropyl ester,
9-[Acetyl-(3,5-bis-trifluoromethylbenzyl)amino]-3-chloro-2-methoxy-6,7,8,9-tetrahydropyrido[3,2-*b*]azepine-5-carboxylic acid isopropyl ester,
9-[Acetyl-(3,5-bis-trifluoromethylbenzyl)amino]-3-chloro-2-ethoxy-6,7,8,9-tetrahydropyrido[3,2-*b*]azepine-5-carboxylic acid isopropyl ester,
9-[Acetyl-(3,5-bis-trifluoromethyl-benzyl)amino]-2-methyl-3-trifluoromethyl-6,7,8,9-tetrahydro-pyrido[3,2-*b*]azepine-5-carboxylic acid isopropyl ester,
9-[Acetyl-(3,5-bis-trifluoromethyl-benzyl)amino]-2-methyl-3-trifluoromethyl-6,7,8,9-tetrahydro-pyrido[3,2-*b*]azepine-5-carboxylic acid *tert*-butyl ester,
9-[(3,5-Bis-trifluoromethyl-benzyl)-2-methyl-2*H*-tetrazol-5-yl)-amino]-2-methyl-3-trifluoromethyl-6,7,8,9-tetrahydro-pyrido[3,2-*b*]azepine-5-carboxylic acid isopropyl ester,
9-[(3,5-Bis-trifluoromethyl-benzyl)-2-methyl-2*H*-tetrazol-5-yl)-amino]-2-methyl-3-trifluoromethyl-6,7,8,9-tetrahydro-pyrido[3,2-*b*]azepine-5-carboxylic acid *tert*-butyl ester,
(3,5-Bis-trifluoromethyl-benzyl)-(5-cyclopentylmethyl-2-methyl-3-trifluoromethyl-6,7,8,9-tetrahydro-5H-pyrido[3,2-b]azepin-9-yl)-(2-methyl-2H-tetrazol-5-yl)-amine,
(3,5-Bis-trifluoromethyl-benzyl)-(5-cyclopropylmethyl-2-methyl-3-trifluoromethyl-6,7,8,9-tetrahydro-5H-pyrido[3,2-b]azepin-9-yl)-(2-methyl-2H-tetrazol-5-yl)-amine,
(3,5-Bis-trifluoromethyl-benzyl)-(2-methyl-5-pyridin-3-ylmethyl-3-trifluoromethyl-6,7,8,9-tetrahydro-5H-pyrido[3,2-b]azepin-9-yl)-(2-methyl-2H-tetrazol-5-yl)-amine,
(3,5-Bis-trifluoromethyl-benzyl)-(2-methyl-5-pyridin-4-ylmethyl-3-trifluoromethyl-6,7,8,9-tetrahydro-5H-pyrido[3,2-b]azepin-9-yl)-(2-methyl-2H-tetrazol-5-yl)-amine,
3-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl}-benzoic acid,
4- {9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl}-benzoic acid,
5-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-yl}-3,3-dimethyl-pentanoic acid,
(4-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl}-cyclohexyl)-acetic acid,
(3,5-Bis-trifluoromethyl-benzyl)-(5-ethyl-2-methyl-3-trifluoromethyl-6,7,8,9-tetrahydro-5H-pyrido[3,2-b]azepin-9-yl)-(2-methyl-2H-tetrazol-5-yl)-amine,
5-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-ylmethyl }-thiophene-2-carboxylic acid,
2-{9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepin-5-yl }-ethanol,
(5-Benzyl-2-methyl-3-trifluoromethyl-6,7,8,9-tetrahydro-5H-pyrido[3,2-b]azepin-9-yl)-(3,5-bis-trifluoromethyl-benzyl)-(2-methyl-2H-tetrazol-5-yl)-amine,
(3,5-Bis-trifluoromethyl-benzyl)-(2-methyl-2H-tetrazol-5-yl)-(2-methyl-5-thiazol-2-ylmethyl-3-trifluoromettiyl-6,7,8,9-tetrahydro-5H-pyrido[3,2-b]azepin-9-yl)-amine,
9-[(3,5-Bis-trifluoromethyl-benzyl)-(2-methyl-2H-tetrazol-5-yl)-amino]-2-methyl-3-trifluoromethyl-6,7,8,9-tetrahydro-pyrido[3,2-b]azepine-5-carboxylic acid tetrahydrofuran-3-yl ester,
(3,5-Bis-trifluoromethyl-benzyl)-(2-methyl-5-pyridin-4-ylmethyl-3-trifluoromethyl-6,7,8,9-tetrahydro-5H-pyrido[3,2-b]azepin-9-yl)-carbamic acid methyl ester,
N-(3,5-Bis-trifluoromethyl-benzyl)-N-(2-methyl-5-pyridin-4-ylmethyl-3-trifluoromethyl-6,7,8,9-tetrahydro-5H-pyrido[3,2-b]azepin-9-yl)-acetamide
or a pharmaceutically acceptable salt, solvate enantiomer or diastereomer or mixture thereof.

10. A pharmaceutical composition comprising a compound according to any one of claims 1 to 9, or a pharmaceutically acceptable salt, enantiomer, racemate, diastereomer or mixture of diastereomers thereof, and a carrier, diluent and/or excipient.

11. A compound according to any one of claims 1 to 9, or a pharmaceutically acceptable salt, enantiomer, racemate, diastereomer or a mixture of diastereomers thereof, for use as a medicament.

12. Use of a compound according to any one of claims 1 to 9, or a pharmaceutically acceptable salt, enantiomer, racemate, diastereomer or a mixture of diastereomers thereof, for the manufacture of a medicament for the treatment or prevention of dyslipidemia.

13. Use of a compound according to any one of claims 1 to 9, or a pharmaceutically acceptable salt, enantiomer, racemate, diastereomer or a mixture of diastereomers thereof, for the manufacture of a medicament for the treatment of hyperlipidemia, atherosclerosis, coronary heart disease, elevated blood pressure, CHF, stroke, hypertension, hypertriglyceridemia, diabetes, obesity, inflammatory diseases including dermatitis, arthritis and pain, and diseases of the central nervous system including dementia and cognitive disorders.

14. Use of a compound according to any one of claims 1 to 9, or a pharmaceutically acceptable salt, enantiomer, racemate, diastereomer or a mixture of diastereomers thereof, for the manufacture of a medicament for raising the level of plasma HDL cholesterol in a mammal.

15. Use of a compound according to any one of claims 1 to 9, or a pharmaceutically acceptable salt, enantiomer, racemate, diastereomer or a mixture of diastereomers thereof, for the manufacture of a medicament for lowering the level of plasma LDL cholesterol in a mammal.

16. A compound according to any one of claims 1 to 9, or a pharmaceutically acceptable salt, enantiomer, racemate, diastereomer or a mixture of diastereomers thereof, for use in the treatment or prevention of dyslipidemia.

17. A compound according to any one of claims 1 to 9, or a pharmaceutically acceptable salt, enantiomer, racemate, diastereomer or a mixture of diastereomers thereof, for use in the treatment of hyperlipidemia, atherosclerosis, coronary heart disease, elevated blood pressure, CHF, stroke, hypertension, hypertriglyceridemia, diabetes, obesity, inflammatory diseases including dermatitis, arthritis and pain, and diseases of the central nervous system including dementia and cognitive disorders.

18. A compound according to any one of claims 1 to 9, or a pharmaceutically acceptable salt, enantiomer, racemate, diastereomer or a mixture of diastereomers thereof, for use in raising the level of plasma HDL cholesterol in a mammal.

19. A compound according to any one of claims 1 to 9, or a pharmaceutically acceptable salt, enantiomer, racemate, diastereomer or a mixture of diastereomers thereof, for use in lowering the level of plasma LDL cholesterol in a mammal.

## Patentansprüche

1. Verbindung der Formel I 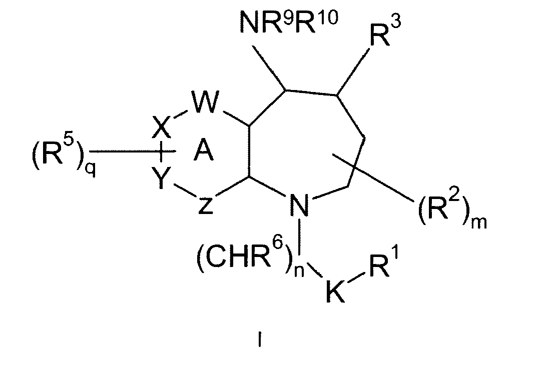 worin
n für 0, 1, 2 oder 3 steht,
m für 0, 1, 2, 3, 4, 5 oder 6 steht,
q für 0, 1 oder 2 steht,
W, X, Y und Z jeweils unabhängig mit geeigneten Einzelbindungen oder Doppelbindungen für CH, C, N, S oder O stehen und/oder für Wasserstoffatome zur Komplettierung der Valenzerfordernisse stehen, der Ring A ein fünf- oder sechsgliedriger Ring ist, worin eines von W, X, Y oder Z fehlen kann, mit der Maßgabe, dass der Ring A nicht für Phenyl steht,
K für eine Bindung, C=O oder S(O)ₚ steht,
p für 0, 1 oder 2 steht,
R¹ ausgewählt ist aus Hydroxy, C₁-C₆-Alkyl, C₂-C₆-Alkenyl, C₁-C₆-Halogenalkyl, C₁-C₆-Alkylheterocydyl, C₃-C₈-Cycloalkyl, C₁-C₆-Alkyl-C₃-C₈-cycloalkyl, C₁-C₆-Alkylaryl, Aryl, Heterocyclyl, C₂-C₆-Alkylalkohol, -O-C₁-C₆-Alkyl, -O-Aryl, -O-C₂-C₆-Alkenyl, -O-C₁-C₆-Halogenalkyl, -O-C₁-C₆-Alkylheterocyclyl, -O-C₃-C₈-Cycloalkyl, -O-C₁-C₆-Alkyl-C₃-C₈-cycloalkyl, -NR⁷R⁸, -O-C₁-C₆-Alkylaryl, -O-Heterocyclyl, -O-C₁-C₆-Alkyl-CO₂R¹¹, -O-C₂-C₆-Alkylalkohol, -O-C₁-C₆-Alkyl-NR⁷R⁸, -O-C₂-C₆-Alkylcyano, CONR¹¹R¹², NR¹¹SO₂R¹², NR¹¹COR¹², C₀-C₃-Alkyl-NR¹¹R¹², C₁-C₃-Alkyl-COR¹¹ und C₀-C₆-Alkyl-COOR¹¹, mit der Maßgabe, dass R¹ nicht für Hydroxy steht, wenn K für S(O)ₚ oder CO steht, und/oder wenn n für Null steht und K für eine Bindung steht, und worin Cycloalkyl, Aryl oder Heterocyclyl jeweils optional substituiert ist mit 1 bis 3 Gruppen, die unabhängig ausgewählt sind aus Oxo, Hydroxy, Halogen, C₁-C₆-Alkyl, C₂-C₆-Alkenyl, C₂-C₆-Alkinyl, C₁-C₆-Alkoxy, C₁-C₆-Halogenalkyl, C₁-C₆-Alkylalkohol, -O-C₂-C₆-Alkylalkohol, C₁-C₆-Halogen-alkoxy, CONR¹¹R¹², NR¹¹SO₂R¹², NR¹¹COR¹², C₀-C₃-Alkyl-NR¹¹R¹², C₁-C₃-Alkyl-COR¹¹, C₀-C₆-Alkyl-COOR¹¹, C₀-C₆-Alkylcyano, -O-C₂-C₆-Alkylcyano, C₁-C₆-Alkyl-C₃-C₈-cycloalkyl, Phenyl, -O-C₁-C₆-Alkyl-C₃-C₈-cycloalkyl, -O-C₁-C₆-Alkylaryl, -O-C₁-C₆-Alkylheterocyclyl und C₁-C₆-Alkylaryl,
R² unabhängig ausgewählt ist aus Wasserstoff, C₁-C₆-Alkyl und C₃-C₈-Cycloalkyl,
R³ für Wasserstoff, C₁-C₆-Alkyl, Aryl, C₂-C₆-Alkenyl, C₂-C₆-Alkinyl, C₁-C₆-Alkylaryl, C₁-C₆-Alkylheterocyclyl, C₃-C₈-Cycloalkyl oder C₁-C₆-Alkyl-C₃-C₈-cycloalkyl steht,
R⁵ ausgewählt ist aus Wasserstoff, Halogen, Hydroxy, C₁-C₆-Halogenalkyl, C₁-C₆-Alkyl, C₂-C₆-Alkenyl, C₁-C₆-Alkylheterocyclyl, C₁-C₆-Alkylaryl, Aryl, -O-C₁-C₆-Alkyl, -O-Aryl, -O-C₂-C₆-Alkenyl, -O-C₁-C₆-Halogenalkyl, NR⁷R⁸, -CH₂-NR⁷R⁸, -CN, -COOH und NO₂,
R⁶ unabhängig ausgewählt ist aus Wasserstoff, C₁-C₆-Alkyl, C₂-C₆-Alkenyl, Hydroxy, -O-C₁-C₆-Alkyl, -O-Aryl, -O-C₂-C₆-Alkenyl, C₁-C₆-Halogenalkyl, -O-C₁-C₆-Halogenalkyl, C₁-C₆-Alkyl-NR⁷R⁸, C₃-C₈-Cycloalkyl und C₁-C₆-Alkyl-C₃-C₈-Cycloalkyl,
R⁷ und R⁸ unabhängig ausgewählt sind aus Wasserstoff, C₁-C₆-Alkyl, C₂-C₆-Alkenyl, C₃-C₈-Cycloalkyl, C₁-C₆-Alkyl-C₃-C₈-cycloalkyl, C₁-C₆-Alkylheterocyclyl, Heterocyclyl, Aryl, C₁-C₆-Alkylaryl, Hydroxy, Oxo, COOH, C(O)O-C₁-C₄-Alkyl, C₂-C₆-Alkinyl, C₁-C₆-Alkoxy, C₁-C₆-Halogenalkyl, C₁-C₆-Alkylalkohol, C₁-C₆-Alkylamin, C₂-C₆-Alkenylaryl, C₂-C₆-Alkinylaryl, C₁-C₆-Alkyl-O-C₁-C₆-alkylaryl, C₁-C₆-Alkyl-NR¹¹-C₁-C₆-alkylaryl, C₁-C₆-Alkylcyano, C₁-C₆-Alkyl-CONR⁷R⁸, C₁-C₆-Alkyl-NR⁷R⁸ und C₁-C₆-Alkyl-NR¹¹COR¹², worin jedes Alkyl, Cycloalkyl, Heterocyclyl oder Aryl optional substituiert ist mit 1 bis 3 Gruppen, die unabhängig ausgewählt sind aus Hydroxy, Oxo, Amino, Halogen, C₁-C₆-Alkyl-C₃-C₈-cycloalkyl, C₃-C₈-Cycloalkyl, C₁-C₆-Alkylheterocyclyl, C₁-C₆-Halogenalkyl, COOH, C(O)O-C₁-C₄-Alkyl, C₁-C₆-Alkyl, C₂-C₆-Alkenyl, C₂-C₆-Alkinyl, C₁-C₆-Alkoxy, C₁-C₆-Alkylalkohol, C₁-C₆-Alkylamin und NR¹¹R¹², oder R⁷ und R⁸ zusammen einen Stickstoff enthaltenden heterocyclischen Ring bilden, der 0, 1 oder 2 weitere Heteroatome enthalten kann, die ausgewählt sind aus Sauerstoff, Stickstoff oder Schwefel, und der optional substituiert sein kann durch Oxo oder C₁-C₆-Alkyl,
R⁹ ausgewählt ist aus C₁-C₆-Alkyl, C₂-C₆-Alkenyl, C₃-C₈-Cycloalkyl, C₁-C₆-Alkylcycloalkyl, Aryl, Heterocyclyl, C₁-C₆-Alkylheterocyclyl, COR⁷, CO₂R⁷, C₀-C₃-Alkyl-CONR⁷R⁸, C₀-C₃-Alkyl-S(O)ₚNR⁷R⁸ und C₀-C₃-Alkyl-S(O)ₚR⁷, worin R⁷ wie oben definiert ist, und worin jedes Alkyl, Cycloalkyl, Aryl und Heterocyclyl optional substituiert ist durch ein oder zwei Gruppen, die unabhängig ausgewählt sind aus Halogen, Hydroxy, Oxo, COOH, C(O)O-C₁-C₄-Alkyl, C₁-C₆-Halogenalkyl, C₁-C₆-Alkyl, C₂-C₆-Alkenyl, C₂-C₆-Alkinyl, C₁-C₆-Alkoxy, C₁-C₆-Alkylalkohol, C₁-C₆-Alkylamin, C₁-C₆-Alkylaryl, C₂-C₆-Alkenylaryl, C₂-C₆-Alkinylaryl, C₁-C₆-Alkylheterocyclyl, -NR⁷R⁸, C₃-C₈-Cycloalkyl, C₁-C₆-Alkyl-C₃-C₈-cycloalkyl, C₁-C₆-Alkyl-O-C₁-C₆-alkylaryl, C₁-C₆-Alkylcyano, C₁-C₆-Alkyl-CONR⁷R⁸, C₁-C₆-Alkyl-NR⁷R⁸, C₁-C₆-Alkyl-NR¹¹COR¹² und Aryl, worin jedes Cycloalkyl oder Aryl optional substituiert ist mit Halogen, Hydroxy, Oxo, Amino, COOH, C(O)O-C₁-C₄-Alkyl, C₁-C₆-Halogenalkyl, C₁-C₆-Alkyl, C₂-C₆-Alkenyl, C₂-C₆-Alkinyl, C₁-C₆-Alkoxy, C₁-C₆-Alkylalkohol und C₁-C₆-Alkylamin,
R¹⁰ ausgewählt ist aus Aryl, C₁-C₆-Alkylaryl, C₂-C₆-Alkenylaryl, C₂-C₆-Alkinylaryl, C₁-C₆-Halogenalkylaryl, C₁-C₆-Alkylheterocyclyl, C₂-C₆-Alkenylheterocyclyl, C₁-C₆-Alkyl-C₃-C₈-cydoalkyl, C₃-C₈-Cycloalkyl und C₁-C₆-Alkyl-O-C₁-C₆-alkylaryl, worin jedes Cycloalkyl, Aryl oder Heterocyclyl optional substituiert ist mit 1 bis 3 Gruppen, die unabhängig ausgewählt sind aus Hydroxy, Oxo, -S-C₁-C₆-Alkyl, C₁-C₆-Alkyl, C₁-C₆-Alkenyl, C₁-C₆-Alkinyl, C₁-C₆-Halogenalkyl, Halogen, C₁-C₆-Alkoxy, Aryloxy, C₁-C₆-Alkenyloxy, C₁-C₆-Halogenalkoxyalkyl, C₀-C₆-Alkyl-NR¹¹R¹², -O-C₁-C₆-Alkylaryl, Nitro, Cyano, -O-C₁-C₆-Halogenalkyl, C₁-C₆-Halogenalkylalkohol und C₁-C₆-Alkylalkohol, und
R¹¹ und R¹² unabhängig ausgewählt sind aus Wasserstoff, C₁-C₆-Alkyl, C₂-C₆-Alkenyl, C₃-C₈-Cycloalkyl, Heterocyclyl, Aryl und C₁-C₆-Alkylaryl, worin jedes Aryl optional substituiert ist mit 1 bis 3 Gruppen, die unabhängig ausgewählt sind aus Halogen, C₁-C₆-Alkylheterocyclyl und C₁-C₆-Halogenalkyl, oder R¹¹ und R¹² zusammen einen Stickstoff enthaltenden heterocyclischen Ring bilden, der 0, 1 oder 2 weitere Heteroatome enthalten kann, die ausgewählt sind aus Sauerstoff, Stickstoff oder Schwefel, und der optional substituiert ist mit Oxo oder C₁-C₆-Alkyl, oder ein pharmazeutisch akzeptables, Salz, Enantiomer, Racemat, Diastereomer oder Gemisch von Diastereomeren hiervon.

2. Verbindung nach Anspruch 1 oder ein pharmazeutisch akzeptables Salz, Enantiomer, Racemat, Diastereomer oder Gemisch von Diastereomeren hiervon, worin n für 0 steht, K für C=O steht und R¹ ausgewählt ist aus Hydroxy, C₁-C₆-Alkyl, C₁-C₆-Alkyl-C₃-C₈-cycloalkyl, C₁-C₆-Alkylheterocyclyl, C₁-C₆-Halogenalkyl, C₁-C₆-Alkylaryl, -O-C₁-C₆-Alkyl, -O-C₃-C₈-Cycloalkyl, -O-C₁-C₆-Alkyl-C₃-C₈-cycloalkyl, -O-C₁-C₆-Alkylaryl, -O-C₁-C₆-Halogenalkyl, -O-C₂-C₆-Alkylcyano, -O-C₁-C₆-Alkyl-CO₂R¹¹, -O-C₂-C₆-Alkylalkohol, -O-C₁-C₆-Alkyl-NR⁷R⁸ und -O-C₁-C₆-Alkylheterocyclyl und worin jedes Cycloalkyl, Aryl oder Heterocyclyl optional substituiert ist mit 1 oder 2 Gruppen, die ausgewählt sind aus Halogen, C₁-C₃-Alkylalkohol, -C₀-C₃-Alkylamin, C₀-C₃-Alkyl-COOH, -CONH₂ und C₀-C₃-Alkylcyano.

3. Verbindung nach Anspruch 1 oder ein pharmazeutisch akzeptables Salz, Enantiomer, Racemat, Diastereomer oder Gemisch von Diastereomeren hiervon, worin R⁹ für Heterocyclyl steht, das optional substituiert ist mit ein oder zwei Gruppen, die unabhängig ausgewählt sind aus Hydroxy, Halogen, Amino, C(O)O-C₁-C₄-Alkyl, C₁-C₆-Halogenalkyl, C₁-C₆-Alkyl, C₂-C₆-Alkenyl, C₂-C₆-Alkinyl, C₁-C₆-Alkoxy, C₁-C₆-Alkylalkohol, C₁-C₆-Alkylamin, C₃-C₈-Cycloalkyl, C₁-C₆-Alkyl-CONR⁷R⁸, C₁-C₆-Alkylcyano, C₁-C₆-Alkyl-NR⁷R⁸ und C₁-C₆-Alkyl-C₃-C₈-cycloalkyl.

4. Verbindung nach Anspruch 1 oder ein pharmazeutisch akzeptables Salz, Enantiomer, Racemat, Diastereomer oder Gemisch von Diastereomeren hiervon, worin n, m und q jeweils unabhängig für 0 oder 1 stehen.

5. Verbindung nach Anspruch 1 oder ein pharmazeutisch akzeptables Salz, Enantiomer, Racemat, Diastereomer oder Gemisch von Diastereomeren hiervon, worin der Ring A ausgewählt ist aus Pyridin, Pyrazin, Thiophen, Pyrazol, Isoxazol, Oxazol und Thiazol.

6. Verbindung nach Anspruch 1 oder ein pharmazeutisch akzeptables Salz, Enantiomer, Racemat, Diastereomer oder Gemisch von Diastereomeren hiervon, worin der Ring A für Pyridin steht.

7. Verbindung nach Anspruch 1 oder ein pharmazeutisch akzeptables Salz, Enantiomer, Racemat, Diastereomer oder Gemisch von Diastereomeren hiervon, worin der Ring A für Thiophen steht.

8. Verbindung nach Anspruch 1 oder ein pharmazeutisch akzeptables Salz, Enantiomer, Racemat, Diastereomer oder Gemisch von Diastereomeren hiervon, worin R⁹ für COOR⁷ steht.

9. Verbindung nach Anspruch 1, die ausgewählt ist aus
5-[Acetyl-(3,5-bistrifluormethylbenzyl)-amino]-2,3,4,5-tetrahydrothieno[3,4-b]azepin-1-carbonsäureisopropylester,
8-[Acetyl-(3,5-bistrifluormethylbenzyl)-amino]-3-methyl-5,6,7,8-tetrahydrothieno[3,2-b]azepin-4.-carbonsäureisopropylester,
8-[Acetyl-(3,5-bistrifluormethylbenzyl)-amino]-2-brom-5,6,7,8-tetrahydrothieno[3,2-b]azepin-4-carbonsäureisopropylester,
5-[Acetyl-(3,5-bistrifluormethylbenzyl)-amino]-5,6,7,8-tetrahydropyrido[2,3-b]azepin-9-carbonsäureisopropylester,
5-[Acetyl-(3,5-bistrifluormethylbenzyl)-amino]-2,3,4,5-tetrahydropyrido[3,4-b]azepin-1-carbonsäureisopropylester,
5-[Acetyl-(3,5-bistrifluormethylbenzyl)-amino]-2,3,4,5-tetrahydropyrido[4,3-b]azepin-1-carbonsäureisopropylester,
9-[Acetyl-(3,5-bistrifluormethylbenzyl)-amino]-6,7,8,9-tetrahydropyrido[3,2-b]azepin-5-carbonsäureisopropylester,
9-[Acetyl-(3,5-bistrifluormethylbenzyl)-amino]-2-trifluormethyl-6,7,8,9-tetrahydropyrido[3,2-b]azepin-5-carbonsäureisopropylester,
9-[Acetyl-(3,5-bistrifluormethylbenzyl)-amino]-3-trifluormethyl-6,7,8,9-tetrahydropyrido[3,2-b]azepin-5-carbonsäureisopropylester,
4-[Acetyl-(3,5-bistrifluormethylbenzyl)-amino]-1-methyl-4.,5,6,7-tetrahydro-1 H-1,2,8-triazaazulen-8-carbonsäureisopropylester,
9-[Acetyl-(3,5-bistrifluormethylbenzyl)-amino]-2-chlor-6,7,8,9-tetrahydropyrido[3,2-b]azepin-5-carbonsäureisopropylester,
9-[Acetyl-(3,5-bistrifluormethylbenzyl)-amino]-2-methoxy-6,7,8,9-tetrahydropyrido[3,2-b]azepin-5-carbonsäureisopropylester,
9-[Acetyl-(3,5-bistrifluormethylbenzyl)-amino]-2-brom-6,7,8,9-tetrahydropyrido[3,2-b]azepin-5-carbonsäureisopropylester,
9-[Acetyl-(3,5-bistrifluormethylbenzyl)-amino]-2-dimethylamino-6,7,8,9-tetrahydropyrido[3,2-b]azepin-5-carbonsäureisopropylester,
9-[Acetyl-(3,5-bistrifluormethylbenzyl)-amino]-2-methyl-6,7,8,9-tetrahydropyrido[3,2-b]azepin-5-carbonsäureisopropylester,
9-[Acetyl-(3,5-bistrifluormethylbenzyl)-amino]-2-cyano-6,7,8,9-tetrahydropyrido[3,2-b]azepin-5-carbonsäureisopropylester,
9-[Acetyl-(3,5-bistrifluormethylbenzyl)-amino]-3-chlor-2-methoxy-6,7,8,9-tetrahydropyrido[3,2-b]azepin-5-carbonsäureisopropylester,
9-[Acetyl-(3,5-bistrifluormethylbenzyl)-amino]-3-chlor-2-ethoxy-6,7,8,9-tetrahydropyrido[3,2-b]azepin-5-carbonsäureisopropylester,
9-[Acetyl-(3,5-bistrifluormethylbenzyl)-amino]-2-methyl-3-trifluormethyl-6,7,8,9-tetrahydropyrido[3,2-b]-azepin-5-carbonsäureisopropylester,
9-[Acetyl-(3,5-bistrifluormethylbenzyl)-amino]-2-methyl-3-trifluormethyl-6,7,8,9-tetrahydropyrido[3,2-b]-azepin-5-carbonsäure-tert-butylester,
9-[(3,5-Bistrifluormethylbenzyl)-2-methyl-2H-tetrazol-5-yl)-amino]-2-methyl-3-trifluormethyl-6,7,8,9-tetrahydropyrido[3,2-b]azepin-5-carbonsäureisopropylester,
9-[(3,5-Bistrifluormethylbenzyl)-2-methyl-2H-tetrazol-5-yl)-amino]-2-methyl-3-trifluormethyl-6,7,8,9-tetrahydropyrido[3,2-b]azepin-5-carbonsäure-tert-butylester,
(3,5-Bistrifluormethylbenzyl)-(5-cyclopentylmethyl-2-methyl-3-trifluormethyl-6,7,8,9-tetrahydro-5H-pyrido-[3,2-b]azepin-9-yl)-(2-methyl-2H-tetrazol-5-yl)-am in,
(3,5-Bistrifluormethylbenzyl)-(5-cyclopropylmethyl-2-methyl-3-trifluormethyl-6,7,8,9-tetrahydro-5H-pyrido-[3,2-b]azepin-9-yl)-(2-methyl-2H-tetrazol-5-yl)-amin,
(3,5-Bistrifluormethylbenzyl)-(2-methyl-5-pyridin-3-ylmethyl-3-trifluormethyl-6,7,8,9-tetrahydro-5H-pyrido-[3,2-b]azepin-9-yl)-(2-methyl-2H-tetrazol-5-yl)-amin,
(3,5-Bistrifluormethylbenzyl)-(2-methyl-5-pyridin-4-ylmethyl-3-trifluormethyl-6,7,8,9-tetrahydro-5H-pyrido-[3,2-b]azepin-9-yl)-(2-methyl-2H-tetrazol-5-yl)-amin,
3-{9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-ylmethyl}-benzoesäure,
4-{9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-ylmethyl}-benzoesäure,
5-{9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-yl}-3,3-dimethylpentansäure,
(4-{9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-ylmethyl}-cyclohexyl)-essigsäure,
(3,5-Bistrifluormethylbenzyl)-(5-ethyl-2-methyl-3-trifluormethyl-6,7,8,9-tetrahydro-5H-pyrido[3,2-b]azepin-9-yl)-(2-methyl-2H-tetrazol-5-yl)-amin,
5-{9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-ylmethyl}-thiophen-2-carbonsäure,
2-{9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-yl}-ethanol,
(5-Benzyl-2-methyl-3-trifluormethyl-6,7,8,9-tetrahydro-5H-pyrido[3,2-b]azepin-9-yl)-(3,5-bistrifluormethylbenzyl)-(2-methyl-2H-tetrazol-5-yl)-amin,
(3,5-Bistrifluormethylbenzyl)-(2-methyl-2H-tetrazol-5-yl)-(2-methyl-5-thiazol-2-ylmethyl-3-trifluormethyl-6,7,8,9-tetrahydro-5H-pyrido[3,2-b]azepin-9-yl)-amin,
9-[(3,5-Bistrifluormethylbenzyl)-(2-methyl-2H-tetrazol-5-yl)-amino]-2-methyl-3-trifluormethyl-6,7,8,9-tetrahydropyrido[3,2-b]azepin-5-carbonsäuretetrahydrofuran-3-ylester,
(3,5-Bistrifluormethylbenzyl)-(2-methyl-5-pyridin-4-ylmethyl-3-trifluormethyl-6,7,8,9-tetrahydro-5H-pyrido-[3,2-b]azepin-9-yl)-carbaminsäuremethylester,
N-(3,5-Bistrifluormethylbenzyl)-N-(2-methyl-5-pyridin-4-ylmethyl-3-trifluormethyl-6,7,8,9-tetrahydro-5H-pyrido[3,2-b]azepin-9-yl)-acetamid,
oder ein pharmazeutisch akzeptables Salz, Solvat, Enantiomer oder Diastereomer oder Gemisch von Diastereomeren hiervon.

10. Pharmazeutische Zusammensetzung, umfassend eine Verbindung nach einem der Ansprüche 1 bis 9 oder ein pharmazeutisch akzeptables Salz, Enantiomer, Racemat, Diastereomer oder Gemisch von Diastereomeren hiervon, und einen Träger, ein Verdünnungsmittel und/oder ein Exzipient.

11. Verbindung nach einem der Ansprüche 1 bis 9 oder ein pharmazeutisch akzeptables Salz, Enantiomer, Racemat, Diastereomer oder Gemisch von Diastereomeren hiervon, zur Verwendung als ein Arzneimittel.

12. Verwendung einer Verbindung nach einem der Ansprüche 1 bis 9 oder eines pharmazeutisch akzeptablen Salzes, Enantiomers, Racemats, Diastereomers oder Gemisches von Diastereomeren hiervon, zur Herstellung eines Arzneimittels für die Behandlung oder Prävention von Dyslipidämie.

13. Verwendung einer Verbindung nach einem der Ansprüche 1 bis 9 oder eines pharmazeutisch akzeptablen Salzes, Enantiomers, Racemats, Diastereomers oder Gemisches von Diastereomeren hiervon, zur Herstellung eines Arzneimittels für die Behandlung von Hyperlipidämie, Atherosklerose, koronarem Herzversagen, erhöhtem Blutdruck, CHF, Schlag, Hypertension, Hypertriglyceridämie, Diabetes, Obesität, inflammatorischer Krankheiten unter Einschluss von Dermatitis, Arthritis und Schmerz und Krankheiten des zentralen Nervensystems unter Einschluss von Demenz und Kognitionsstörungen.

14. Verwendung einer Verbindung nach einem der Ansprüche 1 bis 9 oder eines pharmazeutisch akzeptablen Salzes, Enantiomers, Racemats, Diastereomers oder Gemisches von Diastereomeren hiervon, zur Herstellung eines Arzneimittels für eine Erhöhung des Plasmaspiegels von HDL Cholesterin bei einem Säuger.

15. Verwendung einer Verbindung nach einem der Ansprüche 1 bis 9 oder eines pharmazeutisch akzeptablen Salzes, Enantiomers, Racemats, Diastereomers oder Gemisches von Diastereomeren hiervon, zur Herstellung eines Arzneimittels für eine Erniedrigung des Plasmaspiegels von LDL Cholesterin bei einem Säuger.

16. Verbindung nach einem der Ansprüche 1 bis 9 oder ein pharmazeutisch akzeptables Salz, Enantiomer, Racemat, Diastereomer oder Gemisch von Diastereomeren hiervon, zur Verwendung für die Behandlung oder Prävention von Dyslipidämie.

17. Verbindung nach einem der Ansprüche 1 bis 9, oder ein pharmazeutisch akzeptables Salz, Enantiomer, Racemat, Diastereomer oder Gemisch von Diastereomeren hiervon, zur Verwendung für die Behandlung von Hyperlipidämie, Atherosklerose, koronarem Herzversagen, erhöhtem Blutdruck, CHF, Schlag, Hypertension, Hypertriglyceridämie, Diabetes, Obesität, inflammatorischer Krankheiten unter Einschluss von Dermatitis, Arthritis und Schmerz und Krankheiten des zentralen Nervensystems unter Einschluss von Demenz und Kognitionsstörungen.

18. Verbindung nach einem der Ansprüche 1 bis 9 oder ein pharmazeutisch akzeptables Salz, Enantiomer, Racemat, Diastereomer oder Gemisch von Diastereomeren hiervon, zur Verwendung für eine Erhöhung des Plasmaspiegels von HDL Cholesterin bei einem Säuger.

19. Verbindung nach einem der Ansprüche 1 bis 9 oder ein pharmazeutisch akzeptables Salz, Enantiomer, Racemat, Diastereomer oder Gemisch von Diastereomeren hiervon, zur Verwendung für eine Erniedrigung des Plasmaspiegels von LDL Cholesterin bei einem Säuger.

## Revendications

1. Composé de formule I 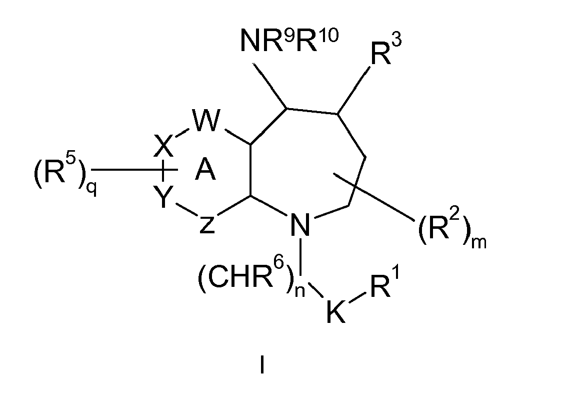 dans laquelle
n vaut 0, 1, 2, ou 3;
m vaut 0, 1, 2, 3, 4, 5 ou 6;
q vaut 0, 1, ou 2 ;
W, X, Y et Z représentent chacun, de manière indépendante, CH, C, N, S, ou O avec les simples ou doubles liaisons et/ou les atomes d'hydrogène appropriés pour satisfaire les exigences de valence ;
Le cycle A est un cycle de cinq ou six membres dans lequel l'un parmi W, X, Y ou Z peut être absent; à condition que le cycle A ne soit pas un groupe phényle ;
K représente une liaison, C=O, ou S(O)ₚ ;
p vaut 0, 1 ou 2 ;
R¹ est choisi parmi un groupe hydroxy, alkyle en C₁-C₆, alcényle en C₂-C₆, halogénoalkyle en C₁-C₆, alkyle en C₁-C₆ hétérocyclique, cycloalkyle en C₃-C₈, (alkyl en C₁-C₆)cycloalkyle en C₃-C₈, alkylaryle en C₁-C₆, aryle, hétérocyclyle, (alkyl en C₂-C₆)alcool, -O(alkyle en C₁-C₆), -O(aryle), -O(alcényle en C₂-C₆), -O(halogénoalkyle en C₁-C₆), -O(alkyle en C₁-C₆ hétérocyclique), -O(cycloalkyle en C₃-C₈), -O(alkyl en C₁-C₆)cycloalkyle en C₃-C₈, -NR⁷R⁸, -O(alkylaryle en C₁-C₆), -O(hétérocyclique), -O(alkyl en C₁-C₆)CO₂R¹¹, -O(alkyl en C₂-C₆)alcool, -O(alkyl en C₁-C₆)NR⁷R⁸, -O(alkyl en C₂-C₆)cyano, CONR¹¹R¹², NR¹¹SO₂R¹², NR¹¹COR¹², (alkyl en C₀-C₃)NR¹¹R¹², (alkyl en C₁-C₃)COR¹¹ et (alkyl en C₀-C₆)COOR¹¹; à condition que R¹ ne soit pas un groupe hydroxy lorsque K est S(O)ₚ, CO, et/ou lorsque n vaut zéro et K est une liaison ; et dans laquelle chaque groupe cycloalkyle, aryle ou hétérocyclique est éventuellement substitué par 1 à 3 groupes choisi(s) de manière indépendante parmi un groupe oxo, hydroxy, halogéno, alkyle en C₁-C₆, alcényle en C₂-C₆, alcynyle en C₂-C₆, alcoxy en C₁-C₆, halogénoalkyle en C₁-C₆, (alkyl en C₁-C₆)alcool, -O(alkyl en C₂-C₆)alcool, halogénoalcoxy en C₁-C₆, CONR¹¹R¹², NR¹¹SO₂R¹², NR¹¹COR¹², (alkyl en C₀-C₃)NR¹¹R¹², (alkyl en C₁-C₃)COR¹¹, (alkyl en C₀-C₆)COOR¹¹, (alkyl en C₀-C₆)cyano, -O(alkyl en C₂-C₆)cyano, (alkyl en C₁-C₆)cycloalkyle en C₃-C₈, phényle, -O(alkyl en C₁-C₆)cycloalkyle en C₃-C₈, -O(alkylaryl en C₁-C₆), -O(alkyl en C₁-C₆ hétérocyclique), et alkylaryle en C₁-C₆ ;
R² est choisi de manière indépendante parmi un atome d'hydrogène, un groupe alkyle en C₁-C₆ et cycloalkyle en C₃-C₈ ;
R³ représente un atome d'hydrogène, un groupe alkyle en C₁-C₆, aryle, alcényle en C₂-C₆, alcynyle en C₂-C₆, alkylaryle en C₁-C₆, alkyle en C₁-C₆ hétérocyclique, cycloalkyle en C₃-C₈, ou (alkyl en C₁-C₆)cycloalkyle en C₃-C₈ ;
R⁵ est choisi parmi un atome d'hydrogène, un atome d'halogène, un groupe hydroxy, halogénoalkyle en C₁-C₆, alkyle en C₁-C₆, alcényle en C₂-C₆, alkyle en C₁-C₆ hétérocyclique, alkylaryle en C₁-C₆, aryle, -O(alkyle en C₁-C₆), - O-aryle, -O(alcényle en C₂-C₆), -O(halogénoalkyle en C₁-C₆), NR⁷R⁸, - CH₂NR⁷R⁸, -CN, -COOH et NO₂ ;
R⁶ est choisi de manière indépendante parmi un atome d'hydrogène, un groupe alkyle en C₁-C₆, alcényle en C₂-C₆, hydroxy, -O(alkyle en C₁-C₆), -O-aryle, -O(alcényle en C₂-C₆), halogénoalkyle en C₁-C₆, -O(halogénoalkyle en C₁-C₆), (alkyl en C₁-C₆)NR⁷R⁸, cycloalkyle en C₃-C₈, et (alkyl en C₁-C₆)cycloalkyle en C₃-C₈ ;
R⁷ et R⁸ sont choisis de manière indépendante parmi un atome d'hydrogène, un groupe alkyle en C₁-C₆, alcényle en C₂-C₆, cycloalkyle en C₃-C₈, (alkyl en C₁-C₆)cycloalkyle en C₃-C₈, alkyle en C₁-C₆ hétérocyclique, hétérocyclique, aryle, alkylaryle en C₁-C₆, hydroxy, oxo, COOH, C(O)O(alkyle en C₁-C₄), alcynyle en C₂-C₆, alcoxy en C₁-C₆, halogénoalkyle en C₁-C₆, (alkyl en C₁-C₆)alcool, alkylamine en C₁-C₆, (alcényl en C₂-C₆)aryle, (alcynyl en C₂-C₆)aryle, alkyl en C₁-C₆-O-(alkylaryle en C₁-C₆), alkyl en C₁-C₆-NR¹¹-(alkylaryle en C₁-C₆), (alkyl en C₁-C₆)cyano, (alkyl en C₁-C₆)CONR⁷R⁸, (alkyl en C₁-C₆)NR⁷R⁸ et (alkyl en C₁-C₆)NR¹¹COR¹², dans lesquels chaque groupe alkyle, cycloalkyle, aryle ou hétérocyclique est éventuellement substitué par 1 à 3 groupes choisi(s) de manière indépendante parmi un groupe hydroxy, oxo, amino, un atome d'halogène, un groupe (alkyl en C₁-C₆)cycloalkyle en C₃-C₈, cycloalkyle en C₃-C₈, alkyl en C₁-C₆ hétérocyclique, halogénoalkyle en C₁-C₆, COOH, C(O)O(alkyle en C₁-C₄), alkyle en C₁-C₆, alcényle en C₂-C₆, alcynyle en C₂-C₆, alcoxy en C₁-C₆, (alkyl en C₁-C₆)alcool, alkylamine en C₁-C₆ et NR¹¹R¹²; ou bien R⁷ et R⁸ se combinent pour former un cycle hétérocyclique contenant un atome d'azote qui peut avoir 0, 1, ou 2 hétéroatomes supplémentaire(s) choisi(s) parmi un atome d'oxygène, d'azote ou de soufre et peut être éventuellement substitué par un groupe oxo, ou alkyle en C₁-C₆ ;
R⁹ est choisi parmi un groupe alkyle en C₁-C₆, alcényle en C₂-C₆, cycloalkyle en C₃-C₈, (alkyl en C₁-C₆)cycloalkyle, aryle, hétérocyclique, alkyle en C₁-C₆ hétérocyclique, COR⁷, CO₂R⁷, (alkyl en C₀-C₃)CONR⁷R⁸, (alkyl en C₀-C₃)S(O)ₚNR⁷R⁸ et (alkyl en C₀-C₃)S(O)ₚR⁷ dans lesquels R⁷ est tel que défini ci-dessus, et dans lesquels chaque groupe alkyle, cycloalkyle, aryle, et hétérocyclique est éventuellement substitué par un à deux groupes choisi(s), de manière indépendante, parmi un groupe halogéno, hydroxy, oxo, COOH, C(O)O(alkyle en C₁-C₄), halogénoalkyle en C₁-C₆, alkyle en C₁-C₆, alcényle en C₂-C₆, alcynyle en C₂-C₆, alcoxy en C₁-C₆, (alkyl en C₁-C₆)alcool, alkylamine en C₁-C₆, alkylaryle en C₁-C₆, alcénylaryle en C₂-C₆, alcynylaryle en C₂-C₆, alkyle en C₁-C₆ hétérocyclique, -NR⁷R⁸, cycloalkyle en C₃-C₈, (alkyl en C₁-C₆)cycloalkyle en C₃-C₈, (alkyl en C₁-C₆)-O-(alkylaryle en C₁-C₆), (alkyl en C₁-C₆)cyano, (alkyl en C₁-C₆)CONR⁷R⁸, (alkyl en C₁-C₆)NR⁷R⁸, (alkyl en C₁-C₆)NR¹¹COR¹², et aryle, dans lesquels chaque groupe cycloalkyle ou aryle est éventuellement substitué par un groupe halogéno, hydroxy, oxo, amino, COOH, C(O)O(alkyle en C₁-C₄), halogénoalkyle en C₁-C₆, alkyle en C₁-C₆, alcényle en C₂-C₆, alcynyle en C₂-C₆, alcoxy en C₁-C₆, (alkyl en C₁-C₆)alcool et alkylamine en C₁-C₆;
R¹⁰ est choisi parmi un groupe aryle, alkylaryle en C₁-C₆, (alcényl en C₂-C₆)aryle, (alcynyl en C₂-C₆)aryle, (halogénoalkyl en C₁-C₆)aryle, alkyle en C₁-C₆ hétérocyclique, alcényle en C₂-C₆ hétérocyclique, (alkyl en C₁-C₆)cycloalkyle en C₃-C₈, cycloalkyle en C₃-C₈ et (alkyl en C₁-C₆)-O-(alkylaryle en C₁-C₆), dans lesquels chaque groupe cycloalkyle, aryle ou hétérocyclique est éventuellement substitué par 1 à 3 groupes choisi(s) de manière indépendante parmi un groupe hydroxy, oxo, -S(alkyle en C₁-C₆), alkyle en C₁-C₆, alcényle en C₁-C₆, alcynyle en C₁-C₆, halogénoalkyle en C₁-C₆, un atome d'halogène, un groupe alcoxy en C₁-C₆, aryloxy, alcényloxy en C₁-C₆, halogénoalcoxyalkyle en C₁-C₆, (alkyl en C₀-C₆)NR¹¹R¹², -O(alkylaryle en C₁-C₆), nitro, cyano, -O(halogénoalkyle en C₁-C₆), (halogénoalkyl en C₁-C₆)alcool, et (alkyl en C₁-C₆)alcool ;
R¹¹ et R¹² sont choisis, de manière indépendante, parmi un atome d'hydrogène, un groupe alkyle en C₁-C₆, alcényle en C₂-C₆, cycloalkyle en C₃-C₈, hétérocyclique, aryle, et alkylaryle en C₁-C₆, dans lesquels chaque groupe aryle est éventuellement substitué par 1 à 3 groupe(s) choisi(s), de manière indépendante, parmi un atome d'halogène, un groupe alkyle en C₁-C₆ hétérocyclique, et halogénoalkyle en C₁-C₆, ou bien R¹¹ et R¹² se combinent pour former un cycle hétérocyclique contenant un atome d'azote qui peut avoir 0, 1, ou 2 hétéroatome(s) supplémentaire(s) choisi(s) parmi l'oxygène, l'azote ou le soufre et, est éventuellement substitué par un groupe oxo, ou alkyle en C₁-C₆ ou un sel, un énantiomère, un racémate, un diastéréomère ou un mélange de diastéréomères pharmaceutiquement acceptable de celui-ci.

2. Composé selon la revendication 1, ou un sel, un énantiomère, un racémate, un diastéréomère ou un mélange de diastéréomères pharmaceutiquement acceptable de celui-ci, dans lequel n vaut 0, K est C=O et R¹ est choisi parmi un groupe hydroxy, alkyle en C₁-C₆, (alkyl en C₁-C₆)cycloalkyle en C₃-C₈, alkyle en C₁-C₆ hétérocyclique, halogénoalkyle en C₁-C₆, alkylaryle en C₁-C₆, -O(alkyle en C₁-C₆), -O(cycloalkyle en C₃-C₈), -O(alkyl en C₁-C₆)cycloalkyle en C₃-C₈, -O(alkylaryle en C₁-C₆), -O(halogénoalkyle en C₁-C₆), -O(alkyl en C₂-C₆)cyano, -O(alkyl en C₁-C₆)CO₂R¹¹, -O(alkyl en C₂-C₆)alcool, -O(alkyl en C₁-C₆)NR⁷R⁸, et -O(alkyle en C₁-C₆ hétérocyclique) et dans lequel chaque groupe cycloalkyle, aryle, ou hétérocyclique est éventuellement substitué par 1 ou 2 groupe(s) choisi(s) parmi un groupe halogéno, (alkyl en C₁-C₃)alcool, -alkylamine en C₀-C₃, (alkyl en C₀-C₃)COOH, -CONH₂ et (alkyl en C₀-C₃)cyano.

3. Composé selon la revendication 1, ou un sel, un énantiomère, un racémate, un diastéréomère ou un mélange de diastéréomères pharmaceutiquement acceptable de celui-ci, dans lequel R⁹ représente un groupe hétérocyclique éventuellement substitué par 1 ou 2 groupe(s) choisi(s), de manière indépendante, parmi un groupe hydroxy, halogéno, aminé, C(O)O(alkyle en C₁-C₄), halogénoalkyle en C₁-C₆, alkyle en C₁-C₆, alcényle en C₂-C₆, alcynyle en C₂-C₆, alcoxy en C₁-C₆, (alkyl en C₁-C₆)alcool, alkylamine en C₁-C₆, cycloalkyle en C₃-C₈, (alkyl en C₁-C₆)CONR⁷R⁸, (alkyl en C₁-C₆)cyano, (alkyl en C₁-C₆)NR⁷R⁸, et (alkyl en C₁-C₆)cycloalkyle en C₃-C₈.

4. Composé selon la revendication 1, ou un sel, un énantiomère, un racémate, un diastéréomère ou un mélange de diastéréomères pharmaceutiquement acceptable de celui-ci, dans lequel n, m, et q valent de manière indépendante 0 ou 1.

5. Composé selon la revendication 1, ou un sel, un énantiomère, un racémate, un diastéréomère ou un mélange de diastéréomères pharmaceutiquement acceptable de celui-ci, dans lequel le cycle A est choisi parmi un cycle pyridine, pyrazine, thiophène, pyrazole, isoxazole, oxazole, et thiazole.

6. Composé selon la revendication 1, ou un sel, un énantiomère, un racémate, un diastéréomère ou un mélange de diastéréomères pharmaceutiquement acceptable de celui-ci, dans lequel le cycle A est une pyridine.

7. Composé selon la revendication 1, ou un sel, un énantiomère, un racémate, un diastéréomère ou un mélange de diastéréomères pharmaceutiquement acceptable de celui-ci, dans lequel le cycle A est un thiophène.

8. Composé selon la revendication 1, ou un sel, un énantiomère, un racémate, un diastéréomère ou un mélange de diastéréomères pharmaceutiquement acceptable de celui-ci, dans lequel R⁹ représente COOR⁷.

9. Composé selon la revendication 1 choisi parmi :
l'ester isopropylique d'acide 5-[acétyl-(3,5-bis-trifluorométhyl-benzyl)-amino]-2,3,4,5-tétrahydro-thiéno[3,4-b]azépine-1- carboxylique,
l'ester isopropylique d'acide 8-[acétyl-(3,5-bis-trifluorométhyl-benzyl)-amino]-3-méthyl-5,6,7,8-tétrahydro-thiéno[3,2-b]azépine-4-carboxylique,
l'ester isopropylique d'acide 8-[acétyl-(3,5-bis-trifluorométhyl-benzyl)-amino]-2-bromo-5,6,7,8-tétrahydro-thiéno[3,2-b]azépine-4-carboxylique,
l'ester isopropylique d'acide 5-[acétyl-(3,5-bis-trifluorométhyl-benzyl)-amino]-5,6,7,8-tétrahydro-pyrido[2,3-b]azépine-9-carboxylique,
l'ester isopropylique d'acide 5-[acétyl-(3,5-bis-trifluorométhyl-benzyl)-amino]-2,3,4,5-tétrahydro-pyrido[3,4-b]azépine-1-carboxylique,
l'ester isopropylique d'acide 5-[acétyl-(3,5-bis-trifluorométhyl-benzyl)-amino]-2,3,4,5-tétrahydro-pyrido[4,3-b]azépine-1-carboxylique,
l'ester isopropylique d'acide 9-[acétyl-(3,5-bis-trifluorométhyl-benzyl)-amino]-6,7,8,9-tétrahydro-pyrido[3,2-b]azépine-5-carboxylique,
l'ester isopropylique d'acide 9-[acétyl-(3,5-bis-trifluorométhyl-benzyl)-amino]-2-trifluorométhyl-6,7,8,9-tétrahydro-pyrido[3,2-b]azépine-5-carboxylique,
l'ester isopropylique d'acide 9-[acétyl-(3,5-bis-trifluorométhyl-benzyl)-amino]-3-trifluorométhyl-6,7,8,9-tétrahydro-pyrido[3,2-b]azépine-5-carboxylique,
l'ester isopropylique d'acide 4-[acétyl-(3,5-bis-trifluorométhyl-benzyl)-amino]-1-méthyl-4,5,6,7-tétrahydro-1H-1,2,8-triaza-azulène-8-carboxylique,
l'ester isopropylique d'acide 9-[acétyl-(3,5-bis-trifluorométhylbenzyl)amino]-2-chloro-6,7,8,9-tétrahydro-pyrido[3,2-*b*]azépine-5-carboxylique,
l'ester isopropylique d'acide 9-[acétyl-(3,5-bis-trifluorométhylbenzyl)amino]-2-méthoxy-6,7,8,9-tétrahydro-pyrido[3,2-*b*]azépine-5-carboxylique,
l'ester isopropylique d'acide 9-[acétyl-(3,5-bis-trifluorométhylbenzyl)amino]-2-bromo-6,7,8,9-tétrahydro-pyrido[3,2-b]azépine-5-carboxylique,
l'ester isopropylique d'acide 9-[acétyl-(3,5-bis-trifluorométhylbenzyl)amino]-2-diméthylamino-6,7,8,9-tétrahydro-pyrido[3,2-*b*]azépine-5-carboxylique,
l'ester isopropylique d'acide 9-[acétyl-(3,5-bis-trifluorométhylbenzyl)amino]-2-méthyl-6,7,8,9-tétrahydro-pyrido[3,2-*b*]azépine-5-carboxylique,
l'ester isopropylique d'acide 9-[acétyl-(3,5-bis-trifluorométhylbenzyl)amino]-2-cyano-6,7,8,9-tétrahydro-pyrido[3,2-*b*]azépine-5-carboxylique,
l'ester isopropylique d'acide 9-[acétyl-(3,5-bis-trifluorométhylbenzyl)amino]-3-chloro-2-méthoxy-6,7,8,9-tétrahydro-pyrido[3,2-*b*]azépine-5-carboxylique,
l'ester isopropylique d'acide 9-[acétyl-(3,5-bis-trifluorométhylbenzyl)amino]-3-chloro-2-éthoxy-6,7,8,9-tétrahydro-pyrido[3,2-*b*]azépine-5-carboxylique,
l'ester isopropylique d'acide 9-[acétyl-(3,5-bis-trifluorométhyl-benzyl)amino]-2-méthyl-3-trifluorométhyl-6,7,8,9-tétrahydro-pyrido[3,2-*b*]azépine-5-carboxylique,
l'ester tert-butylique d'acide 9-[acétyl-(3,5-bis-trifluorométhyl-benzyl)amino]-2-méthyl-3-trifluorométhyl-6,7,8,9-tétrahydro-pyrido[3,2-*b*]azépine-5-carboxylique,
l'ester isopropylique d'acide 9-[(3,5-bis-trifluorométhyl-benzyl)-2-méthyl-2H-tétrazol-5-yl)-amino]-2-méthyl-3-trifluorométhyl-6,7,8,9-tétrahydro-pyrido[3,2-b]azépine-5-carboxylique,
l'ester *tert*-butylique d'acide 9-[(3,5-bis-trifluorométhyl-benzyl)-2-méthyl-2*H-*tétrazol-5-yl)-amino]-2-méthyl-3-trifluorométhyl-6,7,8,9-tétrahydro-pyrido[3,2-b]azépine-5-carboxylique,
la (3,5-bis-trifluorométhyl-benzyl)-(5-cyclopentylméthyl-2-méthyl-3-trifluorométhyl-6,7,8,9-tétrahydro-5H-pyrido[3,2-b]azépin-9-yl)-(2-méthyl-2H-tétrazol-5-yl)-amine,
la (3,5-bis-trifluorométhyl-benzyl)-(5-cyclopropylméthyl-2-méthyl-3-trifluorométhyl-6,7,8,9-tétrahydro-5H-pyrido[3,2-b]azépin-9-yl)-(2-méthyl-2H-tétrazol-5-yl)-amine,
la (3,5-bis-trifluorométhyl-benzyl)-(2-méthyl-5-pyridin-3-ylméthyl-3-trifluorométhyl-6,7,8,9-tétrahydro-5H-pyrido[3,2-b]azépin-9-yl)-(2-méthyl-2H-tétrazol-5-yl)-amine,
la (3,5-bis-trifluorométhyl-benzyl)-(2-méthyl-5-pyridin-4-ylméthyl-3-trifluorométhyl-6,7,8,9-tétrahydro-5H-pyrido[3,2-b]azépin-9-yl)-(2-méthyl-2H-tétrazol-5-yl)-amine,
l'acide 3-{9-[(3,5-bis-trifluorométhyl-benzyl)-(2-méthyl-2H-tétrazol-5-yl)-amino]-2-méthyl-3-trifluorométhyl-6,7,8,9-tétrahydro-pyrido[3,2-b]azépin-5-ylméthyl}-benzoïque,
l'acide 4-{9-[(3,5-bis-trifluorométhyl-benzyl)-(2-méthyl-2H-tétrazol-5-yl)-amino]-2-méthyl-3-trifluorométhyl-6,7,8,9-tétrahydro-pyrido[3,2-b]azépin-5-ylméthyl}benzoïque,
l'acide 5-{9-[(3,5-bis-trifluorométhyl-benzyl)-(2-méthyl-2H-tétrazo1-5-yl)-amino]-2-méthyl-3-trifluorométhyl-6,7,8,9-tétrahydro-pyrido[3,2-b]azépin-5-yl}-3,3-diméthyl-pentanoïque,
l'acide (4-{9-[(3,5-bis-trifluorométhyl-benzyl)-(2-méthyl-2H-tétrazol-5-yl)-amino]-2-méthyl-3-trifluorométhyl-6,7,8,9-tétrahydro-pyrido[3,2-b]azépin-5-ylméthyl}-cyclohexyl)-acétique,
la (3,5-bis-trifluorométhyl-benzyl)-(5-éthyl-2-méthyl-3-trifluorométhyl-6,7,8,9-tétrahydro-5H-pyrido[3,2-b]azépin-9-yl)-(2-méthyl-2H-tétrazol-5-yl)-amine,
l'acide 5-{9-[(3,5-bis-trifluorométhyl-benzyl)-(2-méthyl-2H-tétrazol-5-yl)-amino]-2-méthyl-3-trifluorométhyl-6,7,8,9-tétrahydro-pyrido[3,2-b]azépin-5-ylméthyl}thiophène-2-carboxylique,
le 2-{9-[(3,5-bis-trifluorométhyl-benzyl)-(2-méthyl-2H-tétrazol-5-yl)-amino]-2-méthyl-3-trifluorométhyl-6,7,8,9-tétrahydro-pyrido[3,2-b]azépin-5-yl}-éthanol,
la (5-benzyl-2-méthyl-3-trifluorométhyl-6,7,8,9-tétrahydro-5H-pyrido[3,2-b]azépin-9-yl)-(3,5-bis-trifluorométhyl-benzyl)-(2-méthyl-2H-tétrazol-5-yl)-amine,
la (3,5-bis-trifluorométhyl-benzyl)-(2-méthyl-2H-tétrazol-5-yl)-(2-méthyl-5-thiazol-2-ylméthyl-3-trifluorométhyl-6,7,8,9-tétrahydro-5H-pyrido[3,2-b]azépin-9-yl)-amine,
l'ester de tétrahydro-furan-3-yle et d'acide 9-[(3,5-bis-trifluorométhyl-benzyl)-(2-méthyl-2H-tétrazol-5-yl)-amino]-2-méthyl-3-trifluorométhyl-6,7,8,9-tétrahydropyrido[3,2-b]azépine-5-carboxylique,
l'ester méthylique d'acide (3,5-bis-trifluorométhyl-benzyl)-(2-méthyl-5-pyridin-4ylméthyl-3-trifluorométhyl-6,7,8,9-tétrahydro-5H-pyrido[3,2-b]azépin-9-yl)-carbamique,
le N-(3,5-bis-trifluorométhyl-benzyl)-N-(2-méthyl-5-pyridin-4-ylméthyl-3-trifluorométhyl-6,7,8,9-tétrahydro-5H-pyrido[3,2-b]azépin-9-yl)-acétamide ou un sel, un solvate, un énantiomère, ou un diastéréomère ou un mélange pharmaceutiquement acceptable de celui-ci.

10. Composition pharmaceutique comprenant un composé selon l'une quelconque des revendications 1 à 9, ou un sel, un énantiomère, un racémate, un diastéréomère, ou un mélange de diastéréomères pharmaceutiquement acceptable de celui-ci, et un support, un diluant et/ou un excipient.

11. Composé selon l'une quelconque des revendications 1 à 9, ou un sel, un énantiomère, un racémate, un diastéréomère, ou un mélange de diastéréomères pharmaceutiquement acceptable de celui-ci pour une utilisation en tant que médicament.

12. Utilisation d'un composé selon l'une quelconque des revendications 1 à 9, ou d'un sel, d'un énantiomère, d'un racémate, d'un diastéréomère, ou d'un mélange de diastéréomères pharmaceutiquement acceptable de celui-ci pour la fabrication d'un médicament destiné au traitement ou la prévention d'une dyslipidémie.

13. Utilisation d'un composé selon l'une quelconque des revendications 1 à 9, ou d'un sel, d'un énantiomère, d'un racémate, d'un diastéréomère, ou d'un mélange de diastéréomères pharmaceutiquement acceptable de celui-ci pour la fabrication d'un médicament destiné au traitement d'une hyperlipidémie, d'une athérosclérose, d'une maladie coronarienne, d'une pression sanguine élevée, d'une ICC, d'un accident vasculaire cérébral, d'une hypertension, d'une hypertriglycéridémie, d'un diabète, d'une obésité, des maladies inflammatoires incluant une dermatite, une arthrite et une douleur, et des maladies du système nerveux central incluant une démence et des troubles cognitifs.

14. Utilisation d'un composé selon l'une quelconque des revendications 1 à 9, ou d'un sel, d'un énantiomère, d'un racémate, d'un diastéréomère, ou d'un mélange de diastéréomères pharmaceutiquement acceptable de celui-ci pour la fabrication d'un médicament destiné à élever le taux de cholestérol HDL plasmatique chez un mammifère.

15. Utilisation d'un composé selon l'une quelconque des revendications 1 à 9, ou d'un sel, d'un énantiomère, d'un racémate, d'un diastéréomère, ou d'un mélange de diastéréomères pharmaceutiquement acceptable de celui-ci pour la fabrication d'un médicament destiné à abaisser le taux de cholestérol LDL plasmatique chez un mammifère.

16. Composé selon l'une quelconque des revendications 1 à 9, ou un sel, un énantiomère, un racémate, un diastéréomère, ou un mélange de diastéréomères pharmaceutiquement acceptable de celui-ci pour une utilisation dans le traitement ou la prévention d'une dyslipidémie.

17. Composé selon l'une quelconque des revendications 1 à 9, ou un sel, un énantiomère, un racémate, un diastéréomère, ou un mélange de diastéréomères pharmaceutiquement acceptable de celui-ci pour une utilisation dans le traitement d'une hyperlipidémie, d'une athérosclérose, d'une maladie coronarienne, d'une pression sanguine élevée, d'une ICC, d'un accident vasculaire cérébral, d'une hypertension, d'une hypertriglycéridémie, d'un diabète, d'une obésité, des maladies inflammatoires incluant une dermatite, une arthrite et une douleur, et des maladies du système nerveux central incluant une démence et des troubles cognitifs.

18. Composé selon l'une quelconque des revendications 1 à 9, ou un sel, un énantiomère, un racémate, un diastéréomère, ou un mélange de diastéréomères pharmaceutiquement acceptable de celui-ci pour une utilisation dans l'élévation du taux de cholestérol HDL plasmatique chez un mammifère.

19. Composé selon l'une quelconque des revendications 1 à 9, ou un sel, un énantiomère, un racémate, un diastéréomère, ou un mélange de diastéréomères pharmaceutiquement acceptable de celui-ci pour une utilisation dans l'abaissement du taux de cholestérol LDL plasmatique chez un mammifère.
